# EUROPEAN PATENT SPECIFICATION

(11) **EP 1 838 707 B1**
(45) Date of publication and mention of the grant of the patent: **29.04.2009**
(21) Application number: 06701124.7
(22) Date of filing: 11.01.2006
(51) Int. Cl.: C07D 471/04, A61K 31/4745, A61P 25/00

(54) **NOVEL PYRROLODIHYDROISOQUINOLINES AS PDE10 INHIBITORS**
NEUE PYRROLODIHYDROISOCHINOLINE ALS PDE10-INHIBITOREN
NOUVELLES PYRROLODIHYDROISOQUINOLINES UTILISEES EN TANT QU'INHIBITEURS DE PDE10

(30) Priority: 12.01.2005 EP 05100154
(43) Date of publication of application: 03.10.2007
(73) Proprietor: Nycomed GmbH, 78467 Konstanz (DE)
(72) Inventor: CONTRERAS, Jean-Marie, 67230 Benfeld (FR); CIAPETTI, Paola, 67120 Altdorf (FR); WERMUTH, Camille, Georges, 67100 Strasbourg (FR); BRAUNGER, Jürgen, 2340Mödling (AT); BÄR, Thomas, 78479 Reichenau (DE); VENNEMANN, Matthias, 78462 Konstanz (DE)
(74) Representative: Wild, Robert
(86) International application number: PCT/EP2006/050167
(87) International publication number: WO 2006/089815

(56) References cited:
- WO-A-03/014115
- WO-A-03/051877
- WO-A-20/05003129

## Description

### Field of application of the invention

The invention relates to novel pyrrolodihydroisoquinoline derivatives, which are used in the pharmaceutical industry for the production of pharmaceutical compositions.

### Technical Background

The International applications WO 02/48144, WO 03/014115, WO 03/014116, WO 03/014117 and WO 03/051877 disclose pyrrolodihydroisoquinoline derivatives with PDE10 inhibitory activity useful in cancer therapy.

The European application EP 1250923 discloses the use of selective PDE10 inhibitors in general, and papaverine in particular, for the treatment of certain neurologic and psychiatric disorders.

Additionally, the US applications US 2003/0008806, US 2003/0018047, US 2003/032579, US 2004/162293 and US 2004/162294 likewise disclose the use of selective PDE10 inhibitors in general, and papaverine in particular, for the treatment of certain neurologic and psychiatric disorders.

Yet additionally, the WO application WO 03/093499 also disclose the use of selective PDE10 inhibitors in general, and papaverine in particular, for the treatment of certain neurologic and psychiatric disorders.

The WO application WO 2005/120514 disclose methods to decrease body weight and/or body fat in animals, methods to treat non-insulin dependent diabetes (NIDDM), metabolic syndrome or glucose intolerance by administering a PDE10 inhibitor.

The WO application WO 2005/012485 relates to the treatment of diabetes, including type 2 diabetes, by administration of a PDE10 inhibitor.

The US patent US 5965575 discloses pyrrolodihydroisoquinoline derivatives as 5HT_{1B} antagonists. The International application WO 03/000269 disclose the use of PDE10A inhibitors for the treatment of neurodegenerative diseases, especially Parkinson's disease.

The International application WO 2005/003129 relates to pyrrolodihydroisoquinoline derivatives, which are efficacious inhibitors of PDE10.

The International application WO 2005/002579 relates to the use of a pyrrolo[2.1-a]isoquinoline structure-element as an integral part of the overall structure of compounds, which inhibit PDE10.

### Description of the Invention

It has now been found that the pyrroloisoquinoline derivatives, which are described in greater details below, differ from prior art compounds by unanticipated structural features and have surprising and particularly advantageous properties.

The invention thus relates to compounds of formula I 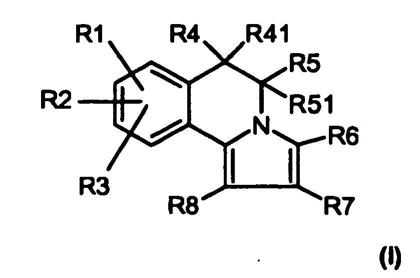 in which
- R1: is halogen, nitro, amino, mono- or di-1-4C-alkylamino, 1-4C-alkyl, hydroxyl, 1-4C-alkoxy, 1-4C-alkoxy-2-4C-alkoxy, 3-7C-cycloalkyl, 3-7C-cycloalkylmethoxy, or completely or predominantly fluorine-substituted 1-4C-alkoxy,
- R2: is hydrogen, halogen or 1-4C-alkoxy, and
- R3: is hydrogen or 1-4C-alkoxy, or
- R2 and R3: bound to the benzo ring moiety in ortho-position to each other together form a 1-2C-alkylenedioxy bridge, or
- R2 and R3: bound to the benzo ring moiety in ortho-position to each other together form a completely or predominantly fluorine-substituted 1-2C-alkylenedioxy bridge, or
- R1 and R2: bound to the benzo ring moiety in ortho-position to each other together form a 1-2C-alkylenedioxy bridge and R3 is hydrogen, or
- R1 and R2: bound to the benzo ring moiety in ortho-position to each other together form a completely or predominantly fluorine-substituted 1-2C-alkylenedioxy bridge and R3 is hydrogen,
- R4: is hydrogen, fluorine, chlorine, 1-4C-alkyl, trifluoromethyl, cyclopropyl, cyano, 1-4C-alkoxycarbonyl or -CH₂-O-R411, in which
- R411: is hydrogen, 1-4C-alkyl, 1-4C-alkoxy-2-4-alkyl or 1-4C-alkylcarbonyl,
- R41: is hydrogen or 1-4C-alkyl,
- R5: is hydrogen, fluorine or 1-4C-alkyl, and
- R51: is hydrogen or 1-4C-alkyl,
or
- R4: is hydrogen, fluorine, chlorine or 1-4C-alkyl,
- R41: is hydrogen or 1-4C-alkyl,
- R5: is hydrogen, fluorine, 1-4C-alkyl, trifluoromethyl, cyclopropyl, cyano, 1-4C-alkoxycarbonyl or -CH₂-O-R511, in which
- R511: is hydrogen, 1-4C-alkyl, 1-4C-alkoxy-2-4-alkyl or 1-4C-alkylcarbonyl, and
- R51: is hydrogen or 1-4C-alkyl,
or
- R4 and R5: together form a 1-4C-alkylene bridge and R41 and R51 are both hydrogen,
- R6: is 1-6C-alkyl, amino, formyl, or 1-4C-alkyl substituted by R61, in which
- R61: is 1-4C-alkoxycarbonyl, carboxyl, 1-4C-alkoxy, hydroxyl, halogen or -N(R611)R612, in which
- R611: is hydrogen, 1-4C-alkyl, 3-7C-cycloalkyl or 3-7C-cycloalkyl-1-4C-alkyl, and
- R612: is hydrogen or 1-4C-alkyl, or
- R611: and R612 together and with inclusion of the nitrogen atom to which they are bound form a radical Het1, in which
- Het1: is a 5- to 7-membered saturated heterocyclic ring radical comprising one nitrogen atom, to which R611 and R612 are bound, and, optionally, one further heteroatom selected from a group consisting of nitrogen, oxygen and sulfur, and optionally substituted by R613 on a ring nitrogen atom, in which
- R613: is 1-4C-alkyl, 3-7C-cycloalkyl, 3-7C-cycloalkyl-1-4C-alkyl, hydroxy-2-4C-alkyl, 1-4C-alkoxy-2-4C-alkyl, amino-2-4C-alkyl, mono- or di-1-4C-alkylamino-2-4C-alkyl, formyl, pyridyl or pyrimidinyl,
- R7: is phenyl, Het2, R71- and/or R72- and/or R73-substituted phenyl, R74- and/or R75-substituted Het2, naphthyl, or R76- and/or R77-substituted naphthyl, in which
- Het2: is bonded to the pyrroloisoquinoline scaffold via a ring carbon atom, and is a monocyclic or fused bicyclic 5- to 10-membered partially or fully aromatic heterocyclic ring radical comprising one to four heteroatoms, each of which is selected from a group consisting of nitrogen, oxygen and sulfur,
- R71: is hydroxyl, halogen, nitro, cyano, trifluoromethyl, 1-4C-alkyl, 1-4C-alkoxy, 3-7C-cycloalkoxy, 3-7C-cydoalkylmethoxy, amino, mono- or di-1-4C-alkylamino, 1-4C-alkylsulphonylamino, arylsulphonylamino, 1-4C-alkoxycarbonyl, carboxyl, 1-4C-alkylthio, aryloxy-2-4C-alkoxy, aryloxy-1-4C-alkyl, aryloxy, aryl-1-4C-alkoxy, aryl, 1-4C-alkoxy-2-4C-alkoxy, 1-4C-alkoxy-1-4C-alkyl, hydroxy-2-4C-alkoxy, amino-2-4C-alkoxy, mono- or di-1-4C-alkylamino-2-4C-alkoxy, completely or predominantly fluorine-substituted 1-4C-alkoxy, mono- or di-1-4C-alkylaminocarbonyl, carbamoyl, tetrazolyl, or -N(H)S(O)₂-N(R712)R713, in which
- aryl: is phenyl or R711-substituted phenyl, in which
- R711: is halogen, 1-4C-alkyl, 1-4C-alkoxy, nitro or cyano,
- R712: is 1-4C-alkyl,
- R713: is 1-4C-alkyl, or
- R712: and R713 together and with inclusion of the nitrogen atom to which they are bound form a radical Het3, in which
- Het3: is pyrrolidin-1-yl, piperidin-1-yl or morpholin-4-yl,
- R72: is halogen, 1-4C-alkyl, 1-4C-alkoxy or 1-4C-alkoxycarbonyl,
- R73: is 1-4C-alkyl or 1-4C-alkoxy,
- R74: is halogen, 1-4C-alkyl, trifluoromethyl, 1-4C-alkoxy, cyano, amino, mono- or di-1-4C-alkylamino, 1-4C-alkoxycarbonyl, morpholino, carboxyl, nitro, phenyl, phenyloxy, phenyl-1-4C-alkyl, arylsulphonyl, 1-4C-alkylsulphonyl, or -S(O)₂-N(R712)R713,
- R75: is 1-4C-alkyl or halogen,
- R76: is halogen, hydroxyl, 1-4C-alkyl, 1-4C-alkoxy, carboxyl or 1-4C-alkoxycarbonyl,
- R77: is 1-4C-alkyl or 1-4C-alkoxy,
- R8: is optionally substituted by R81, and is Het4, in which
- Het4: is bonded to the pyrroloisoquinoline scaffold via a ring carbon atom, and is an oxadiazolyl or an oxazolyl radical,
- R81: is 1-4C-alkyl, 3-7C-cycloalkyl, 3-7C-cycloalkylmethyl, phenyl, or R811- and/or R812-substituted phenyl, in which
- R811: is 1-4C-alkyl, 1-4C-alkoxy, or halogen,
- R812: is 1-4C-alkyl, 1-4C-alkoxy, or halogen,
and the stereoisomers as well as the salts of these compounds and stereoisomers.

1-4C-Alkyl represents a straight-chain or branched alkyl radical having 1 to 4 carbon atoms. Examples which may be mentioned are the butyl, isobutyl, sec-butyl, tert-butyl, propyl, isopropyl and preferably the ethyl and methyl radicals.

2-4C-Alkyl represents a straight-chain or branched alkyl radical having 2 to 4 carbon atoms. Examples which may be mentioned are the butyl, isobutyl, sec-butyl, tert-butyl, propyl, isopropyl and preferably the ethyl radical.

1-6C-Alkyl represents a straight-chain or branched alkyl radical having 1 to 6 carbon atoms. Examples which may be mentioned are the hexyl, isohexyl (4-methylpentyl), neohexyl (3,3-dimethylbutyl), pentyl, isopentyl (3-methylbutyl), neopentyl (2,2-dimethylpropyl), butyl, isobutyl, sec-butyl, tert-butyl, propyl, isopropyl, ethyl or methyl radicals.

1-4C-Alkoxy represents radicals which, in addition to the oxygen atom, contain a straight-chain or branched alkyl radical having 1 to 4 carbon atoms. Examples which may be mentioned are the butoxy, isobutoxy, sec-butoxy, tert-butoxy, propoxy, isopropoxy and preferably the ethoxy and methoxy radicals.

1-4C-Alkylthio represents radicals which, in addition to the sulfur atom, contain a straight-chain or branched alkyl radical having 1 to 4 carbon atoms. Examples which may be mentioned are the ethylthio and the methylthio radicals.

2-4C-Alkoxy represents radicals which, in addition to the oxygen atom, contain a straight-chain or branched alkyl radical having 2 to 4 carbon atoms. Examples which may be mentioned are the butoxy, isobutoxy, sec-butoxy, tert-butoxy, propoxy, isopropoxy and preferably the ethoxy radical.

3-7C-Cycloalkoxy represents cyclopropyloxy, cyclobutyloxy, cyclopentyloxy, cyclohexyloxy and cycloheptyloxy, of which cyclopropyloxy, cyclobutyloxy and cyclopentyloxy are preferred.

3-7C-Cycloalkyl represents cyclopropyl, cyclobutyl, cyclopentyl, cyclohexyl and cycloheptyl, of which cyclopropyl, cyclobutyl and cyclopentyl are preferred.

3-7C-Cycloalkylmethoxy represents cyclopropylmethoxy, cyclobutylmethoxy, cyclopentylmethoxy, cyclohexylmethoxy and cycloheptylmethoxy, of which cyclopropylmethoxy, cyclobutylmethoxy and cyclopentylmethoxy are preferred.

3-7C-Cycloalkyl-1-4C-alkyl represents one of the abovementioned 1-4C-alkyl radicals, which is substituted by one of the abovementioned 3-7C-cycloalkyl radicals. Examples which may be mentioned are the 3-7C-cycloalkylethyl radicals (e.g. the cyclohexylethyl radical), or the 3-7C-cycloalkylmethyl radicals (such as e.g. cyclopropylmethyl, cyclobutylmethyl, cyclopentylmethyl, cyclohexylmethyl and cycloheptylmethyl, of which cyclopropylmethyl, cyclobutylmethyl and cyclopentylmethyl are preferred).

As completely or predominantly fluorine-substituted 1-4C-alkoxy, for example, the 2,2,3,3,3-pentafluoropropoxy, the perfluoroethoxy, the 1,2,2-trifluoroethoxy, in particular the 1,1,2,2-tetrafluoroethoxy, the 2,2,2-trifluoroethoxy, the trifluoromethoxy and preferably the difluoromethoxy radicals may be mentioned. "Predominantly" in this connection means that more than half of the hydrogen atoms of the 1-4C-alkoxy radicals are replaced by fluorine atoms.

1-4C-Alkoxy-2-4C-alkoxy represents one of the abovementioned 2-4C-alkoxy radicals, which is substituted by one of the abovementioned 1-4C-alkoxy radicals. Examples which may be mentioned are the 2-methoxyethoxy, 2-ethoxyethoxy and the 2-isopropoxyethoxy radicals.

1-4C-Alkoxy-2-4C-alkyl represents one of the abovementioned 2-4C-alkyl radicals, which is substituted by one of the abovementioned 1-4C-alkoxy radicals. Examples which may be mentioned are the 2-methoxyethyl and the 2-isopropoxyethyl radicals.

1-4C-Alkoxy-1-4C-alkyl stands for one of the abovementioned 1-4C-alkyl radicals, which is substituted by one of the abovementioned 1-4C-alkoxy radicals. Examples which may be mentioned are the 2-methoxyethyl and 2-isopropoxyethyl radicals.

1-2C-Alkylenedioxy represents, for example, the methylenedioxy [-O-CH₂-O-] and the ethylenedioxy [-O-CH₂-CH₂-O-] radicals.

As completely or predominantly fluorine-substituted 1-2C-alkylenedioxy bridge, for example, the difluoromethylenedioxy [-O-CF₂-O-] radical may be mentioned. "Predominantly" in this connection means that more than half of the hydrogen atoms of the 1-4C-alkylenedioxy radical are replaced by fluorine atoms.

Phenyl-1-4C-alkyl stands for one of the abovementioned 1-4C-alkyl radicals, which is substituted by a phenyl radical. Examples which may be mentioned are the phenethyl and the benzyl radicals.

1-4C-Alkoxycarbonyl represents a radical which, in addition to the carbonyl group, contains one of the abovementioned 1-4C-alkoxy radicals. Examples which may be mentioned are the methoxycarbonyl and ethoxycarbonyl radicals.

1-4C-Alkylcarbonyl represents a radical which, in addition to the carbonyl group, contains one of the abovementioned 1-4C-alkyl radicals. An example which may be mentioned is the acetyl radical.

1-4C-Alkylene is a straight-chain alkylene radical such as, for example, the methylene (-CH₂-) or, particularly, the trimethylene (-CH₂-CH₂-CH₂-) or the tetramethylene (-CH₂-CH₂-CH₂-CH₂-) radical.

Halogen within the meaning of the invention is bromine and, preferably, chlorine and fluorine.

Hydroxy-2-4C-alkyl stands for one of the abovementioned 2-4C-alkyl radicals which is substituted by a hydroxyl group. Examples which may be mentioned are the 2-hydroxyethyl and 3-hydroxypropyl radicals.

Hydroxy-2-4C-alkoxy stands for one of the abovementioned 2-4C-alkoxy radicals which is substituted by a hydroxyl group. Examples which may be mentioned are the 2-hydroxyethoxy and 3-hydroxypropoxy radicals.

Amino-2-4C-alkyl stands for one of the abovementioned 2-4C-alkyl radicals which is substituted by an amino group. Examples which may be mentioned are the 2-aminoethyl and 3-aminopropyl radicals.

Amino-2-4C-alkoxy stands for one of the abovementioned 2-4C-alkoxy radicals which is substituted by an amino group. Examples which may be mentioned are the 2-aminoethoxy and 3-aminopropoxy radicals.

In addition to the nitrogen atom, mono- or di-1-4C-alkylamino radicals contain one or two of the abovementioned 1-4C-alkyl radicals. Di-1-4C-alkylamino is to be emphasized and here, in particular, dimethyl-, diethyl- and diisopropylamino.

Mono- or Di-1-4C-alkylamino-2-4C-alkyl stands for one of the abovementioned 2-4C-alkyl radicals which is substituted by one of the abovementioned mono- or di-1-4C-alkylamino radicals. Examples which may be mentioned are the 2-dimethylaminoethyl and 3-dimethylaminopropyl radicals.

Mono- or Di-1-4C-alkylamino -2-4C-alkoxy stands for one of the abovementioned 2-4C-alkoxy radicals which is substituted by one of the abovementioned mono- or di-1-4C-alkylamino radicals. Examples which may be mentioned are the 2-dimethylaminoethoxy and 3-dimethylaminopropoxy radicals.

1-4C-Alkylsulfonyl is a sulfonyl group to which one of the abovementioned 1-4C-alkyl radicals is bonded. An example is the methanesulfonyl radical (CH₃SO₂-).

1-4C-Alkylsulfonylamino is an amino group which is substituted by one of the abovementioned 1-4C-alkylsulfonyl radicals. An example is the methanesulfonylamino radical (CH₃SO₂NH-).

Aryl radicals referred to herein, including those forming part of other groups or radicals, include phenyl or R711-substituted phenyl radicals.

Aryloxy stands for phenoxy or R711-substituted phenoxy.

Aryl-1-4C-alkoxy stands for one of the abovementioned 1-4C-alkoxy radicals, which is substituted by one of the abovementioned aryl radicals. Examples which may be mentioned are the 2-arylethoxy (e.g. phenethoxy) and the arylmethoxy (e.g. benzyloxy) radicals.

Aryloxy-2-4C-alkoxy stands for one of the abovementioned 2-4C-alkoxy radicals, which is substituted by one of the abovementioned aryloxy radicals. An example which may be mentioned is the 2-aryloxyethoxy (e.g. 2-phenoxyethoxy) radical.

Aryloxy-1-4C-alkyl stands for one of the abovementioned 1-4C-alkyl radicals, which is substituted by one of the abovementioned aryloxy radicals. Examples which may be mentioned are the 2-aryloxyethyl (e.g. 2-phenoxyethyl) and the aryloxymethyl (e.g. phenoxymethyl) radicals.

Mono- or Di-1-4C-alkylaminocarbonyl radicals contain in addition to the carbonyl group one of the abovementioned mono- or di-1-4C-alkylamino radicals. Examples which may be mentioned are the N-methyl- the N,N-dimethyl-, the N-ethyl-, the N-propyl-, the N,N-diethyl- and the N-isopropylaminocarbonyl radical.

Het1 refers to a 5- to 7-membered saturated heterocyclic ring radical comprising one nitrogen atom, to which R611 and R612 are bound, and, optionally, one further heteroatom selected from a group consisting of nitrogen, oxygen and sulfur, and optionally substituted by R613 on a ring nitrogen atom. Examples for Het1 include e.g. piperidin-1-yl, 4-methyl-piperidin-1-yl, 4-hydroxypiperidin-1-yl, morpholin-4-yl, pyrrolidin-1-yl, piperazin-1-yl, imidazolidin-1-yl, thiomorpholin-4-yl, homopiperidin-1-yl, homopiperazin-1-yl, 4-N-(1-4C-alkyl)-homopiperazin-1-yl or piperazinyl substituted on a ring nitrogen atom by R613 [4-N-(R613)-piperazin-1-yl] such as, for example, 4-N-(1-4C-alkyl)-piperazin-1-yl, 4-N-(hydroxy-2-4C-alkyl)-piperazin-1-yl, 4-N-(dimethylamino-2-4C-alkyl)-piperazin-1-yl, 4-N-(3-6C-cycloalkyl)-piperazin-1-yl, 4-N-formyl-piperazin-1-yl, 4-N-(pyridin-4-yl)-piperazin-1-yl, 4-N-(pyrimidin-2-yl)-piperazin-1-yl or 4-N-(3-6C-cycloalkylmethyl)-piperazin-1-yl.

Har refers to a monocyclic or fused bicyclic 5- to 10-membered partially or fully aromatic heterocyclic ring or ring system comprising one to four, particularly one to three, heteroatoms, each of which is selected from a group consisting of nitrogen, oxygen and sulphur.
The Har radical is bonded via a ring carbon atom to the adjacent pyrroloisoquinoline scaffold.

In one embodiment (embodiment a) Har refers to a monocyclic 5-membered fully aromatic heteroaryl radical comprising one to four heteroatoms, each of which is selected from a group consisting of nitrogen, oxygen and sulphur,

Exemplary Har radicals according to embodiment a may include, without being restricted to, furanyl, thiophenyl, pyrrolyl, oxazolyl, isoxazolyl, thiazolyl, isothiazolyl, imidazolyl, pyrazolyl, triazolyl, thiadiazolyl or oxadiazolyl.

In another embodiment (embodiment b) Har refers to a monocyclic 6-membered fully aromatic heteroaryl radical comprising one or two nitrogen atoms.
Exemplary Har radicals according to embodiment b may include pyridinyl, pyrimidinyl, pyrazinyl or pyridazinyl.

A Har radical according to embodiment a worthy to be mentioned is pyridinyl, such as e.g. pyridin-4-yl.

In another embodiment (embodiment c) Har refers to a fused bicyclic 9- or 10-membered fully aromatic heteroaryl radical comprising one to four, in particular one to three, in more particular one or two, heteroatoms, each of which is selected from a group consisting of nitrogen, oxygen and sulphur.

Exemplary Har radicals according to embodiment c may include, without being restricted to, the benzo-fused analogues of the Har radicals mentioned exemplarily above in embodiment a or b, such as, for example, quinazolinyl, quinoxalinyl, cinnolinyl, quinolyl, isoquinolyl, indolyl, isoindolyl, indazolyl, benzothiophenyl, benzofuranyl, benzoxazolyl, benzothiazolyl or benzimidazolyl; or naphthyridinyl, phthalazinyl, imidazopyridinyl, purinyl, pteridinyl or imidazopyridazinyl.

The Har radicals according to embodiment c, which contain a benzene ring, can be attached to the parent molecular group via any ring carbon atom of the heteroatom containing ring or of the benzene ring.

Har radicals according to embodiment c worthy to be mentioned are indolyl, benzothiophenyl, or quinolinyl, such as e.g. indol-3-yl, benzothiophen-3-yl, or quinolin-4-yl.

In another embodiment (embodiment d) Har refers to a bicyclic partially aromatic heterocyclic radical made up of
a first constituent being a 5- or 6-membered monocyclic fully saturated heterocyclic ring, which heterocyclic ring comprises one or two heteroatoms independently selected from nitrogen, oxygen and sulphur,
and, fused to said first constituent,
a second constituent being benzene ring,
whereby said Har ring system is attached to the parent molecular group via any ring carbon atom of the benzene moiety.

Exemplary Har radicals according to embodiment d may include, without being restricted to, indolinyl, isoindolinyl, 1,2,3,4-tetrahydroquinolinyl, 1,2,3,4-tetrahydroisoquinolinyl, 1,3-benzodioxolyl, 2,3-dihydro-1,4-benzodioxinyl, 2,3-dihydrobenzothiophenyl, 2,3-ihydrobenzofuranyl, or chromanyl.

In another embodiment (embodiment e) Har refers to a stabile N-oxide derivative of any nitrogen-containing heteroaryl ring, particularly of any imino type nitrogen (=N-) containing heteroaryl ring, according to embodiment a or b.

Exemplary Har radicals according to embodiment d may include, without being restricted to, N-oxypyridinyl.

A Har radical according to embodiment c in particular worthy to be mentioned is 1N-oxy-pyridin-4-yl.

Naphthyl includes naphthalen-1-yl and naphthalen-2-yl.

N-(1-4C-alkyl)-piperazinyl stands for the piperazin-1-yl radical substituted by one of the abovementioned 1-4C-alkyl radicals on the 4N ring nitrogen atom.

The heterocyclic rings mentioned herein include, unless otherwise noted, all the possible isomeric forms thereof. Thus, for example, the heterocyclic rings mentioned herein include possible tautomers and the positional isomers thereof (such as e.g. the term pyridyl or pyridinyl includes pyridin-2-yl, pyridin-3-yl and pyridin-4-yl).

Constituents which are optionally substituted as stated herein, may be substituted, unless otherwise noted, at any possible position.
The substituents R1, R2 and/or R3 may be attached, unless otherwise noted, at any position of the benzo moiety of the pyrrolodihydroisoquinoline ring.
The given heterocyclic rings may be substituted, unless otherwise noted, by their substituents as mentioned herein at any possible position, such as e.g. at any substitutable ring carbon or ring nitrogen atom.

Heteroaryl rings containing imino-type ring nitrogen atoms (-N=) may be preferably not substituted (i.e. quaternized) on these imino-type ring nitrogen atoms by the mentioned substituents.

The substituents R71, R72 and/or R73 of the compounds according to this invention can be each attached in the ortho, meta or para position with respect to the binding position in which the phenyl ring is bonded to the pyrrolo moiety of the pyrrolodihydroisoquinoline ring, whereby in an embodiment of the present invention the attachement in the meta or in para position is to be emphasized.

When any variable occurs more than one time in any constituent, each definition is independent.

Suitable salts for compounds of the formula I - depending on substitution - are all acid addition salts or all salts with bases. Particular mention may be made of the pharmacologically tolerable inorganic and organic acids and bases customarily used in pharmacy. Those suitable are, on the one hand, water-insoluble and, particularly, water-soluble acid addition salts with adds such as, for example, hydrochloric acid, hydrobromic acid, phosphoric acid, nitric add, sulphuric acid, acetic acid, citric acid, D-gluconic acid, benzoic acid, 2-(4-hydroxybenzoyl)benzoic acid, butyric acid, sulphosalicylic add, maleic acid, lauric acid, malic acid, fumaric acid, succinic acid, oxalic acid, tartaric acid, embonic add, stearic acid, toluenesulphonic acid, methanesulphonic acid or 3-hydroxy-2-naphthoic acid, the acids being employed in salt preparation - depending on whether a mono- or polybasic acid is concerned and depending on which salt is desired - in an equimolar quantitative ratio or one differing therefrom.

On the other hand, salts with bases are - depending on substitution - also suitable. As examples of salts with bases are mentioned the lithium, sodium, potassium, calcium, aluminium, magnesium, titanium, ammonium, meglumine or guanidinium salts, here, too, the bases being employed in salt preparation in an equimolar quantitative ratio or one differing therefrom.

Pharmacologically intolerable salts, which can be obtained, for example, as process products during the preparation of the compounds of formula I according to the invention on an industrial scale, are converted into pharmacologically tolerable salts by processes known to the person skilled in the art.

According to expert's knowledge the compounds of formula I of the invention as well as their salts may contain, e.g. when isolated in crystalline form, varying amounts of solvents. Included within the scope of the invention are therefore all solvates and in particular all hydrates of the compounds of formula I as well as all solvates and in particular all hydrates of the salts of the compounds of formula I.

Depending on substitution the compounds of formula I can be chiral compounds having, for example, chiral centers and/or chiral axes due to hindered rotation about single bonds. Chiral axes can be present in particular in those compounds according to the invention, in which R7 is a bicyclic ring, or a monocyclic ring substituted in the ortho position with respect to the binding position in which said monocyclic ring is bonded to the pyrrolo[2.1-a]isoquinoline ring system. Chiral centers can be, for example, -depending on the meaning of R4, R41, R5 and R51- located at position 5 and/or 6 of the pyrrolo[2.1-a]isoquinolin scaffold. The invention therefore includes all conceivable pure diastereomers and pure enantiomers and mixtures thereof in any mixing ratio including the racemates, as well as the salts thereof. The diastereomer mixtures can be separated into the individual isomers by standard methods, e.g. by chromatographic processes. The enantiomers can be separated in a known manner (e.g. by chromatographic processes on chiral phases or by resolution).

Therefore, e.g. the pure (5R)- and the pure (5S)-enantiomers, as well as mixtures thereof in any mixing ratio including the racemates, and the salts thereof, are part of this invention.

Compounds according to the present invention more worthy to be mentioned are those compounds of formula I,
in which
- R1: is 1-4C-alkoxy, 3-7C-cycloalkoxy, 3-7C-cycloalkylmethoxy, or completely or predominantly fluorine-substituted 1-4C-alkoxy,
- R2: is hydrogen, halogen or 1-4C-alkoxy, and
- R3: is 1-4C-alkoxy, or
- R2 and R3: bound to the benzo ring moiety in ortho-position to each other together form a 1-2C-alkylenedioxy bridge, or
- R2 and R3: bound to the benzo ring moiety in ortho-position to each other together form a completely or predominantly fluorine-substituted 1-2C-alkylenedioxy bridge, or
- R1 and R2: bound to the benzo ring moiety in ortho-position to each other together form a 1-2C-alkylenedioxy bridge and R3 is hydrogen, or
- R1 and R2: bound to the benzo ring moiety in ortho-position to each other together form a completely or predominantly fluorine-substituted 1-2C-alkylenedioxy bridge and R3 is hydrogen,
and none of R1, R2 and R3 is bound to the 10-position of the pyrrolo[2.1-a]isoquinoline ring,
- R4: is hydrogen or 1-4C-alkyl,
- R41: is hydrogen or 1-4C-alkyl,
- R5: is hydrogen, 1-4C-alkyl, cyano or 1-4C-alkoxycarbonyl,
- R51: is hydrogen or 1-4C-alkyl,
or
- R4 and R5: together form a 3-4C-alkylene bridge and R41 and R51 are both hydrogen,
- R6: is 1-6C-alkyl, or 1-4C-alkyl substituted by R61, in which
- R61: is 1-4C-alkoxycarbonyl or carboxyl,
- R7: is Het2, R71- and/or R72- and/or R73-substituted phenyl, R74-substituted Het2, or naphthyl, in which
- Het2: is either
a monocyclic 5-membered heteroaryl radical comprising one to four heteroatoms, each of which is selected from a group consisting of nitrogen, oxygen and sulfur,
or
a monocyclic 6-membered heteroaryl radical comprising one or two nitrogen atoms,
or
a fused bicyclic 9- or 10-membered heteroaryl comprising one to three heteroatoms, each of which is selected from a group consisting of nitrogen, oxygen and sulfur,
or
N-oxy-pyridyl,
- R71: is hydroxyl, halogen, nitro, 1-4C-alkyl, 1-4C-alkoxy, amino, mono- or di-1-4C-alkylamino, 1-4C-alkylsulphonylamino, carboxyl, aryloxy, mono- or di-1-4C-alkylaminocarbonyl, carbamoyl, tetrazolyl, or -N(H)S(O)₂-N(R712)R713, in which
- aryl: is phenyl or R711-substituted phenyl, in which
- R711: is halogen or 1-4C-alkyl,
- R712: is 1-4C-alkyl, and
- R713: is 1-4C-alkyl, or
- R712 and R713: together and with inclusion of the nitrogen atom to which they are bound form a radical Het3, in which
- Het3: is morpholin-4-yl,
- R72: is halogen, 1-4C-alkyl or 1-4C-alkoxy,
- R73: is 1-4C-alkyl or 1-4C-alkoxy,
- R74: is 1-4C-alkyl, phenyl-1-4C-alkyl, arylsulphonyl, 1-4C-alkylsulphonyl, or -S(O)₂-N(R712)R713,
- R8: is optionally substituted by R81 on a ring carbon atom, and is Het4, in which
- Het4: is bonded to the pyrroloisoquinoline scaffold via a ring carbon atom, and is an [1,2,4]oxadiazolyl or an oxazolyl radical,
- R81: is 1-4C-alkyl, 3-7C-cycloalkyl, 3-7C-cycloalkylmethyl, phenyl, or R811- and/or R812-substituted phenyl, in which
- R811: is 1-4C-alkyl, 1-4C-alkoxy, or halogen,
- R812: is 1-4C-alkyl, 1-4C-alkoxy, or halogen,
and the stereoisomers as well as the salts of these compounds and stereoisomers.

Compounds according to the present invention further more worthy to be mentioned are those compounds of formula I,
in which
- R1: is bound to the 8-position of the pyrrolo[2.1-a]isoquinoline ring, and is 1-4C-alkoxy,
- R2: is bound to the 7-position of the pyrrolo[2.1-a]isoquinoline ring, and is hydrogen, halogen or 1-4C-alkoxy,
- R3: is bound to the 9-position of the pyrrolo[2.1-a]isoquinoline ring, and is 1-4C-alkoxy,
- R4: is hydrogen,
- R41: is hydrogen,
- R5: is hydrogen or 1-4C-alkyl,
- R51: is hydrogen or 1-4C-alkyl,
or
- R4 and R5: together form a tetramethylene bridge and R41 and R51 are both hydrogen,
- R6: is 1-4C-alkyl,
- R7: is Het2, R71- and/or R72- and/or R73-substituted phenyl, R74-substituted Het2, or naphthyl, in which
- Het2: is either
a monocyclic 5-membered heteroaryl radical comprising one to four heteroatoms, each of which is selected from a group consisting of nitrogen, oxygen and sulfur, or
a monocyclic 6-membered heteroaryl radical comprising one or two nitrogen atoms, such as, for example, pyridyl, e.g. pyridin-4-yl,
or
a fused bicyclic 9- or 10-membered heteroaryl comprising one to three heteroatoms, each of which is selected from a group consisting of nitrogen, oxygen and sulfur, such as, for example, quinolinyl, e.g. quinolin-4-yl,
or
N-oxy-pyridyl, such as e.g. 1N-oxy-pyridin-4-yl,
- R71: is hydroxyl, halogen, 1-4C-alkyl, 1-4C-alkoxy or aryloxy, in which
- aryl: is phenyl or R711-substituted phenyl, in which
- R711: is halogen or 1-4C-alkyl,
- R72: is halogen, 1-4C-alkyl or 1-4C-alkoxy,
- R73: is 1-4C-alkyl or 1-4C-alkoxy,
- R74: is 1-4C-alkyl or phonyl-1-4C-alkyl,
- R8: is substituted by R81 on a ring carbon atom, and is Het4, in which
- Het4: is bonded to the pyrroloisoquinoline scaffold via a ring carbon atom, and is an [1,2,4]oxadiazolyl or an oxazolyl radical,
- R81: is 1-4C-alkyl, 3-5C-cycloalkyl, 3-5C-cycloalkylmethyl, phenyl, or R811- and/or R812-substituted phenyl, in which
- R811: is 1-4C-alkyl, 1-4C-alkoxy, or halogen,
- R812: is 1-4C-alkyl, 1-4C-alkoxy, or halogen,
and the stereoisomers as well as the salts of these compounds and stereoisomers.

Yet compounds according to the present invention further more worthy to be mentioned are those compounds of formula **I,**
in which
- R1: is bound to the 8-position of the pyrrolo[2.1-a]isoquinoline ring, and is 1-4C-alkoxy,
- R2: is bound to the 7-position of the pyrrolo[2.1-a]isoquinoline ring, and is hydrogen, halogen or 1-4C-alkoxy,
- R3: is bound to the 9-position of the pyrrolo[2.1-a]isoquinoline ring, and is 1-4C-alkoxy,
- R4: is hydrogen,
- R41: is hydrogen,
- R5: is hydrogen or 1-4C-alkyl,
- R51: is hydrogen or 1-4C-alkyl,
or
- R4 and R5: together form a tetramethylene bridge and R41 and R51 are both hydrogen,
- R6: is 1-4C-alkyl, or 1-4C-alkyl substituted by R61, in which
- R61: is 1-4C-alkoxycarbonyl or carboxyl,
- R7: is Het2, R71- and/or R72- and/or R73-substituted phenyl, R74-substituted Het2, or naphthyl, in which
- Het2: is either
a monocyclic 5-membered heteroaryl radical comprising one to four heteroatoms, each of which is selected from a group consisting of nitrogen, oxygen and sulfur,
or
a monocyclic 6-membered heteroaryl radical comprising one or two nitrogen atoms, such as, for example, pyridyl, e.g. pyridin-4-yl,
or
a fused bicyclic 9- or 10-membered heteroaryl comprising one to three heteroatoms, each of which is selected from a group consisting of nitrogen, oxygen and sulfur, such as, for example, quinolinyl, e.g. quinolin-4-yl,
or
N-oxy-pyridyl, such as e.g. 1N-oxy-pyridin-4-yl,
- R71: is hydroxyl, halogen, 1-4C-alkyl, 1-4C-alkoxy, carboxyl or aryloxy, in which
- aryl: is phenyl or R711-substituted phenyl, in which
- R711: is halogen or 1-4C-alkyl,
- R72: is halogen, 14C-alkyl or 1-4C-alkoxy,
- R73: is 1-4C-alkyl or 1-4C-alkoxy,
- R74: is 1-4C-alkyl or phenyl-1-4C-alkyl,
- R8: is substituted by R81 on a ring carbon atom, and is Het4, in which
- Het4: is bonded to the pyrroloisoquinoline scaffold via a ring carbon atom, and is an [1,2,4]oxadiazolyl or an oxazolyl radical,
- R81: is 1-4C-alkyl, 3-5C-cycloalkyl, 3-5C-cycloalkylmethyl, phenyl, or R811- and/or R812-substituted phenyl, in which
- R811: is 1-4C-alkyl, 1-4C-alkoxy, or halogen,
- R812: is 1-4C-alkyl, 1-4C-alkoxy, or halogen,
and the stereoisomers as well as the salts of these compounds and stereoisomers.

Compounds according to the present invention in particular worthy to be mentioned are those compounds of formula I,
in which
- R1: is bound to the 8-position of the pyrrolo[2.1-a]isoquinoline ring, and is 1-2C-alkoxy,
- R2: is bound to the 7-position of the pyrrolo[2.1-a]isoquinoline ring, and is hydrogen, chlorine or fluorine,
- R3: is bound to the 9-position of the pyrrolo[2.1-a]isoquinoline ring, and is 1-2C-alkoxy,
- R4: is hydrogen,
- R41: is hydrogen,
- R5: is hydrogen or 1-2C-alkyl,
- R51: is hydrogen,
- R6: is 1-4C-alkyl,
- R7: is naphthyl, or
4-hydroxy-3,5-dimethylphonyl, 4-methoxy-3,5-dimethylphenyl, 2-methyl-4-hydroxy-phenyl or 2-fluoro-3,4-dimethoxy-phenyl,
pyridyl or quinolinyl, such as e.g. pyridin-4-yl or quinolin-4-yl, or
2-methyl-pyridin-4-yl or 3-methyl-pyridin-4-yl,
- R8: is Het4, in which
- Het4: is 3-(R81)-[1,2,4]oxadiazol-5-yl or 5-(R81)-oxazol-2-yl,
- R81: is 1-4C-alkyl, 3-5C-cycloalkyl, 3-5C-cycloalkylmethyl, phenyl, or R811-substituted phenyl, in which
- R811: is 1-4C-alkyl, 1-4C-alkoxy, or halogen,
and the stereoisomers as well as the salts of these compounds and stereoisomers.

Yet compounds according to the present invention in particular worthy to be mentioned are those compounds of formula I,
in which
- R1: is bound to the 8-position of the pyrrolo[2.1-a]isoquinoline ring, and is 1-2C-alkoxy,
- R2: is bound to the 7-position of the pyrrolo[2.1-a]isoquinoline ring, and is hydrogen, chlorine or fluorine,
- R3: is bound to the 9-position of the pyrrolo[2.1-a]isoquinoline ring, and is 1-2C-alkoxy,
- R4: is hydrogen,
- R41: is hydrogen,
- R5: is hydrogen or 1-2C-alkyl,
- R51: is hydrogen,
- R6: is 1-2C-alkyl, or 1-2C-alkyl substituted by R61, in which
- R61: is 1-2C-alkoxycarbonyl or carboxyl,
- R7: is Het2, R71- and/or R72- and/or R73-substituted phenyl, R74-substituted Het2, or naphthyl, in which
- Het2: is either
a monocyclic 5-membered heteroaryl radical comprising one to four heteroatoms, each of which is selected from a group consisting of nitrogen, oxygen and sulfur,
or
a monocyclic 6-membered heteroaryl radical comprising one or two nitrogen atoms, such as, for example, pyridyl, e.g. pyridin-4-yl,
or
a fused bicyclic 9- or 10-membered heteroaryl comprising one to three heteroatoms, each of which is selected from a group consisting of nitrogen, oxygen and sulfur, such as, for example, quinolinyl, e.g. quinolin-4-yl,
or
N-oxy-pyridyl, such as e.g. 1N-oxy-pyridin-4-yl,
- R71: is hydroxyl, chlorine, fluorine, 1-2C-alkyl, 1-2C-alkoxy, carboxyl or aryloxy, in which
- aryl: is phenyl or R711-substituted phenyl, in which
- R711: is chlorine, fluorine or 1-2C-alkyl,
- R72: is chlorine, fluorine, 1-2C-alkyl or 1-2C-alkoxy,
- R73: is 1-2C-alkyl or 1-2C-alkoxy,
- R74: is 1-2C-alkyl or phenyl-1-2C-alkyl,
- R8: is Het4, in which
- Het4: is 3-(R81)-[1,2,4]oxadiazol-5-yl or 5-(R81)-oxazol-2-yl,

- R81: is 1-4C-alkyl, 3-5C-cycloalkyl, 3-5C-cycloalkylmethyl, phenyl, or R811-substituted phenyl, in which
- R811: is 1-2C-alkyl, 1-2C-alkoxy, chlorine or fluorine,
and the stereoisomers as well as the salts of these compounds and stereoisomers.

Compounds according to the present invention in more particular worthy to be mentioned are those compounds of formula I,
in which
- R1: is bound to the 8-position of the pyrrolo[2.1-a]isoquinoline ring, and is methoxy,
- R2: is bound to the 7-position of the pyrrolo[2.1-a]isoquinoline ring, and is hydrogen, chlorine or fluorine,
- R3: is bound to the 9-position of the pyrrolo[2.1-a]isoquinoline ring, and is methoxy,
- R4: is hydrogen,
- R41: is hydrogen,
- R5: is hydrogen, methyl or ethyl,
- R51: is hydrogen,
- R6: is methyl,
- R7: is naphthyl, or
4-hydroxy-3,5-imethylphenyl, 4-methoxy-3,5-dimethylphenyl, 2-methyl-4-hydroxy-phenyl or 2-fluoro-3,4-dimethyloxy-phenyl,
pyridyl or quinolinyl, such as e.g. pyridin-4-yl or quinolin-4-yl, or
2-methyl-pyridin-4-yl or 3-methyl-pyridin-4-yl,
- R8: is Het4, in which
- Het4: is 3-(R81)-[1,2,4]oxadiazol-5-yl or 5-(R81)-oxazol-2-yl,
- R81: is methyl, ethyl, cyclopropyl or phenyl,
and the stereoisomers as well as the salts of these compounds and stereoisomers.

Yet compounds according to the present invention in more particular worthy to be mentioned are those compounds of formula I,
in which
- R1: is bound to the 8-position of the pyrrolo[2.1-a]isoquinoline ring, and is methoxy,
- R2: is bound to the 7-position of the pyrrolo[2.1-a]isoquinoline ring, and is hydrogen, chlorine or fluorine,
- R3: is bound to the 9-position of the pyrrolo[2.1-a]isoquinoline ring, and is methoxy,
- R4: is hydrogen,
- R41: is hydrogen,
- R5: is hydrogen, methyl or ethyl,
- R51: is hydrogen,
- R6: is methyl, ethyl or 2-methoxycarbonylethyl,
- R7: is naphthyl, or
4-hydroxy-3,5-dimethylphenyl, 4-methoxy-3,5-dimethylphenyl, 2-methyl-4-hydroxy-phenyl, 2-fluoro-3,4-dimethoxy-phenyl or 4-carboxy-phenyl,
pyridyl or quinolinyl, such as e.g. pyridin-4-yl or quinolin-4-yl, or
2-methyl-pyridin-4-yl or 3-methyl-pyridin-4-yl,
- R8: is Het4, in which
- Het4: is 3-(R81)-[1,2,4]oxadiazol-5-yl or 5-(R81)-oxazol-2-yl,
- R81: is methyl, ethyl, cyclopropyl or phenyl,
and the stereoisomers as well as the salts of these compounds and stereoisomers.

As exemplary compounds according to the present invention the following compounds of formula la 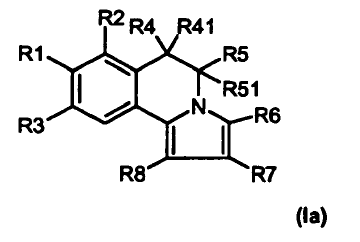 in which
- R1: is methoxy,
- R2: is hydrogen,
- R3: is methoxy,
- R4: is hydrogen,
- R41: is hydrogen,
- R51: is hydrogen,
- R6: is methyl,
and the stereoisomers as well as the salts of these compounds and stereoisomers,
may be mentioned by means of the substituent meanings for R5, R7 and R8 shown in the Table 1 given below.

As further exemplary compounds according to the present invention the following compounds of formula la
in which
- R1: is methoxy,
- R2: is fluorine,
- R3: is methoxy,
- R4: is hydrogen,
- R41: is hydrogen,
- R51: is hydrogen,
- R6: is methyl,
and the stereoisomers as well as the salts of these compounds and stereoisomers, may be mentioned by means of the substituent meanings for R5, R7 and R8 shown in the Table 1 given below.

As further exemplary compounds according to the present invention the following compounds of formula la
in which
- R1: is methoxy,
- R2: is chlorine,
- R3: is methoxy,
- R4: is hydrogen,
- R41: is hydrogen,
- R51: is hydrogen,
- R6: is methyl,
and the stereoisomers as well as the salts of these compounds and stereoisomers, may be mentioned by means of the substituent meanings for R5, R7 and R8 shown in the Table 1 given below.

**Table 1:**

| R5 | R7 | R8 |
|---|---|---|
| methyl | 4-hydroxy-3,5-dimethylphenyl | 3-methyl-[1,2,4]oxadiazol-5-yl |
| methyl | 4-methoxy-3,5-dimethylphenyl | 3-methyl-[1,2,4]oxadiazol-5-yl |
| methyl | 4-carboxy-phenyl | 3-methyl-[1,2,4]oxadiazol-5-yl |
| methyl | 2-methyl-4-hydroxy-phenyl | 3-methyl-[1,2,4]oxadiazol-5-yl |
| methyl | 4-amino-phenyl | 3-methyl-[1,2,4]oxadiazol-5-yl |
| methyl | 4-(2H-tetrazol-5-yl)-phenyl | 3-mothyl-[1,2,4]oxadiazol-5-yl |
| methyl | 4-morpholino-sulphonylamino-phenyl | 3-methyl-[1,2,4]oxadiazol-5-yl |
| methyl | 4-methylsulphonylamino-phenyl | 3-methyl-[1,2,4]oxadiazol-5-yl |
| methyl | pyridin-4-yl | 3-methyl-[1,2,4]oxadiazol-5-yl |
| methyl | quinolin-4-yl | 3-methyl-[1,2,4]oxadiazol-5-yl |
| methyl | 2-methyl-pyridin-4-yl | 3-methyl-[1,2,4]oxadiazol-5-yl |
| methyl | 3-methyl-pyridin-4-yl | 3-methyl-[1,2,4]oxadiazol-5-yl |
| methyl | 1-tolylsulphonyl-pyrrol-3-yl | 3-methyl-[1,2,4]oxadiazol-5-yl |
| methyl | 1-tolylsulphonyl-indol-3-yl | 3-methyl-[1,2,4]oxadiazol-5-yl |
| methyl | 1-phenylsulphonyl-indol-3-yl | 3-methyl-[1,2,4]oxadiazol-5-yl |
| methyl | 1-methylsulphonyl-indol-3-yl | 3-methyl-[1,2,4]oxadiazol-5-yl |
| methyl | 1-dimethylaminosulphonyl-indol-3-yl | 3-methyl-[1,2,4]oxadiazol-5-yl |
| methyl | 1-morpholinosulphonyl-indol-3-yl | 3-methyl-[1,2,4]oxadiazol-5-yl |
| hydrogen | 4-hydroxy-3,5-dimethylphenyl | 3-methyl-[1,2,4]oxadiazol-5-yl |
| hydrogen | 4-methoxy-3,5-dimethylphenyl | 3-methyl-[1,2,4]oxadiazol-5-yl |
| hydrogen | 4-carboxy-phenyl | 3-methyl-[1,2,4]oxadiazol-5-yl |
| hydrogen | 2-methyl-4-hydroxy-phenyl | 3-methyl-[1,2,4]oxadiazol-5-yl |
| hydrogen | 4-amino-phenyl | 3-methyl-[1,2,4]oxadiazol-5-yl |
| hydrogen | 4-(2H-tetrazol-5-yl)-phenyl | 3-methyl-[1,2,4]oxadiazol-5-yl |
| hydrogen | 4-morpholino-sulphonylamino-phenyl | 3-methyl-[1,2,4]oxadiazol-5-yl |
| hydrogen | 4-methylsulphonylamino-phenyl | 3-methyl-[1,2,4]oxadiazol-5-yl |
| hydrogen | pyridin-4-yl | 3-methyl-[1,2,4]oxadiazol-5-yl |
| hydrogen | quinolin-4-yl | 3-methyl-[1,2,4]oxadiazol-5-yl |
| hydrogen | 2-methyl-pyridin-4-yl | 3-methyl-[1,2,4]oxadiazol-5-yl |
| hydrogen | 3-methyl-pyridin-4-yl | 3-methyl-[1,2,4]oxadiazol-5-yl |
| hydrogen | 1-tolylsulphonyl-pyrrol-3-yl | 3-methyl-[1,2,4]oxadiazol-5-yl |
| hydrogen | 1-tolysulphonyl-indol-3-yl | 3-methyl-[1,2,4]oxadiazol-5-yl |
| hydrogen | 1-phenylsulphonyl-indol-3-yl | 3-methyl-[1,2,4]oxadiazol-5-yl |
| hydrogen | 1-methylsulphonyl-indol-3-yl | 3-methyl-[1,2,4]oxadiazol-5-yl |
| hydrogen | 1-dimethylaminosulphonyl-indol-3-yl | 3-methyl-[1,2,4]oxadiazol-5-yl |
| hydrogen | 1-morpholinosulphonyl-indol-3-yl | 3-methyl-[1,2,4]oxadiazol-5-yl |
| methyl | 4-hydroxy-3,5-dimethylphenyl | 3-cyclopropyl-[1,2,4]oxadiazol-5-yl |
| methyl | 4-methoxy-3,5-dimethylphenyl | 3-cycloprropyl-[1,2,4]oxadiazol-5-yl |
| methyl | 4-carboxy-phenyl | 3-cyclopropyl-[1,2,4]oxadiazol-5-yl |
| methyl | 2-methyl-4-hydroxy-phenyl | 3-cyclopropyl-[1,2,4]oxadiazol-5-yl |
| methyl | 4-amino-phenyl | 3-cyclopropyl-[1,2,4]oxadiazol-5-yl |
| methyl | 4-(2H-tetrazol-5-yl)-phenyl | 3-cyclopropyl-[1,2,4]oxadiazol-5-yl |
| methyl | 4-morpholino-sulphonylamino-phenyl | 3-cyclopropyl-[1,2,4]oxadiazol-5-yl |
| methyl | 4-methylsulphonylamino-phenyl | 3-cyclopropyl-[1,2,4]oxadiazol-5-yl |
| methyl | pyridin-4-yl | 3-cyclopropyl-[1,2,4]oxadiazol-5-yl |
| methyl | quinolin-4-yl | 3-cyclopropyl-[1,2,4]oxadiazol-5-yl |
| methyl | 2-methyl-pyridin-4-yl | 3-cyclopropyl-[1,2,4]oxadiazol-5-yl |
| methyl | 3-methyl-pyridin-4-yl | 3-cyclopropyl-[1,2,4]oxadiazol-5-yl |
| methyl | 1-tolylsulphonyl-pyrrol-3-yl | 3-cyclopropyl-[1,2,4]oxadiazol-5-yl |
| methyl | 1-tolysulphonyl-indol-3-yl | 3-cyclopropyl-[1,2,4]oxadiazol-5-yl |
| methyl | 1-phenylsulphonyl-indol-3-yl | 3-cyclopropyl-[1,2,4]oxadiazol-5-yl |
| methyl | 1-methylsulphonyl-indol-3-yl | 3-cyclopropyl-[1,2,4]oxadiazol-5-yl |
| methyl | 1-dimethylaminosulphonyl-indol-3-yl | 3-cyclopropyl-[1,2,4]oxadiazol-5-yl |
| methyl | 1-morpholinosulphonyl-indol-3-yl | 3-cyclopropyl-[1,2,4]oxadiazol-5-yl |
| hydrogen | 4-hydroxy-3,5-dimethylphenyl | 3-cyclopropyl[1,2,4]oxadiazol-5-yl |
| hydrogen | 4-methoxy-3,5-dimethylphenyl | 3-cyclopropyl[1,2,4]oxadiazol-5-yl |
| hydrogen | 4-carboxy-phenyl | 3-cyclopropyl[1,2,4]oxadiazol-5-yl |
| hydrogen | 2-methyl-4-hydroxy-phenyl | 3-cyclopyropyl-[1,2,4]oxadiazol-5-yl |
| hydrogen | 4-amino-phenyl | 3-cyclopropyl-[1,2,4]oxadiazol-5-yl |
| hydrogen | 4-(2H-tetrazol-5-yl)-phenyl | 3-cyclopropyl-[1,2,4]oxadiazol-5-yl |
| hydrogen | 4-morpholino-sulphonylamino-phenyl | 3-cyclopropyl-[1,2,4]oxadiazol-5-yl |
| hydrogen | 4-methylsulphonylamino-phenyl | 3-cyclopropyl-[1,2,4]oxadiazol-5-yl |
| hydrogen | pyridin-4-yl | 3-cyclopropyl-[1,2,4]oxadiazol-5-yl |
| hydrogen | quinolin-4-yl | 3-cyclopropyl-[1,2,4]oxadiazol-5-yl |
| hydrogen | 2-methyl-pyridin-4-yl | 3-cyclopropyl-[1,2,4]oxadiazol-5-yl |
| hydrogen | 3-methyl-pyridin-4-yl | 3-cyclopropyl-[1,2,4]oxadiazol-5-yl |
| hydrogen | 1-tolylsulphonyl-pylrrol-3-yl | 3-cyclopropyl-[1,2,4]oxadiazol-5-yl |
| hydrogen | 1-tolylsulphonyl-indol-3-yl | 3-cyclopropyl-[1,2,4]oxadiazol-5-yl |
| hydrogen | 1-phenylsulphonyl-indol-3-yl | 3-cyclopropyl-[1,2,4]oxadiazol-5-yl |
| hydrogen | 1-methylsulphonyl-indol-3-yl | 3-cyclopropyl-[1,2,4]oxadiazol-5-yl |
| hydrogen | 1-dimethylaminosulphonyl-indol-3-yl | 3-cyclopropyl-[1,2,4]oxadiazol-5-yl |
| hydrogen | 1-morpholinosulphonyl-indol-3-yl | 3-cyclopropyl-[1,2,4]oxadiazol-5-yl |
| methyl | 4-hydroxy-3,5-dimethylphenyl | 3-phenyl-[1,2,4]oxadiazol-5-yl |
| methyl | 4-methoxy-3,5-dimethylphenyl | 3-phenyl-[1,2,4]oxadiazol-5-yl |
| methyl | 4-carboxy-phenyl | 3-phenyl-[1,2,4]oxadiazol-5-yl |
| methyl | 2-methyl-4-hydroxy-phenyl | 3-phenyl-[1,2,4]oxadiazol-5-yl |
| methyl | 4-amino-phenyl | 3-phenyl-[1,2,4]oxadiazol-5-yl |
| methyl | 4-(2H-tetrazol-5-yl)-phenyl | 3-phenyl-[1,2,4]oxadiazol-5-yl |
| methyl | 4-morpholino-sulphonylamino-phenyl | 3-phenyl-[1,2,4]oxadiazol-5-yl |
| methyl | 4-methylsulphonylamino-phenyl | 3-phenyl-[1,2,4]oxadiazol-5-yl |
| methyl | pyridin-4-yl | 3-phenyl-[1,2,4]oxadiazol-5-yl |
| methyl | quinolin-4-yl | 3-phenyl-[1,2,4]oxadiazol-5-yl |
| methyl | 2-methyl-pyridin-4-yl | 3-phenyl-[1,2,4]oxadiazol-5-yl |
| methyl | 3-methyl-pyridin-4-yl | 3-phenyl-[1,2,4]oxadiazol-5-yl |
| methyl | 1-tolylsulphonyl-pyrrol-3-yl | 3-phenyl-[1,2,4]oxadiazol-5-yl |
| methyl | 1-tolylsulphonyl-indol-3-yl | 3-phenyl-[1,2,4]oxadiazol-5-yl |
| methyl | 1-phenylsulphonyl-indol-3-yl | 3-phenyl-[1,2,4]oxadiazol-5-yl |
| methyl | 1-methylsulphonyl-indol-3-yl | 3-phenyl-[1,2,4]oxadiazol-5-yl |
| methyl | 1-dimethylaminosulphonyl-indol-3-yl | 3-phenyl-[1,2,4]oxadiazol-5-yl |
| methyl | 1-morpholinosulphonyl-indol-3-yl | 3-phenyl-[1,2,4]oxadiazol-5-yl |
| hydrogen | 4-hydroxy-3,5-dimethylphenyl | 3-phenyl-[1,2,4]oxadiazol-5-yl |
| hydrogen | 4-methoxy-3,5-dimethylphenyl | 3-phenyl-[1,2,4]oxadiazol-5-yl |
| hydrogen | 4-carboxy-phenyl | 3-phenyl-[1,2,4]oxadiazol-5-yl |
| hydrogen | 2-methyl-4-hydroxy-phenyl | 3-phenyl-[1,2,4]oxadiazol-5-yl |
| hydrogen | 4-amino-phenyl | 3-phenyl-[1,2,4]oxadiazol-5-yl |
| hydrogen | 4-(2H-tetrazol-5-yl)-phenyl | 3-phenyl-[1,2,4]oxadiazol-5-yl |
| hydrogen | 4-morpholino-sulphonylamino-phenyl | 3-phenyl-[1,2,4]oxadiazol-5-yl |
| hydrogen | 4-methylsulphonylamino-phenyl | 3-phenyl-[1,2,4]oxadiazol-5-yl |
| hydrogen | pyridin-4-yl | 3-phenyl-[1,2,4]oxadiazol-5-yl |
| hydrogen | quinolin-4-yl | 3-phenyl-[1,2,4]oxadiazol-5-yl |
| hydrogen | 2-methyl-pyridin-4-yl | 3-phenyl-[1,2,4]oxadiazol-5-yl |
| hydrogen | 3-methyl-pyridin-4-yl | 3-phenyl-[1,2,4]oxadiazol-5-yl |
| hydrogen | 1-tolylsulphonyl-pyrrol-3-yl | 3-phenyl-[1,2,4]oxadiazol-5-yl |
| hydrogen | 1-tolylsulphonyl-indol-3-yl | 3-phenyl-[1,2,4]oxadiazol-5-yl |
| hydrogen | 1-phenylsulphonyl-indol-3-yl | 3-phenyl-[1,2,4]oxadiazol-5-yl |
| hydrogen | 1-methylsulphonyl-indol-3-yl | 3-phenyl-[1,2,4]oxadiazol-5-yl |
| hydrogen | 1-dimethylaminosulphonyl-indol-3-yl | 3-phenyl-[1,2,4]oxadiazol-5-yl |
| hydrogen | 1-morpholinosulphonyl-indol-3-yl | 3-phenyl-[1,2,4]oxadiazol-5-yl |

Particular exemplary compounds according to the present invention may include, without being restricted thereto, any compound selected from
(5RS)-4-[8,9-Dimethoxy-3,5-dimethyl-1-(3-methyl-[1,2,4]oxadiazol-5-yl)-5,6-dihydro-pyrrolo[2,1-a]isoquinolin-2-yl]-2,6-dimethyl-phenol,
(SRS)-4-[1-(3-Cyclopropyl-[1,2,4]oxadiazol-5-yl)-8,9-dimethoxy-3,5-dimethyl-5,6-dihydro-pyrrolo[2,1-a]isoquinolin-2-yl]-2,6-dimethyl-phenol,
(5RS)-4-[8,9-Dimethoxy-3,5-dimethyl-1-(3-phenyl-[1,2,4]oxadiazol-5-yl)-5,6-dihydro-pyrrolo[2,1-a]isoquinolin-2-yl]-2,6-dimethyl-phenol,
4-[8,9-Dimethoxy-3-methyl-1-(3-phenyl-[1,2,4]oxadiazol-5-yl)-5,6-dihydro-pyrrolo[2,1-a]isoquinolin-2-yl]-2,6-dimethyl-phenol,
4-[8,9-Dimethoxy-3-methyl-1-(3-methyl-[1,2,4]oxadiazol-5-yl)-5,6-dihydro-pyrrolo[2,1-a]isoquinolin-2-yl]-2,6-dimethyl-phenol and
4-[8,9-Dimethoxy-3-methyl-1-(5-methyl-oxazol-2-yl)-5,6-dihydro-pyroolo[2,1-a]isoquinolin-2-yl]-2,6-dimethyl-phenol,
and the salts, stereoisomers and the salts of the stereoisomers thereof.

A special interest in the compounds according to this invention refers to those compounds of formula I which are included -within the scope of this invention- by one or, when possible, by more of the following special embodiments:

A special embodiment (embodiment 1) of the compounds according to this invention refers to those compounds of formula I, in which
none of R1, R2 and R3 is bound to the 10-position of the pyrrolo[2.1-a]isoquinoline ring.

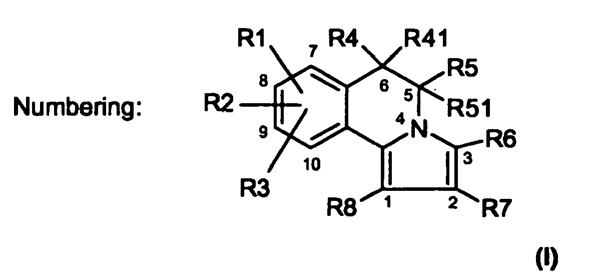

Another special embodiment (embodiment 2) of the compounds according to this invention refers to those compounds of formula I, in which
- R1: is bound to the 8-position of the pyrrolo[2.1-a]isoquinoline ring, and is 1-2C-alkoxy,
- R2: is bound to the 7-position of the pyrrolo[2.1-a]isoquinoline ring, and is hydrogen, and
- R3: is bound to the 9-position of the pyrrolo[2.1-a]isoquinoline ring, and is 1-2C-alkoxy.

Another special embodiment (embodiment 3) of the compounds according to this invention refers to those compounds of formula I, in which
- R1: is bound to the 8-position of the pyrroto[2.1-a]isoquinoline ring, and is 1-2C-alkoxy,
- R2: is bound to the 7-position of the pyrrolo[2.1-a]isoquinoline ring, and is chlorine or fluorine, and
- R3: is bound to the 9-position of the pyrrolo[2.1-a]isoquinoline ring, and is 1-2C-alkoxy.

Another special embodiment (embodiment 4) of the compounds according to this invention refers to those compounds of formula I, in which
- R1: is bound to the 8-position of the pyrrolo[2.1-a]isoquinoline ring, and is methoxy,
- R2: is bound to the 7-position of the pyrrolo[2.1-a]isoquinoline ring, and is chlorine, and
- R3: is bound to the 9-position of the pyrrolo[2.1-a]isoquinoline ring, and is methoxy.

Another special embodiment (embodiment 5) of the compounds according to this invention refers to those compounds of formula I, in which
- R1: is bound to the 8-position of the pyrrolo[2.1-a]isoquinoline ring, and is methoxy,
- R2: is bound to the 7-position of the pyrrolo[2.1-a]isoquinoline ring, and is fluorine, and
- R3: is bound to the 9-position of the pyrrolo[2.1-a]isoquinoline ring, and is methoxy.

Another special embodiment (embodiment 6) of the compounds according to this invention refers to those compounds of formula I, in which
- R1: is bound to the 8-position of the pyrrolo[2.1-a]isoquinoline ring, and is methoxy,
- R2: is bound to the 7-position of the pyrrolo[2.1-a]isoquinoline ring, and is hydrogen, and
- R3: is bound to the 9-poswon of the pyrrolo[2.1-a]isoquinoline ring, and is methoxy.

Another special embodiment (embodiment 7) of the compounds according to this invention refers to those compounds of formula I, in which
- R4: is hydrogen,
- R41: is hydrogen,
- R5: is methyl, and
- R51: is hydrogen.

Another special embodiment (embodiment 8) of the compounds according to this invention refers to those compounds of formula I, in which
- R6: is 1-4C-alkyl, such as e.g. methyl.

Another special embodiment (embodiment 9) of the compounds according to this invention refers to those compounds of formula I, in which
- R7: is naphthyl, or
4-hydroxy-3,5-dimethylphenyl, 4-methoxy-3,5-dimethylphenyl, 2-methyl-4-hydroxy-phenyl or 2-fluoro-3,4-dimethoxy-phenyl,
pyridyl or quinolinyl, such as e.g. pyridin-4-yl or quinolin-4-yl, or
2-methyl-pyridin-4-yl or 3-methyl-pyridin-4-yl.

Another special embodiment (embodiment 10) of the compounds according to this invention refers to those compounds of formula I, in which
- R7: is 4-carboxy-phenyl.

Another special embodiment (embodiment 11) of the compounds according to this invention refers to those compounds of formula I, in which
- R8: is 3-(R81)-[1,2,4]oxadiazol-5-yl or 5-(R81)-oxazol-2-yl,
- R81: is methyl, cyclopropyl or phenyl,
such as, for example,
- R8: is 3-methyl-[1,2,4]oxadiazol-5-yl, 3-cyclopropyl-[1,2,4]oxadiazol-5-yl or 3-phenyl-[1,2,4]oxadiazol-5-yl, or 5-methyl-oxazol-2-yl.

Another special embodiment (embodiment 12) of the compounds according to this invention refers to those compounds of formula I, in which
- R8: is 3-methyl-[1,2,4]oxadiazol-5-yl.

Another special embodiment (embodiment 13) of the compounds according to this invention refers to those compounds of formula I, in which
- R8: is 3-cyclopropyl-[1,2,4]oxadiazol-5-yl.

Another special embodiment (embodiment 14) of the compounds according to this invention refers to those compounds of formula I, in which
- R8: is 3-phenyl-[1,2,4]oxadiazol-5-yl.

Another special embodiment (embodiment 15) of the compounds according to the present invention refers to those compounds of formula I,
in which
- R1: is bound to the 8-position of the pyrrolo[2.1-a]isoquinoline ring, and is 1-2C-alkoxy,
- R2: is bound to the 7-position of the pyrrolo[2.1-a]isoquinoline ring, and is hydrogen, chlorine or fluorine,
- R3: is bound to the 9-position of the pyrrolo[2.1-a]isoquinoline ring, and is 1-2C-alkoxy,
and
- R4: is hydrogen,
- R41: is hydrogen,
- R5: is hydrogen or 1-2C-alkyl,
- R51: is hydrogen.

Another special embodiment (embodiment 16) of the compounds according to the present invention refers to those compounds of formula I,
in which
- R1: is bound to the 8-position of the pyrrolo[2.1-a]isoquinoline ring, and is 1-2C-alkoxy, such as e.g. methoxy,
- R2: is bound to the 7-position of the pyrrolo[2.1-a]isoquinoline ring, and is chlorine or fluorine,
- R3: is bound to the 9-position of the pyrrolo[2.1-a]isoquinoline ring, and is 1-2C-alkoxy, such as e.g. methoxy,
and
- R4: is hydrogen,
- R41: is hydrogen,
- R5: is 1-2C-alkyl, such as e.g. methyl,
- R51: is hydrogen.

Another special embodiment (embodiment 17) of the compounds according to the present invention refers to those compounds of formula I,
in which
- R1: is bound to the 8-position of the pyrrolo[2.1-a]isoquinoline ring, and is 1-2C-alkoxy, such as e.g. methoxy,
- R2: is bound to the 7-position of the pyrrolo[2.1-a]isoquinoline ring, and is fluorine,
- R3: is bound to the 9-position of the pyrrolo[2.1-a]isoquinoline ring, and is 1-2C-alkoxy, such as e.g. methoxy,
and
- R4: is hydrogen,
- R41: is hydrogen,
- R5: is methyl,
- R51: is hydrogen.

Another special embodiment (embodiment 18) of the compounds according to the present invention refers to those compounds of formula I,
in which
- R1: is bound to the 8-position of the pyrrolo[2.1-a]isoquinoline ring, and is 1-2C-alkoxy,
- R: is bound to the 7-position of the pyrrolo[2.1-a]isoquinoline ring, and is hydrogen, chlorine or fluorine,
- R3: is bound to the 9-position of the pyrrolo[2.1-a]isoquinoline ring, and is 1-2C-alkoxy,
and
- R4: is hydrogen,
- R41: is hydrogen,
- R5: is hydrogen or methyl,
- R51: is hydrogen,
and
- R8: is Het4, in which
- Het4: is 3-(R81)-[1,2,4]oxadiazol-5-yl or 5-(R81)-oxazol-2-yl,
- R81: is methyl, cyclopropyl or phenyl.

Another special embodiment (embodiment 19) of the compounds according to the present invention refers to those compounds of formula I,
in which
- R1: is bound to the 8-position of the pyrrolo[2.1-a]isoquinoline ring, and is 1-2C-alkoxy, such as e.g. methoxy,
- R2: is bound to the 7-position of the pyrrolo[2.1-a]isoquinoline ring, and is chlorine or fluorine,
- R3: is bound to the 9-position of the pyn-olo[2.1-a]isoquinoline ring, and is 1-2C-alkoxy, such as e.g. methoxy,
and
- R4: is hydrogen,
- R41: is hydrogen,
- R5: is 1-2C-alkyl, such as e.g. methyl,
- R51: is hydrogen,
and
- R8: is Het4, in which
- Het4: is 3-(R81)-[1,2,4]oxadiazol-5-yl or 5-(R81)-xazol-2-yl,
- R81: is methyl, cyclopropyl or phenyl.

Another special embodiment (embodiment 20) of the compounds according to the present invention refers to those compounds of formula I,
in which
- R1: is bound to the 8-position of the pyrrolo[2.1-a]isoquinoline ring, and is 1-2C-alkoxy, such as e.g. methoxy,
- R2: is bound to the 7-position of the pyrrolo[2.1-a]isoquinoline ring, and is fluorine,
- R3: is bound to the 9-position of the pyrrolo[2.1-a]isoquinoline ring, and is 1-2C-alkoxy, such as e.g. methoxy,
and
- R4: is hydrogen,
- R41: is hydrogen,
- R5: is methyl,
- R51: is hydrogen,
and
- R8: is Het4, in which
- Het4: is 3-(R81)-[1,2,4]oxadiazol-5-yl or 5-(R81)-oxazol-2-yl,
- R81: is methyl, cyclopropyl or phenyl.

Another special embodiment (embodiment 21) of the compounds according to this invention refers to those compounds of formula la 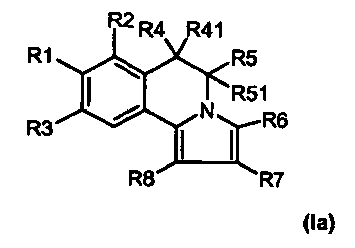 in which
- R1: is 1-2C-alkoxy, such as e.g. methoxy,
- R3: is 1-2C-alkoxy, such as e.g. methoxy,
- R4: is hydrogen,
- R41: is hydrogen,
- R51: is hydrogen,
and in which the following combinations 1.) to 6.) of the substituent meanings for R2, R5 and R6 shown in Table X apply:

**Table X:**

| | R2 | R5 | R6 |
|---|---|---|---|
| 1.) | hydrogen | methyl | methyl |
| 2.) | hydrogen | hydrogen | methyl |
| 3.) | fluorine | methyl | methyl |
| 4.) | fluorine | hydrogen | methyl |
| 5.) | chlorine | methyl | methyl |
| 6.) | fluorine | hydrogen | methyl |

Another special embodiment (embodiment 22) of the compounds according to this invention refers to those compounds of formula la as shown above, in which
- R1: is methoxy,
- R3: is methoxy,
- R4: is hydrogen,
- R41: is hydrogen,
- R51: is hydrogen,
and in which the following combinations 1.) to 18.) of the substituent meanings for R2, R5, R6 and R8 shown in Table Y apply:

**Table Y:**

| | R2 | R5 | R6 | R8 |
|---|---|---|---|---|
| 1.) | hydrogen | methyl | methyl | 3-methyl-[1,2,4]oxadiazol-5-yl |
| 2.) | hydrogen | methyl | methyl | 3-cyclopropyl-[1,2,4]oxadiazol-5-yl |
| 3.) | hydrogen | methyl | methyl | 3-phenyl-[1,2,4]oxadiazol-5-yl |
| 4.) | hydrogen | hydrogen | methyl | 3-methyl-[1,2,4]oxadiazol-5-yl |
| 5.) | hydrogen | hydrogen | methyl | 3-cyclopropyl-[1,2,4]oxadiazol-5-yl |
| 6.) | hydrogen | hydrogen | methyl | 3-phenyl-[1,2,4]oxadiazol-5-yl |
| 7.) | fluorine | methyl | methyl | 3-methyl-[1,2,4]oxadiazol-5-yl |
| 8.) | fluorine | methyl | methyl | 3-cyclopropyl-[1,2,4]oxadiazol-5-yl |
| 9.) | fluorine | methyl | methyl | 3-phenyl-[1,2,4]oxadiazol-5-yl |
| 10.) | chlorine | methyl | methyl | 3-methyl-[1,2,4]oxadiazol-5-yl |
| 11.) | chlorine | methyl | methyl | 3-cyclopropyl-[1,2,4]oxadiazol-5-yl |
| 12.) | chlorine | methyl | methyl | 3-phenyl-[1,2,4]oxadiazol-5-yl |
| 13.) | fluorine | hydrogen | methyl | 3-methyl-[1,2,4]oxadiazol-5-yl |
| 14.) | fluorine | hydrogen | methyl | 3-cyclopropyl-[1,2,4]oxadiazol-5-yl |
| 15.) | fluorine | hydrogen | methyl | 3-phenyl-[1,2,4]oxadiazol-5-yl |
| 16.) | chlorine | hydrogen | methyl | 3-methyl-[1,2,4]oxadiazol-5-yl |
| 17.) | chlorine | hydrogen | methyl | 3-cyclopropyl-[1,2,4]oxadiazol-5-yl |
| 18.) | chlorine | hydrogen | methyl | 3-phenyl-[1,2,4]oxadiazol-5-yl |

It is to be understood that the present invention includes any or all possible combinations and subsets of the special embodiments defined hereinabove.

The compounds according to the present invention can be prepared as specified as follows, or in a manner described in the following examples, or according to art-known procedures, or analogously or similarly thereto.

As shown in reaction scheme 1, compounds of formula I, in which R1, R2, R3, R4, R41, R5, R51, R6, R7 and R8 have the meanings given above, and Het4 is an oxazolyl radical, can be obtained from corresponding amides of formula II by a cyclization reaction using compounds of formula III, in which R81 has the meanings given above and X is a suitable leaving group, such as e.g. bromine or, particularly, chlorine. This cyclization reaction can be carried out as as it is known for the skilled person, or as described by way of example in the following examples, or analogously or similarly thereto.

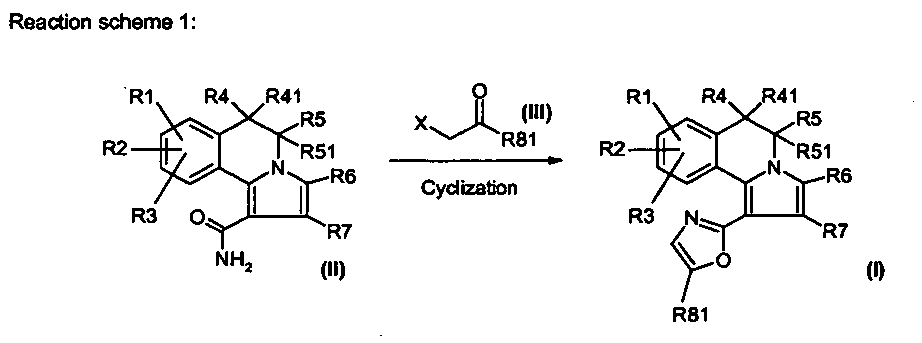

As shown in reaction scheme 2, compounds of formula I, in which R1, R2, R3, R4, R41, R5, R51, R6, R7 and R8 have the meanings given above, and Het4 is an [1,2,4]oxazol-5-yl radical, can be obtained from corresponding ester compounds, particularly the methyl ester compounds of formula IV by a cyclization reaction using compounds of formula Va or Vb, in which R81 has the meanings given above, or a mixture of both. This cyclization reaction can be carried out as it is known for the skilled person, or as described by way of example in the following examples, or analogously or similarly thereto.

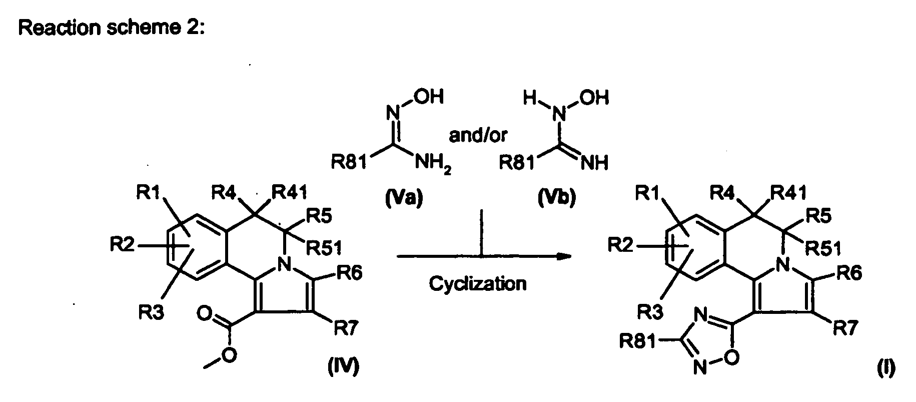

As shown in reaction scheme 3, compounds of formula I, in which R1, R2, R3, R4, R41, R5, R51, R6, R7 and R8 have the meanings given above, and Het4 is an [1,2,4]oxazol-3-yl radical, can be obtained from corresponding nitrile compounds of formula VI by reaction with hydroxylamine, acylation of the intermediate amidoxime using an activated carboxylic acid derivative of the formula R81 C(O)Y, in which Y is a suitable leaving group, such as e.g. chlorine or an acyloxy leaving group (e.g. the R81 C(O)-O- radical), and, finally, cyclization. In the case, if an anhydride of the formula R81 C(O)Y, such as e.g. acetanhydride, is used, acylation and cyclization can be obtained in one step. The reaction steps mentioned can be carried out as it is known for the skilled person, or as described in J. Med. Chem. 1986, 29, 2174-2183, the disclosure of which is incorporated herein, or analogously or similarly thereto.

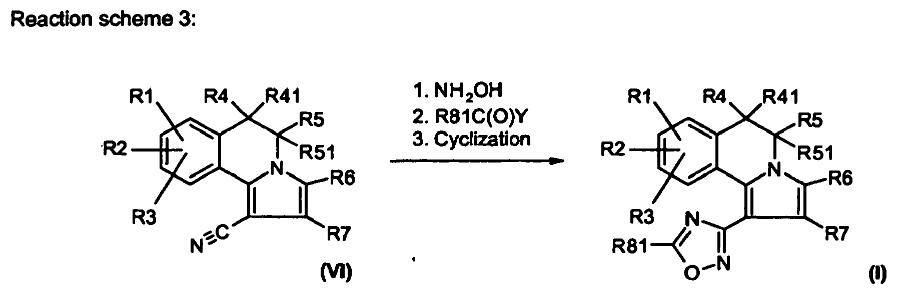

Compounds of formulae III, Va, Vb and R81 C(O)Y are commercially available or can be obtained in a manner known to the skilled person from his/her expert knowledge and/or from literature.

The compounds of formula IV and VI can be obtained in an art-known manner, or in a manner described and shown as follows, or as disclosed in WO 02/48144, WO 03/014115, WO 03/014116, WO 03/014117 or WO 03/051877 (the disclosure of which is incorporated herein), or as described by way of example in the following examples, or analogously or similarly thereto. As shown in reaction scheme 4, in a first reaction step compounds of formula XIII, in which R1, R2, R3, R4, R41, R5 and R51 have the meanings indicated above, are reacted with compounds of formula XII, in which R' is cyano or methoxycarbonyl, and L is a suitable leaving group, for example chlorine or an acyloxy radical (e.g. the R'-CH₂-C(O)-O- radical), to give in the presence of a suitable organic or inorganic base corresponding compounds of formula XI.

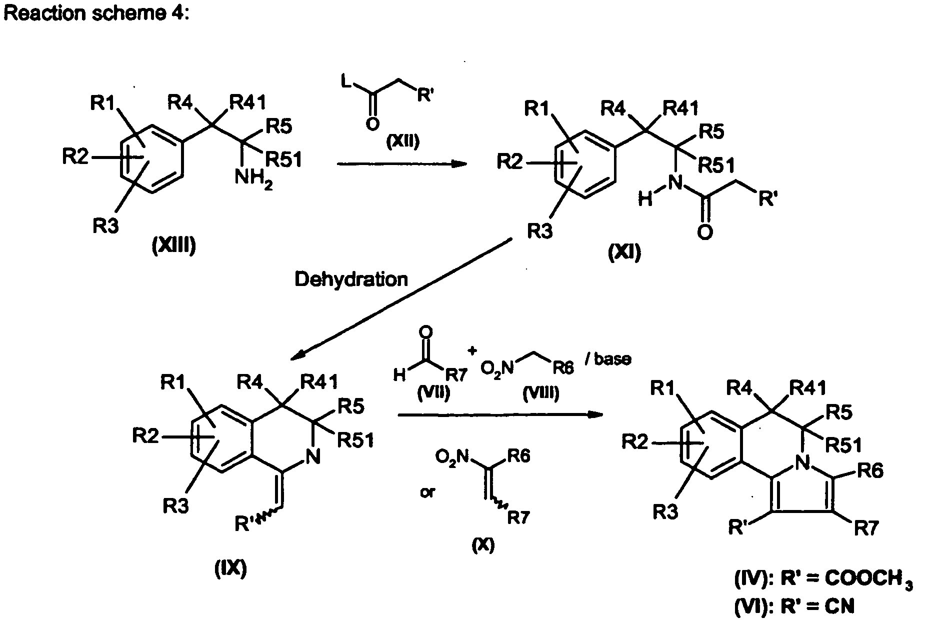

Alternatively, compounds of formula XI are also accessible from compounds of formula XIII, in which R1, R2, R3, R4, R41, R5 and R51 have the meanings indicated above, and compounds of formula XII, in which R' is cyano or methoxycarbonyl and L is hydroxyl, by reaction with amide bond linking reagents known to the person skilled in the art. Exemplary amide bond linking reagents known to the person skilled in the art which may be mentioned are, for example, the carbodiimides (e.g. dicyclohexylcarbodiimide or, preferably, 1-ethyl-3-(3-dimethylaminopropyl)carbodiimide hydrochloride), azodicarboxylic acid derivatives (e.g. diethyl azodicarboxylate), uronium salts [e.g. O-(benzotriazol-1-yl)-N,N,N',N'-tetrarmethyluronium tetrafluoroborate or O-(benzotriazol-1yl)-N,N,N',N'-tetramthyl-uronium-hexafluorophosphate] and N,N'-carbonyldiimidazole. In the scope of this invention preferred amide bond linking reagents are uronium salts and, particularly, carbodiimides, preferably, 1-ethyl-3-(3-dimethylaminopropyl)carbodiimide hydrochloride.
Said reactions are carried out under conditions known to the person skilled in the art or as described exemplarily in the following examples.

As shown in the next step, compounds of the formula IX, in which R1, R2, R3, R4, R41, R5 and R51 have the meanings indicated above and R' is cyano or methoxycarbonyl, can be obtained by cyclocondensation of corresponding compounds of the formula XI. Said cyclocondensation reaction is carried out in a manner habitual per se to the person skilled in the art or as described by way of example in the following examples, according to Bischier-Napieralski (e.g. as described in J. Chem. Soc., 1956, 4280-4282) in the presence of a suitable condensing or dehydrating agent, such as, for example, polyphosphoric acid, phosphorus pentachloride, phosphorus pentoxide or phosphorus oxychloride, in a suitable inert solvent, e.g. in a chlorinated hydrocarbon such as chloroform, or in a cyclic hydrocarbon such as toluene or xylene, or another inert solvent such as acetonitrile, or without further solvent using an excess of condensing agent, at reduced temperature, or at room temperature, or at elevated temperature or at the boiling temperature of the solvent or condensing agent used.

Compounds of formula IX, in which R1, R2, R3, R4, R41, R5 and R51 have the meanings indicated above and R' is methoxycarbonyl, are converted either with compounds of formulae VII, in which R7 has the meanings given above, and VIII, in which R6 is 1-6C-alkyl or 1-4C-alkyl substituted by 1-4C-alkoxycarbonyl, or with compounds of formula X, in which R7 has the meanings given above and R6 is 1-6C-alkyl or 1-4C-alkyl substituted by 1-4C-alkoxycarbonyl, optionally in a one pot synthesis and suitably in the presence of an inorganic or organic base (in particular a cyclic amine, e.g. piperidine) into the corresponding compounds of formula IV. In an analogous manner as described afore, compounds of formula IX, in which R1, R2, R3, R4, R41, R5 and R51 have the meanings indicated above and R' is cyano, are converted into the corresponding compounds of formula VI.

Said conversion can be carried out as known to the skilled person or as described in the following examples or analogously or similarly thereto.

Compounds of formulae XIII, XII, VIII and VII are commercially available or can be obtained in a manner described in the following example or known to the skilled person from his/her expert knowledge and/or from literature, or analogously or similarly thereto.
Thus, e.g, compounds of formula XIII can be obtained starting from the corresponding benzaldehydes or acetophenons by a Henry reaction using the appropriate nitroalkane (e.g. nitromethane or nitroethane) and subsequent reduction of the nitro group and the double bond in a manner customary per se to the skilled person (using e.g. LiAIH₄, see e.g. Zhumal Organicheskoi Khimii, 1989, 25(7), 1477-82 or J. Org. Chem. 2005, 70(14), 5519-27)), or in analogy to the sequence described in J. Med. Chem. 1987, 30(10), 1914-1918.

The mentioned benzaldehydes and acetophenons are known or can be obtained in analogy to known procedures or as described in the following examples.
Compounds of formula X are known or are accessible by reaction of compounds of formula VII with compounds of formula VIII in the presence of a suitable organic or inorganic base in a manner customary per se to the skilled person.

Compounds of formula II are obtained from corresponding compounds of formula IV by an amidification reaction, which can be carried out analogously to procedures known to the skilled person or as described by way of example in the following examples.

Besides the above-described synthesis route to compounds of formula I, wherein the heterocyclic group attached to the 1-position of the pyrrolo[2.1-a]isoquinoline ring is built up in the final step, in an alternative synthesis rout this heterocyclic group can be built up on a previous stage, e.g. starting from compounds of formula IX.

Compounds of formula I obtained can be converted into further compounds of formula I by methods known to one of ordinary skill in the art. More specifically, for example, from compounds of the formula I, in which
a.) R61, R71, R74 or R76 are an ester group, the corresponding adds can be obtained by acidic or, particularly, alkaline hydrolysis;
b.) R6 is 1-4C-alkyl, particularly methyl, the corresponding halogenated, preferably chlorinated, groups can be obtained by halogenation reaction, particularly by reaction with a chlorination reagent such as sulfuryl chloride, thionyl chloride or N-chlorosuccinimide;
c.) R6 is 1-4C-alkyl substituted by halogen obtainable according b.), the corresponding derivatized 1-4C-alkyl radicals substituted by 1-4C-alkoxy, hydroxyl, halogen or -N(R611)R612 can be obtained by nucleophilic substitution reactions with suitable nucleophiles;
d.) R6 is 1-4C-alkyl substituted by hydroxyl obtainable according c.) or e.), the corresponding derivatized 1-4C-alkyl radicals substituted by 1-4C-alkoxycarbonyl can be obtained by oxidation and esterification reactions under suitable conditions;
e.) R6 is methyl, the corresponding oxidized formes thereof (e.g. the hydroxymethyl or formyl radicals) can be obtained stepwise or directly by selective oxidation reactions (e.g. with the aid of manganese dioxide to obtain the formyl radicals);
f.) R6 is formyl obtainable according e.), the corresponding aminated compounds can be obtained by reductive amination reaction;
g.) R6 is hydroxymethyl obtainable according e.), the corresponding fluorine compounds can be obtained by fluorination reaction;
h.) R6 is methyl, the corresponding amino compounds can be obtained by nitration reaction and subsequential reduction of the nitro compounds obtained.

The methods mentioned under a.) to h.) are expediently carried out analogously to the methods known to the person skilled in the art or as described by way of example in the following examples.

Optionally, compounds of the formula I can be converted into their salts, or, optionally, salts of the compounds of the formula I can be converted into the free compounds. Corresponding processes are habitual per se to the skilled person.

It is moreover known to the person skilled in the art that if there are a number of reactive centers on a starting or intermediate compound it may be necessary to block one or more reactive centers temporarily by protective groups in order to allow a reaction to proceed specifically at the desired reaction center. A detailed description for the use of a large number of proven protective groups is found, for example, in "Protective Groups in Organic Synthesis" by T. Greene and P. Wuts (John Wiley & Sons, Inc. 1999, 3rd Ed.) or in "Protecting Groups (Thieme Foundations Organic Chemistry Series N Group" by P. Kocienski (Thieme Medical Publishers, 2000).

The isolation and purification of the substances according to the invention is carried out in a manner known per se, e.g. by distilling off the solvent in vacuo and recrystallizing the resulting residue from a suitable solvent or subjecting it to one of the customary purification methods, such as, for example, column chromatography on suitable support material.

Salts are obtained by dissolving the free compound in a suitable solvent (e.g. a ketone, such as acetone, methyl ethyl ketone or methyl isobutyl ketone, an ether, such as diethyl ether, tetrahydrofuran or dioxane, a chlorinated hydrocarbon, such as methylene chloride or chloroform, or a low molecular weight aliphatic alcohol such as methanol, ethanol or isopropanol) which contains the desired acid or base, or to which the desired acid or base is then added. The salts are obtained by filtering, reprecipitating, precipitating with a nonsolvent for the addition salt or by evaporating the solvent. Salts obtained can be converted by alkalization or by acidification into the free compounds, which in turn can be converted into salts. In this way, pharmacologically intolerable salts can be converted into pharmacologically tolerable salts.

Suitably, the conversions mentioned in this invention can be carried out analogously or similarly to methods which are familiar per se to the person skilled in the art.

The person skilled in the art knows on the basis of his/her knowledge and on the basis of those synthesis routes, which are shown and described within the description of this invention, how to find other possible synthesis routes for compounds of the formula I. All these other possible synthesis routes are also part of this invention.

The present invention also relates to intermediates and methods useful in synthesizing compounds according to this invention.

Having described the invention in detail, the scope of the present invention is not limited only to those described characteristics or embodiments. As will be apparent to persons skilled in the art, modifications, analogies, variations, derivations, homologisations and adaptations to the described invention can be made on the base of the disclosure (e.g. the explicite, implicite or inherent disclosure) of the present invention without departing from the spirit and scope of this invention as defined by the scope of the appended claims.

The following examples serve to illustrate the invention in greater detail without restricting it. Likewise, further compounds of the formula I, whose preparation is not explicitly described, can also be prepared in an analogous manner or in a manner familiar per se to the person skilled in the art using customary process techniques.

In the examples, m.p. stands for melting point, h for hour(s), min for minutes, conc. for concentrated, satd. for saturated, MS for mass spectrum, M for molecular ion, other abbreviations have their meanings customary per se to the skilled person.

Unless otherwise noted, when the exemplary compounds mentioned expressis verbis herein contain a chirality center, they are described illustratively as racemic mixtures herein, without restricting this invention thereto. Accordingly, the pure enantiomers and the salts thereof are also part of the invention.

The compounds of formula I mentioned in the examples, particularly which are mentioned as final compounds, as well as their salts, stereoisomers and salts of the stereoisomers are a preferred subject of the invention.

### Examples

### Final Compounds

### 1. (5RS)-4-[8,9-Dimethoxy-3,5-dimethyl-1-(3-methyl[1,2,4]oxadiazol-5-yl)-5,6-dihydro-pyrrolo[2,1-a]isoquinolin-2-yl]-2,6-dimethyl-phenol

A solution of 68 mg (918 µmol, 2.00 eq) N-hydroxy acetamidine and 55 mg (1.38 mmol, 3.00 eq) sodium hydride In 2 ml THF (and 150 mg molecular sieves) is heated to reflux for 1 h. A solution of 200 mg (459 µmol, 1.00 eq) (5RS)-(4-hydroxy-3,5-dimethyl-phenyl)-8,9-dimethoxy-3,5-dimethyl-5,6-dihydro-pyrrolo[2,1-a]isoquinoline-1-carboxylic acid methyl ester (compound A1) in THF is added. The mixture is heated to reflux for 24 h. Additional 68 mg acetamidine and 55 mg sodium hydride are added before heating to reflux for additional 4.5 h. Water is added and the solution is extracted with ethyl acetate. The organic layer is dried with magnesium sulfate and the solvent is removed at reduced pressure. After purification by column chromatography and washing with ethanol 21 mg of the title compound are obtained as a colorless solid.
M.p.: 182-184 °C MS: 459,8 (MH⁺)

### 2. (5RS)-4-[1-(3-Cyclopropyl-[1,2,4]oxadiazol-5-yl)-8,9-dimethoxy-3,5-dlimethyl-5,6-dihydro-pyrrolo[2,1-a]isoquinolin-2-yl]-2,6-dimethyl-phenol

The title compound can be prepared from compound A1 and N-hydroxy cyclopropanecarboxamidine analogously as described in Example 1.
M.p.: 174-175 °C MS: 485,8 (MH⁺)

### 3. (5RS)-4-[8,9-Dimethoxy-3,5-dimethyl-1-(3-phenyl-[1,2,4]oxadiazol-5-yl)-5,6-dihydro-pyrrolo[2,1-a]isoqulnolin-2-yl]-2,6-dimethyl-phenol

The title compound can be prepared from compound A1 and N-hydroxy benzamidine analogously as described in Example 1.
M.p.: 203-206 °C MS: 522,2 (MH⁺)

### 4. 4-[8,9-Dimethoxy-3-methyl-1-(3-phenyl-[1,2,4]oxadiazol-5-yl)-5,6-dihydro-pyrrolo[2,1-a]isoquinolin-2-yl]-2,6-dimethyl-phenol

The title compound can be prepared from (4-hydroxy-3,5-dimethyl-phenyl)8,9-dimethoxy-3-methyl-5,6-dihydro-pyrrolo[2,1-a]isoquinoline-1-carboxylic acid methyl ester (compound A2) and N-hydroxy benzamidine analogously as described in Example 1.
M.p.: 191-193 °C MS: 508,2 (MH⁺)

### 5. 4-[8,9-Dimethoxy-3-methyl-1-(3-methyl-[1,2,4]oxadiazol-5-yl)-5,6-dihydro-pyrrolo[2,1-a]isoqulnolin-2-yl]-2,6-dimethyl-phenol

The title compound can be prepared from (4-hydroxy-3,5-dimethyl-phenyl)-8,9-dimethoxy-3-methyl-5,6-dihydro-pyrrolo[2,1-a]isoquinoline-1-carboxylic acid methyl ester (compound A2) and N-hydroxy acetamidine analogously as described in Example 1.
M.p.: 218-221 °C MS: 445,9 (MH⁺)

### 6. 4-[8,9-Dimethoxy-3-methyl-1-(5-methyl-oxazol-2-yl)-5,6-dihydro-pyrroloo[2,1-a]isoquinolin-2-yl]-2,6-dimethyl-phenol

A mixture of 5.00 mg (1.23 mmol, 1.00 eq) of 2-(4-hydroxy-3,5-dimethyl-phenyl)-8,9-dimethoxy-3-methyl-5,6-dihydro-pyrrolo[2,1-a]isoquinoline-1-carboxylic acid amide (compound A3), 300 µl (3.69 mmol, 3.00 eq) chloro acetone, 7.00 ml toluene and 7.00 ml THF are heated for 16 h to 120 °C in a sealed tube. The solvents are removed at reduced pressure and the residue is purified by column chromatography. After washing with ethanol 340 mg (62 %) of the title compound are obtained as a white solid.
M.p.: 222-224 °C MS: 444,9 (MH⁺)

### Starting Compounds

### A1. (5RS)-2-(4-Hydroxy-3,5-dimethyl-pheny)-8,9-dimethoxy-3,5-dimethyl-5,6-dihydro-pyrrolo[2,1-a]isoquinoline-1-carboxylic acid methyl ester

Analogously to a procedure described by Meyer in Liebigs Ann. Chem. 1981, 9,1534-1544, (3RS)-(6,7-dimethoxy-3-methyl-3,4-dihydro-2H-isoquinolin-1-ylidene)-acetic acid methyl ester (compound B1) is reacted with nitro ethane and 4-hydroxy-3,5-dimethyl benzaldehyde to afford the title compound.
MS (M+H) = 435.9; m.p. = 177 -179 °C

### A2. (4-Hydroxy-3,5-dimethyl-phenyl)-8,9-dimethoxy-3-methyl-5,6-dihydro-pyrrolo[2,1-a]isoquinoline-1-carboxylic acid methyl ester

The preparation of the title compound is described in example 20 of WO 02/48144.

### A3. 2-(4-Hydroxy-3,5-dimethyl-phenyl)-8,9-dimethoxy-3-methyl-5,6-dihydro-pyrrolo[2,1-a]isoquinoline-1-carboxylic acid amide

To a mixture of 1.40 g (26.1 mmol, 5.00 eq) ammonia chloride in 23 ml toluene at 0 °C are added dropwise 14.4 ml (28.7 mmol, 5.50 eq) of a 2 M solution of AlMe₃ in toluene. The solution is stirred at room temperature for 1 h. A solution of 2.20 g (5.22 mmol, 1.00 eq) 2-(4-hydroxy-3,5-dimethylphenyl)-8,9-dimethoxy-3-methyl-5,6-dihydro-pyrrolo[2,1-a]isoquinoline-1-carboxylic acid methyl ester (compound A2) in 46 ml THF is added. The mixture is stirred at 80 °C for 16 h and cooled to room temperature. A 5 M aqueous solution of sodium hydroxide is added until the pH was basic. Water is added and the mixture was extracted with ethyl acetate. The organic layer is separated and dried with magnesium sulfate. The solvent is removed at reduced pressure and the residue is purified by column chromatography. 1.16 g (45 %) of the title compound are obtained.
MS (M+H) = 407.2; m.p. = 229 - 231 °C

### B1. (3RS)-(6,7-Dimethoxy-3-methyl-3,4-dihydro-2H-isoquinolin-1-ylidene)-acetic acid methyl ester

The title compound can be obtained by a Bischler-Napieralski reaction (e.g. Ber. 1893, 26, 1903) using N-{2-[4-methoxy-3-(2-methoxy-ethoxy)-phenyl]-ethyl}-malonamic acid methyl ester (compound C1) as the starting material.

### C1. N-[(RS)-2-(3,4-Dimethoxy-phenyl)-1-methyl-ethyl]-malonamic acid methyl ester

The title compound can be prepared by a reaction of (RS)-2-(3,4-dimethoxy-phenyl)-1-methyl-ethylamine (compound D1) with methyl maloyl chloride in analogy to procedures in the literature (e.g. Benovsky et al., Tetrahedron Lett. 1997, 38, 8475-8478).

### D1. (RS)-2-(3,4-Dimethoxy-phenyl)-1-methyl-ethylamine

The title compound can be obtained starting from the corresponding benzaldehyde and nitroethane in analogy to a Henry reaction (e.g. Synthesis 1985 (5), 510-512) and subsequent reduction reaction (using e.g. LiAIH₄ in THF).

Further compounds according to this invention can be prepared starting from compounds D2 to D5:

### D2. 2-(3,4-Dimethoxy-pheny)-ethylamine

The title compound is commercially available.

### D3. 2-[4-Methoxy-3-(2-methoxy-ethoxy)-phenyl)-ethylamine

2-[4-Methoxy-3-(2-methoxy-ethoxy)-phenyl)-ethylamine can be prepared by alkylation of 4-methoxy-3-hydroxy benzaldehyde with 2-bromomethyl ethyl ether (analogous to a procedure by Ashton et al., J. Med. Chem. 1994, 37, 1696-1703), followed by a sequence described by Shepard et al. in J. Org. Chem. 1952, 17, 568.
MS (M+H) = 226.0

### D4. 2-[4-(1,1-Difluoro-methoxy)-3-methoxy-phenyl]-ethylamine

2-[4-(1,1-Difluoro-methoxy)-3-methoxy-phenyl]-ethylamine can be prepared by difluoromethylation of 4-hydroxy-3-methoxy benzaldehyde with chloro difluoro methane according to a procedure published by Amschler et al. (WO97/28131), followed by a sequence described by Shepard et al. in J. Org. Chem. 1952, 17, 568.
MS (M+H) = 217.6

### D5. 2-[3-(1,1-Difluoro-methoxy)-4-methoxy-phenyl]-ethylamine

2-[3-(1,1-Difluoro-methoxy)-4-methoxy-phenyl]-ethylamine can be prepared by difluoromethylation of 3-hydroxy-4-methoxy benzaldehyde with chloro difluoro methane according to a procedure published by Amschler et al. (WO97/28131), followed by a sequence described by Shepard et al. in J. Org. Chem. 1952, 17, 568.
MS (M+H) = 217.7

### Commercial applicability

Intracellular levels of the second messengers cAMP and cGMP are regulated by both their rates of synthesis by cyclases and their hydrolysis by phosphodiesterases. Of the 11 phosphodiesterase (PDE) isoenzymes which are presently known, PDE10 was described for the first time in 1999 (Soderling SH, Bayuga SJ, Beavo JA. Isolation and characterization of a dual-substrate phosphodiesterase gene family: PDE10A. Proc Natl Acad Sci U S A. 1999 Jun 8;96(12):7071-6; Fujishige K, Kotera J, Mlchibata H, Yuasa K, Takebayashi S, Okumura K, Omori K. Cloning and characterization of a novel human phosphodiesterase that hydrolyzes both cAMP and cGMP (PDE10A). J Biol Chem. 1999 Jun 25;274(26):18438-45; Loughney K, Snyder PB, Uher L, Rosman GJ, Ferguson K, Florio VA. Isolation and characterization of PDE10A, a novel human 3', 5-cyclic nucleotide phosphodiesterase. Gene. 1999 Jun 24;234(1):109-17). The first gene of this new PDE subfamily was designated PDE10A and the first splice variant was described as PDE10A1, according to the current nomenclature. Due to alternative splicing, other splice variants of PDE10A exist and have been described in the subsequent years (Kotera J, Fujishige K, Yuasa K, Omori K. Characterization and phosphorylation of PDE10A2, a novel alternative splice variant of human phosphodiesterase that hydrolyzes cAMP and cGMP. Biochem Biophys Res Commun. 1999 Aug 11;261(3):551-7; Fujishige K, Kotera J, Omori K. Striatum- and testis-specific phosphodiesterase PDE10A isolation and characterization of a rat PDE10A. Eur J Biochem. 1999 Dec;266(3):1118-27; Fujishige K, Kotera J, Yuasa K, Omori K. The human phosphodiesterase PDE10A gene genomic organization and evolutionary relatedness with other PDEs containing GAF domains. Eur J Biochem. 2000 Oct;267(19):5943-51). PDE10A has been described as a cyclic nucleotide phosphodiesterase exhibiting properties of a cAMP PDE and a cAMP-inhibited cGMP PDE.

Individual representatives of the PDE10 isoenzyme are characterized by being highly expressed in specific areas of the brain (striatum, putamen, caudate nucleus, cerebellum, thalamus), in testis, in kidney and in placenta.

Novel results have demonstrated that PDE10A expression is particularly prominently expressed in neurotransmitter/hormone secreting cells in brain, islets of Langerhans, pituitary and adrenal glands, see e.g. WO2005120474.
As cAMP is a well known stimulus for hormone/neurotransmitter release, it can be claimed that the neuroendocrine-specific mRNA expression of PDE10A reflects an important role in hormone/neurotransmitter secretion. For example, striatal expression and function of PDE10A has been associated with the regulation of dopaminergic neurotransmission (e.g. US 2003/0008806). Therefore, inhibition of PDE10A may be used for the treatment of disorders of the central nervous system (e.g. schizophrenia). Additionally, PDE10A inhibition in pancreatic beta cells might effect in a rise of cAMP and therefore induce or enforce insulin secretion. This effect may improve glucose homeostasis in type II diabetic patients.
Moreover it has been shown that pharmacological inhibition of PDE10 or a genetic knockout of the pde10A gene is an effective means of reducing body weight, reducing body fat, and treating disorders associated with increased adiposity. These results also demonstrated that PDE 10 inhibitors are effective in treating disorders associated with NIDDM (non insulin dependent diabetes mellitus), glucose intolerance, insulin resistance and metabolic syndrome in addition to reducing body weight, body fat, and treating disorders associated with increased adiposity, see e.g. W02005120514, the disclosure of which is incorporated herein.

Increased expression levels and activities of PDE10A in testis suggests that PDE10A may also contribute to spermatogenesis (Fujishige K et al., Eur J Biochem. 1999, 266:1118-27).

The compounds according to the invention have miscellaneous valuable pharmacological properties which make them commercially utilizable.
Thus, for example, the compounds according to this invention are PDE inhibitors.
Yet thus, for example, the compounds according to the invention are potent PDE10 inhibitors, some of which are apparently selective (e.g. by >10 or, particularly, >30 fold, or, for some advantage, >100 fold) among other PDE isoenzymes (such as e.g. PDEs 1A, 2A, 3A, 4B, 5A, 7A, 8A, 9A or 11A) whereby these selective compounds are particularly preferred in the context of the present invention. The compounds according to the invention therefore can be employed as therapeutic agents for the treatment or prophylaxis of diseases in human and veterinary medicine; especially they are particular useful in the therapy of those diseases or conditions mentioned below.

Due to their potent and selective PDE10 inhibitory activity, the compounds according to the present invention may be, in a first facet of the present invention, of potential value in treating disorders of the central nervous system, in particular neurologic and psychiatric disorders, for example those mentioned in EP 1250923 and/or, in more particular, psychotic disorders, anxiety disorders, mood disorders or episodes, drug addiction, movement disorders or disorders comprising deficient cognition as a symptom (e.g. dementia, Parkinson's disease or Alzheimer's disease).

Furthermore, the compounds according to the present invention may be, in a second facet of the present invention, of potential value in treating certain disorders of the central nervous system, in particular neurologic and psychiatric disorders, for example those mentioned generically, specifically or exemplarily in EP 1250923, US 2003/0008806, US 2003/0018047, US 2003/032579, US 2004/162293, US 2004/162294 and/or WO03092499, such as, for example, anxiety or psychotic disorders, movement disorders, obsessive/compulsive disorders, drug addictions, cognition deficiency disorders, mood disorders or mood episodes, or neurodegenerative disorders.

In this context, examples of anxiety disorders, which may be treated by the compounds according to the present invention, include, without being limited thereto, panic disorder, agoraphobia, a specific phobia, social phobia, obsessive-compulsive disorder, post-traumatic stress disorder, acute stress disorder, or generalized anxiety disorder.

Examples of psychotic disorders, which may be treated by the compounds according to the present invention, include, without being limited thereto, schizophrenia (for example of the paranoid, disorganized, catatonic, undifferentiated, or residual type), schizophreniform disorder, schizoaffective disorder (for example of the delusional type or the depressive type), delusional disorder, substance-induced psychotic disorder (for example psychosis induced by alcohol, amphetamine, cannabis, cocaine, hallucinogens, inhalants, opioids, or phencyclidine), personality disorder of the paranoid type, or personality disorder of the schizoid type.

Examples of movement disorders, which may be treated by the compounds according to the present invention, include, without being limited thereto, Parkinson's disease, or restless leg syndrome.

Examples of obsessive/compulsive disorders, which may be treated by the compounds according to the present invention, include, without being limited thereto, Tourette's syndrome, or other tic disorders.

Examples of drug addictions, which may be treated by the compounds according to the present invention, include, without being limited thereto, an alcohol, amphetamine, cocaine, or opiate addiction.

Examples of cognition deficiency disorders, which may be treated by the compounds according to the present invention, include, without being limited thereto, Alzheimer's disease, multi-infarct dementia, alcoholic dementia or other drug-related dementia, dementia associated with intracranial tumors or cerebral trauma, dementia associated with Huntington's disease or Parkinson's disease, or AIDS-related dementia, delirium, amnestic disorder, post-traumatic stress disorder, mental retardation, a learning disorder, for example reading disorder, mathematics disorder, or a disorder of written expression, attention-deficit/hyperactivity disorder, or age-related cognitive decline.

Examples of mood disorders or mood episodes, which may be treated by the compounds according to the present invention, include, without being limited thereto, a major depressive episode of the mild, moderate or severe type, a manic or mixed mood episode, a hypomanic mood episode, a depressive episode with a typical features, a depressive episode with melancholic features, a depressive episode with catatonic features, a mood episode with postpartum onset, post-stroke depression, major depressive disorder, dysthymic disorder, minor depressive disorder, premenstrual dysphoric disorder, post-psychotic depressive disorder of schizophrenia, a major depressive disorder superimposed on a psychotic disorder such as delusional disorder or schizophrenia, a bipolar disorder (for example bipolar I disorder, bipolar II disorder), or cyclothymic disorder.

Examples of neurodegenerative disorders, which may be treated by the compounds according to the present invention, include, without being limited thereto, Parkinson's disease, Huntington's disease, dementia (for example Alzheimer's disease, multi-infarct dementia, AIDS-related dementia, or Fronto temperal Dementia), neurodegeneration associated with cerebral trauma, neurodegeneration associated with stroke, neurodegeneration associated with cerebral infarct, hypoglycemia-induced neurodegeneration, neurodegeneration associated with epileptic seizure, neurodegeneration associated with neurotoxin poisoning, or multi-system atrophy.

Yet in this context, the compounds according to the present invention may be of potential value for treating diseases or conditions, in which abnormal function of the basal ganglia has been implicated. Thus, abnormal function of the basal ganglia may be involved in disregulated motoric, appetitive and/or cognitive processes. Exemplary neuropsychiatric conditions, in which abnormal function of the basal ganglia has been implicated, are mentioned e.g. in EP 1250923, US 2003/0008806, US 2003/0018047, US 2003/032579, US 2004/162293, US 2004/162294 and/or WO03092499, such as e.g. psychosis, attention-deficit/hyperactivity disorder (ADHD) and related attentional disorders, depression, obsessive conpulsive disorders including Tourette's syndrome and other tic disorders, and substance abuse. Several neurological disorders including Parkinson's disease, restless leg syndrom and Huntington's disease can be also linked to basal ganglia dysfunction.

Still yet in this context, the compounds according to the present invention may be of potential value for improving cognition, powers of concentration, learning skills or hypermesia, in particular if the disorder is a symptom of dementia.

Yet furthermore, the compounds according to the present invention may be, in a third facet of the present invention, of potential value for regulating fertility, e.g. via reducing spermatogenesis and/or via reducing sperm motility.

Still yet furthermore, the compounds according to the present invention may be, in a fourth facet of the present invention, of potential value for treating diabetes, such as, for example, typ II diabetes, e.g. via augmenting glucose-induced insulin secretion.

A special interest in the compounds according to the present invention lies in their use in therapy of schizophrenia.

Another special interest in the compounds according to the present invention lies in their use in the therapy of psychotic disorders.

Another special interest in the compounds according to the present invention lies in their use in the therapy of drug addictions.

Furthermore, a special interest in the compounds according to the present invention lies in their properties which make them particularly useful in the therapy of diseases or conditions other than cancer. In this connection, a further special interest in the compounds according to the present invention lies in their reduced cytotoxic activity.

The invention further relates to the compounds according to this invention for use in a method for treating mammals, including humans, which/who are suffering from one of the abovementioned diseases and/or disorders. The method is characterized by the fact that a pharmacologically active and therapeutically effective and tolerated quantity of one or more of the compounds according to the invention is administered to the affected mammal.

The invention further relates to the compounds according to this invention for use in a method for inhibiting PDE10 comprising administering an effective amount of one or more compounds of the present invention to a mammal in need thereof.

The invention further relates to the compounds according to the invention for use in the treatment or prophylaxis of diseases, in particular said diseases and/or disorders.

The invention likewise relates to the use of the compounds according to the invention in the manufacture of pharmaceutical compositions which are employed for the treatment of said diseases or disorders.

The invention further relates to pharmaceutical compositions for the treatment or prophylaxis of the said diseases and/or disorders, which pharmaceutical compositions comprise one or more of the compounds according to the invention.

The present invention further relates to pharmaceutical compositions comprising one or more of the compounds according to this invention and a pharmaceutically acceptable carrier or diluent.

The present invention further relates to combinations comprising one or more of the compounds according to this invention and pharmaceutically acceptable auxiliaries, excipients or vehicles, e.g. for use in the treatment of those conditions mentioned above.

The present invention further relates to the use of the compounds according to this invention for the production of pharmaceutical compositions which can be used use in therapy of disorders responsive to inhibiting of PDE, such as e.g. PDE10.

The present invention further relates to compounds according to this invention having PDE, particularly PDE10, inhibiting properties.

The present invention further relates to pharmaceutical combinations or compositions according to this invention having PDE10 inhibiting properties.
The invention further relates to the use of a pharmaceutical composition comprising one or more of the compounds according to this invention as sole active ingredient(s) and a pharmaceutically acceptable carrier or diluent in the manufacture of pharmaceutical products for therapy, amelioration or prophylaxis of the illnesses, diseases, disorders or conditions mentioned above.

Further, the present invention relates to the use of the compounds according to this invention in the manufacture of pharmaceutical compositions for treating psychotic disorders, anxiety disorders, mood disorders or episodes, drug addictions, movement disorders, cognition deficiency disorders, obsessive/compulsive disorders, or neurodegenerative disorders.

Yet further, the present invention relates to the use of the compounds according to this invention in the manufacture of pharmaceutical compositions for treating diabetes, including type 2 diabetes.

Still yet further, the present invention relates to the compounds according to this invention for use in a method for treating mammals, including humans, suffering from psychotic disorders, anxiety disorders, mood disorders or episodes, drug addictions, movement disorders, cognition deficiency disorders, obsessive/compulsive disorders, or neurodegenerative disorders comprising administering to said mammal in need thereof a therapeutically effective and tolerable quantity of one or more compounds according to this invention.

In further addition, the present invention further relates to the use of the compounds according to this invention for inhibiting spermatogenesis and/or inhibiting sperm motility in a mammal, including human.

In yet further addition, the present invention further relates to the use of the compounds according to this invention for the manufactue of pharmaceutical compositions for regulating fertility in a mammal, including human.

Further on, the present invention further relates to the compounds according to this invention for use in a method to decrease body weight and/or body fat in animals, e.g. in the treatment of overweight or obese patients (e.g. humans or companion animals) or as means to produce leaner meet in food stock animals (e.g. cattle, chickens, pigs), comprising administering to a subject in need thereof an effective amount of one or more compounds according to this invention.

Further on, the present invention further relates to the use of the compounds according to this invention for the manufactue of pharmaceutical compositions for treating non-insulin dependent diabetes, metabolic syndrome, or glucose intolerance.

Further on, the present invention further relates to the use of the compounds according to this invention for the manufactue of pharmaceutical compositions for reducing body fat or body weight

Further, the present invention further relates to the use of the compounds according to this invention for the manufactue of pharmaceutical compositions for inducing or enforcing insulin secretion.

The invention furthermore relates to a commercial product which consists of a customary secondary packaging means, a primary packaging means (for example an ampoule or a blister pack) which contains a pharmaceutical composition, and, if desired, a patient information leaflet, with the pharmaceutical composition exhibiting an antagonistic effect toward type 10 cyclic nucleotide phosphodiesterases (PDE10) and leading to the attenuation of the symptoms of diseases and/or disorders which are associate with type 10 cyclic nucleotide phosphodiesterases, and with reference being made, on the secondary packaging means and/or on the patient information leaflet of the commercial product, to the suitability of the pharmaceutical composition for use in the prophylaxis or treatment of diseases and/or disorders which are associated with type 10 cyclic nucleotide phosphodiesterases, and with the pharmaceutical composition comprising one or more compounds according to this invention. The secondary packaging means, the primary packaging means containing the pharmaceutical composition and the patient information leaflet otherwise correspond to what the skilled person would regard as being the standard for drugs of this nature.

The pharmaceutical compositions according to this invention are produced using methods with which the skilled person is familiar. When employed in pharmaceutical compositions, the compounds according to the invention (= active compounds) are either used as such or, preferably, in combination with suitable pharmaceutical auxiliaries or formulating agents, for example in the form of tablets, coated (e.g. sugar-coated) tablets, capsules, caplets, suppositories, patches (e.g. as TTS), plasters, emulsions, suspensions, gels or solutions, with the content of active compound advantageously being between 0.1 and 95%, and where, by the appropriate choice of the auxiliaries, a pharmaceutical administration form (e.g. a delayed release form or an enteric form) exactly suited to the active compound and/or to the desired onset of action can be achieved.

The person skilled in the art is familiar, on the basis of his/her knowledge, with auxiliaries, vehicles, formulating agents, carriers, diluents, adjuvants or excipients which are suitable to be used for the desired pharmaceutical formulations, preparations or compositions. Beside solvents, gel-forming agents, suppository bases, tablet auxiliaries and other active carriers, it is possible to use, for example, antioxidants, dispersants, emulsifiers, antifoams, flavor corrigents, preservatives, solubilizers, colorants or, in particular, permeation promoters and complexing agents (e.g. cydodextrines).

The administration of the compounds or pharmaceutical compositions according to the invention may be performed in any of the generally accepted modes of administration available in the art. Illustrative examples of suitable modes of administration include intravenous, inhalative, oral, nasal, parenteral, topical, transdermal and rectal delivery. Intravenous or, particularly oral delivery are preferred.

For the treatment of skin diseases, the compounds according to the invention are in particular administered in the form of those pharmaceutical compositions which are suitable for topical application. For the production of the pharmaceutical compositions, the compounds according to the invention (= active compounds) are preferably mixed with suitable pharmaceutical auxiliaries and further processed to give suitable pharmaceutical formulations. Suitable pharmaceutical formulations are, for example, powders, emulsions, suspensions, sprays, oils, ointments, fatty ointments, creams, pastes, gels or solutions.

The pharmaceutical compositions according to the invention are prepared by processes known per se. The required dosage of the active compounds according to this invention can vary depending on the mode of administration, the particular condition to be treated and the effect desired. In general, satisfactory results are indicated to be obtained systemically at daily dosages of from about 0.01 to about 100 mg/kg body weight, conveniently administered, for example, in divided doses up to four times a day or in retard form.
The optimal dose and manner of administration of the active compounds necessary in each case can easily be determined by any person skilled in the art on the basis of his/her expert knowledge.

Depending upon the particular disease, to be treated or prevented, additional therapeutic active agents, which are normally administered to treat or prevent that disease, may optionally be coadministered separately, simultaneously, concurrently, sequentially or chronologically staggered with the compounds according to this invention. As used herein, additional therapeutic agents that are normally administered to treat or prevent a particular disease are known as appropriate for the disease being treated.

The person skilled in the art is aware on the base of his/her expert knowledge of the total daily dosage(s) and administration form(s) of the additional therapeutic agent(s) coadministered. Said total daily dosage(s) can vary within a wide range.

Thus, e.g. the present invention further relates to a method of treating a subject to reduce body fat or body weight, or to treat non-insulin dependent diabetes, metabolic syndrome, or glucose intolerance, comprising administering to a subject, including human, in need thereof (e.g. an overweigt subject or an obese subject) a therapeuticalls effective amount of one or more compounds according to this invention and further comprising administering a second therapeutic agent, such as e.g. an anti-obesity agent, which is e.g. selected from rimonabant, orlistat, sibutramine, bromocriptine, ephedrine, leptin, pseudoephedrine, peptide YY₃₋₃₆, and analogs thereof.

Likewise, the present invention further relates to a kit comprising one or more compound according to this invention and instructions for administering the compound to a subject to reduce body fat, body weight, or to treat non non-insulin dependent diabetes, metabolic syndrome or glucose intolerance, in the subject; optionally, said kit further comprises a second therapeutic agent, such as e.g. an anti-obesity agent, which is e.g. selected from rimonabant, orlistat, sibutramine, bromocriptine, ephedrine, leptin, pseudoephedrine, peptide YY₃₋₃₆, and analogs thereof.

### Biological investigations

Methods to determine the activity and selectivity of a phosphodiesterase inhibitor are known to the person skilled in the art. In this connection it may be mentioned, for example, the methods described by Thompson et al. (Adv Cycl Nucl Res 10: 69-92, 1979), Giembycz et al. (Br J Pharmacol 118: 1945-1958, 1996) and the phosphodiesterase scintillation proximity assay of Amersham Pharmacia Biotech.

### Inhibiting the activity of PDE10A

The PDE10A is cloned into pCR2.1-Topo (Invitrogen) via PCR from human whole brain cDNA using primers OZ 353 (5'- ACCATGTTGACAGATGAAAAAGTGAAGGC -3') and OZ 317 (5'-TCAATCTTCAGATGCAGCTGCC -3'). The ORF encoding for the PDE10A is cut with EcoRV and BamHI and subcloned into SmaI and Bgl II of the expression vector pBP9 (Clontech). The encoded protein represents the PDE10A1 (GenBank Acc.-# AB020593) truncated at its N-terminus at aa 14.

The recombinant baculoviruses are prepared by means of homologous recombination in Sf9 insect cells. The expression plasmids are cotransfected with Bao-N-Blue (Invitrogen) or Baculo-Gold DNA (Pharmingen) using a standard protocol (Pharmingen). Wildtype virus-free recombinant virus supernatants are selected using plaque assay methods. After that, high-titre virus supernatants are prepared by amplifying 3 times. PDE10A1 is expressed in Sf21 cells by infecting 2x10⁸ cells/ml with an MOI (multiplicity of infection) between 1 and 10 in serum-free SF900 medium (Life Technologies, Paisley, UK). Cells are cultured at 28°C, typically for 48 hours, after which they are pelleted for 5-10 min at 1000 g and 4°C. In spinner flasks, cells are cultured at a rotational speed of 75 rpm. The SF21 insect cells are resuspended, at a concentration of approx. 1x10⁷ cells/ml, in ice-cold (4°C) homogenization buffer (20 mM Tris, pH 8.2, containing the following additions: 140 mM NaCl, 3.8 mM KCI, 1 mM EGTA, 1 mM MgCl₂, 10 mM β-mercaptoethanol, 2 mM benzamidine, 0.4 mM Pefabloc, 10 µM leupeptin, 10 µM pepstatin A, 5 µM trypsin inhibitor) and disrupted by ultrasonication on ice. The homogenate is then centrifuged for 10 min at 1000 g (4 °C) and the supernatant is stored at -80 °C until subsequent use (see below). The protein content is determined by the Bradford method (BioRad, Munich) using BSA as the standard.

The PDE10A activity is inhibited by said compounds in a modified SPA (scintillation proximity assay) test, supplied by Amersham Pharmacia Biotech (see procedural instructions "Phosphodiesterase [3H]cAMP SPA enzyme assay, code TRKQ 7090"), carried out in 96-well microtitre plates (MTPs). The test volume was 100 µl and contained 20 mM Tris buffer (pH 7.4), 0.1 mg of BSA (bovine serum albumin)/ml, 5 mM Mg²⁺, 0.5 µM cAMP (including about 50,000 cpm of [3H]cAMP), 1 µl of the respective substance dilution in DMSO and sufficient recombinant PDE10A1 (1000xg supernatant, see above) to ensure that 15-20% of cAMP was converted under said experimental conditions. After a preincubation of 5 min at 37°C, the reaction is started by adding a substrate (cAMP) and the assays are incubated for a further 15 min; after that, they are stopped by adding SPA beads (50 µl). In accordance with the manufacturer's instructions, the SPA beads have previously been resuspended in water and diluted 1:3 (v/v) and added to IBMX (3 mM). After the beads have been sedimented (> 30 min), the MTPs are analyzed in commercially available measuring appliances and the corresponding IC₅₀ values of the compounds for the inhibition of PDE10A activity are determined from concentration-effect curves by means of non-linear regression.

Representative inhibitory values [inhibitory concentration as -logIC₅₀ (mol/l)] which are determined in the aforementioned assay are shown in the following table A, in which the numbers of the compounds correspond to the numbers of the examples.

**Table A: Inhibition of PDE10A activity**

| Compounds | -log IC₅₀ |
|---|---|
| 1 to 5 | The inhibitory values of the mentioned Examples lie in the range from 7.76 to 9.08 |

## Claims

1. Compounds of formula I 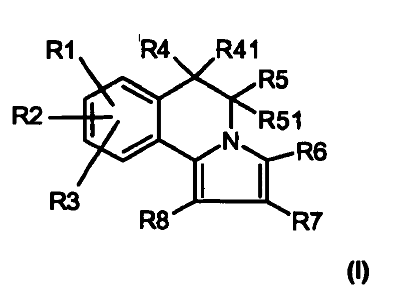 in which
R1 is halogen, nitro, amino, mono- or di-1-4C-alkylamino, 1-4C-alkyl, hydroxyl, 1-4C-alkoxy, 1-4C-alkoxy-2-4C-alkoxy, 3-7C-cycloalkoxy, 3-7C-cycloalkylmethoxy, or completely or predominantly fluorine-substituted 1-4C-alkoxy,
R2 is hydrogen, halogen or 1-4C-alkoxy, and
R3 is hydrogen or 1-4C-alkoxy, or
R2 and R3 bound to the benzo ring moiety in ortho-position to each other together form a 1-2C-alkylenedioxy bridge, or
R2 and R3 bound to the benzo ring moiety in ortho-position to each other together form a completely or predominantly fluorine-substituted 1-2C-alkylenedioxy bridge, or
R1 and R2 bound to the benzo ring moiety in ortho-position to each other together form a 1-2C-alkylenedioxy bridge and R3 is hydrogen, or
R1 and R2 bound to the benzo ring moiety in ortho-position to each other together form a completely or predominantly fluorine-substituted 1-2C-alkylenedioxy bridge and R3 is hydrogen,
R4 is hydrogen, fluorine, chlorine, 1-4C-alkyl, trifluoromethyl, cyclopropyl, cyano, 1-4C-alkoxycarbonyl or -CH₂-O-R411, in which
R411 is hydrogen, 1-4C-alkyl, 1-4C-alkoxy-24-alkyl or 1-4C-alkylcarbonyl,
R41 is hydrogen or 1-4C-alkyl,
R5 is hydrogen, fluorine or 1-4C-alkyl, and
R51 is hydrogen or 1-4C-alkyl,
or
R4 is hydrogen, fluorine, chlorine or 1-4C-alkyl,
R41 is hydrogen or 1-4C-alkyl,
R5 is hydrogen, fluorine, 1-4C-alkyl, trifluoromethyl, cyclopropyl, cyano, 1-4C-alkoxycarbonyl or -CH₂-O-R511, in which
R511 is hydrogen, 1-4C-alkyl, 1-4C-alkoxy-2-4-alkyl or 1-4C-alkylcarbonyl, and
R51 is hydrogen or 1-4C-alkyl,
or
R4 and R5 together form a 1-4C-alkylene bridge and R41 and R51 are both hydrogen,
R6 is 1-6C-alkyl, amino, formyl, or 1-4C-alkyl substituted by R61, in which
R61 is 1-4C-alkoxycarbonyl, carboxyl, 1-4C-alkoxy, hydroxyl, halogen or -N(R611)R612, in which
R611 is hydrogen, 1-4C-alkyl, 3-7C-cycloalkyl or 3-7C-cycloalkyl-1-4C-alkyl, and
R612 is hydrogen or 1-4C-alkyl, or
R611 and R612 together and with inclusion of the nitrogen atom to which they are bound form a radical Het1, in which
Het1 is a 5- to 7-membered saturated heterocyclic ring radical comprising one nitrogen atom, to which R611 and R612 are bound, and, optionally, one further heteroatom selected from a group consisting of nitrogen, oxygen and sulfur, and is optionally substituted by R613 on a ring nitrogen atom, in which
R613 is 1-4C-alkyl, 3-7C-cycloalkyl, 3-7C-cycloalkyl-1-4C-alkyl, hydroxy-2-4C-alkyl, 1-4C-alkoxy-2-4C-alkyl, amino-2-4C-alkyl, mono- or di-1-4C-alkylamino-2-4C-alkyl, formyl, pyridyl or pyrimidinyl,
R7 is phenyl, Het2, R71- and/or R72- and/or R73-substituted phenyl, R74- and/or R75-substituted Het2, naphthyl, or R76- and/or R77-substituted naphthyl, In which
Het2 is bonded to the pyrroloisoquinoline scaffold via a ring carbon atom, and is a monocyclic or fused bicyclic 5- to 10-membered partially or fully aromatic heterocyclic ring radical comprising one to four heteroatoms, each of which is selected from a group consisting of nitrogen, oxygen and sulfur,
R71 is hydroxyl, halogen, nitro, cyano, trifluoromethyl, 1-4C-alkyl, 1-4C-alkoxy, 3-7C-cycloalkoxy, 3-7C-cycloalkylmethoxy, amino, mono- or di-1-4C-alkylamino, 1-4C-alkylsulphonylamino, arylsulphonylamino, 1-4C-alkoxycarbonyl, carboxyl, 1-4C-alkylthio, aryloxy-2-4C-alkoxy, aryloxy-1-4C-alkyl, aryloxy, aryl-1-4C-alkoxy, aryl, 1-4C-alkoxy-2-4C-alkoxy, 1-4C-alkoxy-1-4C-alkyl, hydroxy-2-4C-alkoxy, amino-2-4C-alkoxy, mono- or di-1-4C-alkylamino-2-4C-alkoxy, completely or predominantly fluorine-substituted 1-4C-alkoxy, mono- or di-1-4C-alkylaminocarbonyl, carbamoyl, tetrazolyl, or -N(H)S(O)₂-N(R712)R713, in which
aryl is phenyl or R711-substituted phenyl, in which
R711 is halogen, 1-4C-alkyl, 1-4C-alkoxy, nitro or cyano,
R712 is 1-4C-alkyl,
R713 is 1-4C-alkyl, or
R712 and R713 together and with inclusion of the nitrogen atom to which they are bound form a radical Het3, in which
Het3 is pyrrolidin-1-yl, piperidin-1-yl or morpholin-4-yl,
R72 is halogen, 1-4C-alkyl, 1-4C-alkoxy or 1-4C-alkoxycarbonyl,
R73 is 1-4C-alkyl or 1-4C-alkoxy,
R74 is halogen, 1-4C-alkyl, trifluoromethyl, 1-4C-alkoxy, cyano, amino, mono- or di-1-4C-alkylamino, 1-4C-alkoxycarbonyl, morpholino, carboxyl, nitro, phenyl, phenyloxy, phenyl-1-4C-alkyl, arylsulphonyl, 1-4C-alkylsulphonyl, or -S(O)₂-N(R712)R713,
R75 is 1-4C-alkyl or halogen,
R76 is halogen, hydroxyl, 1-4C-alkyl, 1-4C-alkoxy, carboxyl or 1-4C-alkoxycarbonyl,
R77 is 1-4C-alkyl or 1-4C-alkoxy,
R8 is optionally substituted by R81, and is Het4, in which
Het4 is bonded to the pyrroloisoquinoline scaffold via a ring carbon atom, and is an oxadiazolyl or an oxazolyl radical,
R81 is 1-4C-alkyl, 3-7C-cycloalkyl, 3-7C-cycloalkylmethyl, phenyl, or R811- and/or R812-substituted phenyl, in which
R811 is 1-4C-alkyl, 1-4C-alkoxy, or halogen,
R812 is 1-4C-alkyl, 1-4C-alkoxy, or halogen,
and the stereoisomers as well as the salts of these compounds and stereoisomers.

2. Compounds of formula I according to claim 1,
in which
R1 is 1-4C-alkoxy, 3-7C-cycloalkoxy, 3-7C-cycloalkylmethoxy, or completely or predominantly fluorine-substituted 1-4C-alkoxy,
R2 is hydrogen, halogen or 1-4C-alkoxy, and
R3 is 1-4C-alkoxy, or
R2 and R3 bound to the benzo ring moiety in ortho-position to each other together form a 1-2C-alkylenedioxy bridge, or
R2 and R3 bound to the benzo ring moiety in ortho-position to each other together form a completely or predominantly fluorine-substituted 1-2C-alkylenedioxy bridge, or
R1 and R2 bound to the benzo ring moiety in ortho-position to each other together form a 1-2C-alkylenedioxy bridge and R3 is hydrogen, or
R1 and R2 bound to the benzo ring moiety in ortho-position to each other together form a completely or predominantly fluorine-substituted 1-2C-alkylenedioxy bridge and R3 is hydrogen,
and none of R1, R2 and R3 is bound to the 10-position of the pyrrolo[2.1-a]isoquinoline ring,
R4 is hydrogen or 1-4C-alkyl,
R41 is hydrogen or 1-4C-alkyl,
R5 is hydrogen, 1-4C-alkyl, cyano or 1-4C-alkoxycarbonyl,
R51 is hydrogen or 1-4C-alkyl,
or
R4 and R5 together form a 3-4C-alkylene bridge and R41 and R51 are both hydrogen,
R6 is 1-6C-alkyl, or 1-4C-alkyl substituted by R61, in which
R61 is 1-4C-alkoxycarbonyl or carboxyl,
R7 is Het2, R71- and/or R72- and/or R73-substituted phenyl, R74-substituted Het2, or naphthyl, in which
Het2 is either
a monocyclic 5-membered heteroaryl radical comprising one to four heteroatoms, each of which is selected from a group consisting of nitrogen, oxygen and sulfur,
or
a monocyclic 6-membered heteroaryl radical comprising one or two nitrogen atoms,
or
a fused bicyclic 9- or 10-membered heteroaryl comprising one to three heteroatoms, each of which is selected from a group consisting of nitrogen, oxygen and sulfur,
or
N-oxy-pyridyl,
R71 is hydroxyl, halogen, nitro, 1-4C-alkyl, 1-4C-alkoxy, amino, mono- or di-1-4C-alkylamino, 1-4C-alkylsulphonylamino, carboxyl, aryloxy, mono- or di-1-4C-alkylaminocarbonyl, carbamoyl, tetrazolyl, or -N(H)S(O)₂-N(R712)R713, in which
aryl is phenyl or R711-substituted phenyl, in which
R711 is halogen or 1-4C-alkyl,
R712 is 1-4C-alkyl, and
R713 is 1-4C-alkyl, or
R712 and R713 together and with inclusion of the nitrogen atom to which they are bound form a radical Het3, in which
Het3 is morpholin-4-yl,
R72 is halogen, 1-4C-alkyl or 1-4C-alkoxy,
R73 is 1-4C-alkyl or 1-4C-alkoxy,
R74 is 1-4C-alkyl, phenyl-1-4C-alkyl, arylsulphonyl, 1-4C-alkylsulphonyl, or -S(O)₂-N(R712)R713,
R8 is optionally substituted by R81 on a ring carbon atom, and is Het4, in which
Het4 is bonded to the pyrroloisoquinoline scaffold via a ring carbon atom, and is an [1,2,4]oxadiazolyl or an oxazolyl radical,
R81 is 1-4C-alkyl, 3-7C-cycloalkyl, 3-7C-cycloalkylmethyl, phenyl, or R811- and/or R812-substituted phenyl, in which
R811 is 1-4C-alkyl, 1-4C-alkoxy, or halogen,
R812 is 1-4C-alkyl, 1-4C-alkoxy, or halogen,
and the stereoisomers as well as the salts of these compounds and stereoisomers.

3. Compounds of formula I according to claim 1,
in which
R1 is bound to the 8-position of the pyrrolo[2.1-a]isoquinoline ring, and is 1-4C-alkoxy,
R2 is bound to the 7-position of the pyrrolo[2.1-a]isoquinoline ring, and is hydrogen, halogen or 1-4C-alkoxy,
R3 is bound to the 9-position of the pyrrolo[2.1-a]isoquinoline ring, and is 1-4C-alkoxy,
R4 is hydrogen,
R41 is hydrogen,
R5 is hydrogen or 1-4C-alkyl,
R51 is hydrogen or 1-4C-alkyl,
or
R4 and R5 together form a tetramethylene bridge and R41 and R51 are both hydrogen,
R6 is 1-4C-alkyl,
R7 is Het2, R71- and/or R72- and/or R73-substituted phenyl, R74-substituted Het2, or naphthyl, in which
Het2 is either
a monocyclic 5-membered heteroaryl radical comprising one to four heteroatoms, each of which is selected from a group consisting of nitrogen, oxygen and sulfur,
or
a monocyclic 6-membered heteroaryl radical comprising one or two nitrogen atoms,
or
a fused bicyclic 9- or 10-membered heteroaryl comprising one to three heteroatoms, each of which is selected from a group consisting of nitrogen, oxygen and sulfur,
or
N-oxy-pyridyl,
R71 is hydroxyl, halogen, 1-4C-alkyl, 1-4C-alkoxy or aryloxy, in which
aryl is phenyl or R711-substituted phenyl, in which
R711 is halogen or 1-4C-alkyl,
R72 is halogen, 1-4C-alkyl or 1-4C-alkoxy,
R73 is 1-4C-alkyl or 1-4C-alkoxy,
R74 is 1-4C-alkyl or phenyl-1-4C-alkyl,
R8 is substituted by R81 on a ring carbon atom, and is Het4, in which
Het4 is bonded to the pyrroloisoquinoline scaffold via a ring carbon atom, and is an [1,2,4]oxadiazolyl or an oxazolyl radical,
R81 is 1-4C-alkyl, 3-5C-cycloalkyl, 3-5C-cycloalkylmethyl, phenyl, or R811- and/or R812-substituted phenyl, in which
R811 is 1-4C-alkyl, 1-4C-alkoxy, or halogen,
R812 is 1-4C-alkyl, 1-4C-alkoxy, or halogen,
and the stereoisomers as well as the salts of these compounds and stereoisomers.

4. Compounds of formula I according to claim 1,
in which
R1 is bound to the 8-position of the pyrrolo[2.1-a]isoquinoline ring, and is 1-2C-alkoxy,
R2 is bound to the 7-position of the pyrrolo[2.1-a]isoquinoline ring, and is hydrogen, chlorine or fluorine,
R3 is bound to the 9-position of the pyrrolo[2.1-a]isoquinoline ring, and is 1-2C-alkoxy,
R4 is hydrogen,
R41 is hydrogen,
R5 is hydrogen or 1-2C-alkyl,
R51 is hydrogen,
R6 is 1-2C-alkyl, or 1-2C-alkyl substituted by R61, in which
R61 is 1-2C-alkoxycarbonyl or carboxyl,
R7 is Het2, R71- and/or R72- and/or R73-substituted phenyl, R74-substituted Het2, or naphthyl, in which
Het2 is either
a monocyclic 5-membered heteroaryl radical comprising one to four heteroatoms, each of which is selected from a group consisting of nitrogen, oxygen and sulfur,
or
a monocyclic 6-membered heteroaryl radical comprising one or two nitrogen atoms,
or
a fused bicyclic 9- or 10-membered heteroaryl comprising one to three heteroatoms, each of which is selected from a group consisting of nitrogen, oxygen and sulfur,
or
N-oxy-pyridyl,
R71 is hydroxyl, chlorine, fluorine, 1-2C-alkyl, 1-2C-alkoxy, carboxyl or aryloxy, in which
aryl is phenyl or R711-substituted phenyl, in which
R711 is chlorine, fluorine or 1-2C-alkyl,
R72 is chlorine, fluorine, 1-2C-alkyl or 1-2C-alkoxy,
R73 is 1-2C-alkyl or 1-2C-alkoxy,
R74 is 1-2C-alkyl or phenyl-1-2C-alkyl,
R8 is Het4, in which
Het4 is 3-(R81)-[1,2,4]oxadiazol-5-yl or 5-(R81)-oxazol-2-yl,
R81 is 1-4C-alkyl, 3-5C-cycloalkyl, 3-5C-cycloalkylmethyl, phenyl, or R811-substituted phenyl, in which
R811 is 1-2C-alkyl, 1-2C-alkoxy, chlorine or fluorine,
and the stereoisomers as well as the salts of these compounds and stereoisomers.

5. Compounds of formula I according to claim 1,
in which
R1 is bound to the 8-position of the pyrrolo[2.1-a]isoquinoline ring, and is 1-2C-alkoxy,
R2 is bound to the 7-position of the pyrrolo[2.1-a]isoquinoline ring, and is hydrogen, chlorine or fluorine,
R3 is bound to the 9-position of the pyrrolo[2.1-a]isoquinoline ring, and is 1-2C-alkoxy,
R4 is hydrogen,
R41 is hydrogen,
R5 is hydrogen or 1-2C-alkyl,
R51 is hydrogen,
R6 is 1-4C-alkyl,
R7 is naphthyl, or
4-hydroxy-3,5-dimethylphenyl, 4-methoxy-3,5-dimethylphenyl, 2-methyl-4-hydroxy-phenyl or 2-fluoro-3,4-dimethoxy-phenyl,
pyridyl or quinolinyl, or
2-methyl-pyridin-4-yl or 3-methyl-pyridin-4-yl,
R8 is Het4, in which
Het4 is 3-(R81)-[1,2,4]oxadiazol-5-yl or 5-(R81)-oxazol-2-yl,
R81 is 1-4C-alkyl, 3-5C-cycloalkyl, 3-5C-cycloalkylmethyl, phenyl, or R811-substituted phenyl, in which
R811 is 1-4C-alkyl, 1-4C-alkoxy, or halogen,
and the stereoisomers as well as the salts of these compounds and stereoisomers.

6. Compounds of formula I according to any of claims 1 to 5 comprising one or more of the following:
R1 is bound to the 8-position of the pyrrolo[2.1-a]isoquinoline ring, and is 1-2C-alkoxy, such as e.g. methoxy,
R2 is bound to the 7-position of the pyrrolo[2.1-a]isoquinoline ring, and is chlorine or fluorine, and
R3 is bound to the 9-position of the pyrrolo[2.1-a]isoquinoline ring, and is 1-2C-alkoxy, such as e.g. methoxy;
R4 is hydrogen,
R41 is hydrogen,
R5 is methyl, and
R51 is hydrogen;
and the stereoisomers as well as the salts of these compounds and stereoisomers.

7. Compounds according to any of claims 1 to 5, which are of formula la 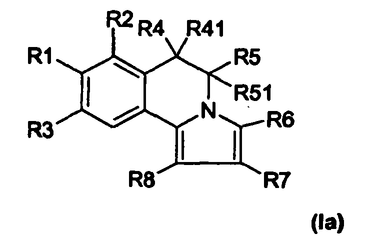 in which
R1 is methoxy,
R3 is methoxy,
R4 is hydrogen,
R41 is hydrogen,
R51 is hydrogen,
and in which any of the following substituent meanings for R2, R5, R6 and R8 apply:
| | R2 | R5 | R6 | R8 |
|---|---|---|---|---|
| 1.) | hydrogen | methyl | methyl | 3-methyl-[1,2,4]oxadiazol-5-yl |
| 2.) | hydrogen | methyl | methyl | 3-cyclopropyl-[1,2,4]oxadiazol-5-yl |
| 3.) | hydrogen | methyl | methyl | 3-phenyl-[1,2,4]oxadiazol-5-yl |
| 4.) | hydrogen | hydrogen | methyl | 3-methyl-[1,2,4]oxadiazol-5-yl |
| 5.) | hydrogen | hydrogen | methyl | 3-cyclopropyl-[1,2,4]oxadiazol-5-yl |
| 6.) | hydrogen | hydrogen | methyl | 3-phenyl-[1,2,4]oxadiazol-5-yl |
| 7.) | fluorine | methyl | methyl | 3-methyl-[1,2,4]oxadiazol-5-yl |
| 8.) | fluorine | methyl | methyl | 3-cyclopropyl-[1,2,4]oxadiazol-5-yl |
| 9.) | fluorine | methyl | methyl | 3-phenyl-[1,2,4]oxadiazol-5-yl |
| 10.) | chlorine | methyl | methyl | 3-methyl-[1,2,4]oxadiazol-5-yl |
| 11.) | chlorine | methyl | methyl | 3-cyclopropyl-[1,2,4]oxadiazol-5-yl |
| 12.) | chlorine | methyl | methyl | 3-phenyl-[1,2,4]oxadiazol-5-yl |
| 13.) | fluorine | hydrogen | methyl | 3-methyl-[1,2,4]oxadiazol-5-yl |
| 14.) | fluorine | hydrogen | methyl | 3-cyclopropyl-[1,2,4]oxadiazol-5-yl |
| 15.) | fluorine | hydrogen | methyl | 3-phenyl-[1,2,4]oxadiazol-5-yl |
| 16.) | chlorine | hydrogen | methyl | 3-methyl-[1,2,4]oxadiazol-5-yl |
| 17.) | chlorine | hydrogen | methyl | 3-cyclopropyl-[1,2,4]oxadiazol-5-yl |
| 18.) | chlorine | hydrogen | methyl | 3-phenyl-[1,2,4]oxadiazol-5-yl |
and the stereoisomers as well as the salts of these compounds and stereoisomers.

8. A compound according to any of the claims 1 to 7 for use in therapy.

9. Use of a compound according to any of the claims 1 to 7 in the manufacture of pharmaceutical compositions for the treatment of neurologic and/or psychiatric disorders, such as e.g. psychotic disorders, anxiety disorders, mood disorders or episodes, drug addictions, movement disorders, cognition deficiency disorders, obsessive/compulsive disorders, or neurodegenerative disorders.

10. A pharmaceutical composition comprising as an active ingredient an effective amount of at least one of the compounds according to any of the claims 1 to 7 together with suitable pharmaceutical auxiliaries and/or excipients.

11. A therapeutically effective and tolerable quantity of one or more compounds according to any of the claims 1 to 7 for use in the treatment of mammals, including humans, suffering from a neurologic or psychiatric disorder.

12. A therapeutically effective and tolerable quantity of one or more compounds according to any of the claims 1 to 7 for use in the regulation of fertility in mammals, including humans.

13. A therapeutically effective and tolerable quantity of one or more compounds according to any of the claims 1 to 7 for use in the treatment of mammals, including humans, of diabetes.

14. A therapeutically effective and tolerable quantity of one or more compounds according to any of the claims 1 to 7 for use in the reduction of body fat or body weight or the treatment of non-insulin dependent diabetes, metabolic syndrome or glucose intolerance in mammals, including humans.

15. The use according to claim 14 further comprising the use of an anti-obesity agent selected from rimonabant, orlistat, sibutramine, bromocriptine, ephedrine, leptin, pseudoephedrine, peptide YY₃₋₃₆ and analogues thereof.

## Patentansprüche

1. Verbindungen der Formel I 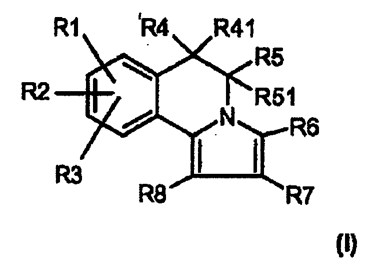 worin
R1 für Halogen, Nitro, Amino, Mono- oder Di-C₁₋₄-alkylamino, C₁₋₄-Alkyl, Hydroxyl, C₁₋₄-Alkoxy, C₁₋₄-Alkoxy-C₂₋₄-alkoxy, C₃₋₇-Cycloalkoxy, C₃₋₇-Cycloalkylmethoxy oder vollständig oder überwiegend fluorsubstituiertes C₁₋₄-Alkoxy steht,
R2 für Wasserstoff, Halogen oder C₁₋₄-Alkoxy steht und
R3 für Wasserstoff oder C₁₋₄-Alkoxy steht oder
R2 und R3 in ortho-Stellung zueinander an die Benzoringgruppierung gebunden sind und gemeinsam eine C₁₋₂-Alkylendioxybrücke bilden oder
R2 und R3 in ortho-Stellung zueinander an die Benzoringgruppierung gebunden sind und gemeinsam eine vollständig oder überwiegend fluorsubstituierte C₁₋₂-Alkylendioxybrücke bilden oder
R1 und R2 in ortho-Stellung zueinander an die Benzoringgruppierung gebunden sind und gemeinsam eine C₁₋₂-Alkylendioxybrücke bilden und R3 für Wasserstoff steht oder
R1 und R2 in ortho-Stellung zueinander an die Benzoringgruppierung gebunden sind und gemeinsam eine vollständig oder überwiegend fluorsubstituierte C₁₋₂-Alkylendioxybrücke bilden und R3 für Wasserstoff steht,
R4 für Wasserstoff, Fluor, Chlor, C₁₋₄-Alkyl, Trifluormethyl, Cyclopropyl, Cyano, C₁₋₄-Alkoxycarbonyl oder -CH₂-O-R411 steht, wobei
R411 für Wasserstoff, C₁₋₄-Alkyl, C₁₋₄-Alkoxy-C₂₋₄-alkyl oder C₁₋₄-Alkylcarbonyl steht,
R41 für Wasserstoff oder C₁₋₄-Alkyl steht,
R5 für Wasserstoff, Fluor oder C₁₋₄-Alkyl steht und
R51 für Wasserstoff oder C₁₋₄-Alkyl steht
oder
R4 für Wasserstoff, Fluor, Chlor oder C₁₋₄-Alkyl steht,
R41 für Wasserstoff oder C₁₋₄-Alkyl steht,
R5 für Wasserstoff, Fluor, C₁₋₄-Alkyl, Trifluormethyl, Cyclopropyl, Cyano, C₁₋₄-Alkoxycarbonyl oder -CH₂-O-R511 steht, wobei
R511 für Wasserstoff, C₁₋₄-Alkyl, C₁₋₄-Alkoxy-C₂₋₄-alkyl oder C₁₋₄-Alkylcarbonyl steht und
R51 für Wasserstoff oder C₁₋₄-Alkyl steht
oder
R4 und R5 gemeinsam eine C₁₋₄-Alkylenbrücke bilden und R41 und R51 beide für Wasserstoff stehen,
R6 für C₁₋₆-Alkyl, Amino, Formyl oder durch R61 substituiertes C₁₋₄-Alkyl steht, wobei
R61 für C₁₋₄-Alkoxycarbonyl, Carboxyl, C₁₋₄-Alkoxy, Hydroxyl, Halogen oder -N(R611)R612 steht, wobei
R611 für Wasserstoff, C₁₋₄-Alkyl, C₃₋₇-Cycloalkyl oder C₃₋₇-Cycloalkyl-C₁₋₄-alkyl steht und
R612 für Wasserstoff oder C₁₋₄-Alkyl steht oder
R611 und R612 gemeinsam und unter Einbeziehung des Stickstoffatoms, an das sie gebunden sind, einen Rest Het1 bilden, wobei
Het1 für einen 5- bis 7-gliedrigen gesättigten heterocylischen Ringrest, der ein Stickstoffatom, an das R611 und R612 gebunden sind, und gegebenenfalls ein weiteres Heteroatom aus der Gruppe bestehend aus Stickstoff, Sauerstoff und Schwefel enthält und der gegebenenfalls an einem Ringstickstoffatom durch R613 substituiert ist, steht, wobei
R613 für C₁₋₄-Alkyl, C₃₋₇-Cycloalkyl, C₃₋₇-Cycloalkyl-C₁₋₄-alkyl, Hydroxy-C₂₋₄-alkyl, C₁₋₄-Alkoxy-C₂₋₄-alkyl, Amino-C₂₋₄-alkyl, Mono- oder Di-C₁₋₄-alkylamino-C₂₋₄-alkyl, Formyl, Pyridyl oder Pyrimidinyl steht,
R7 für Phenyl, Het2, R71- und/oder R72- und/oder R73-substituiertes Phenyl, R74- und/oder R75-substituiertes Het2, Naphthyl oder R76- und/oder R77-substituiertes Naphthyl steht, wobei
Het2 über ein Ringkohlenstoffatom an das Pyrroloisochinolingerüst gebunden ist und für einen monocyclischen oder anellierten bicyclischen 5- bis 10-gliedrigen teil- oder vollaromatischen heterocyclischen Ringrest mit einem bis vier Heteroatomen, die jeweils aus der Gruppe bestehend aus Stickstoff, Sauerstoff und Schwefel ausgewählt sind, steht,
R71 für Hydroxyl, Halogen, Nitro, Cyano, Trifluormethyl, C₁₋₄-Alkyl, C₁₋₄-Alkoxy, C₃₋₇-Cycloalkoxy, C₃₋₇-Cycloalkylmethoxy, Amino, Mono- oder Di-C₁₋₄-alkylamino, C₁₋₄-Alkylsulfonylamino, Arylsulfonylamino, C₁₋₄-Alkoxycarbonyl, Carboxyl, C₁₋₄-Alkylthio, Aryloxy-C₂₋₄-alkoxy, Aryloxy-C₁₋₄-alkyl, Aryloxy, Aryl-C₁₋₄-alkoxy, Aryl, C₁₋₄-Alkoxy-C₂₋₄-alkoxy, C₁₋₄-Alkoxy-C₁₋₄-alkyl, Hydroxy-C₂₋₄-alkoxy, Amino-C₂₋₄-alkoxy, Mono- oder Di-C₁₋₄-alkylamino-C₂₋₄-alkoxy, vollständig oder überwiegend fluorsubstituiertes C₁₋₄-Alkoxy, Mono- oder Di-C₁₋₄-alkylaminocarbonyl, Carbamoyl, Tetrazolyl oder -N(H)S(O)₂-N(R712)R713 steht, wobei
Aryl für Phenyl oder R711-substituiertes Phenyl steht, wobei
R711 für Halogen, C₁₋₄-Alkyl, C₁₋₄-Alkoxy, Nitro oder Cyano steht,
R712 für C₁₋₄-Alkyl steht,
R713 für C₁₋₄-Alkyl steht oder
R712 und R713 gemeinsam und unter Einbeziehung des Stickstoffatoms, an das sie gebunden sind, einen Rest Het3 bilden, wobei
Het3 für Pyrrolidin-1-yl, Piperidin-1-yl oder Morpholin-4-yl steht,
R72 für Halogen, C₁₋₄-Alkyl, C₁₋₄-Alkoxy oder C₁₋₄-Alkoxycarbonyl steht,
R73 für C₁₋₄-Alkyl oder C₁₋₄-Alkoxy steht,
R74 für Halogen, C₁₋₄-Alkyl, Trifluormethyl, C₁₋₄-Alkoxy, Cyano, Amino, Mono- oder Di-C₁₋₄-alkylamino, C₁₋₄-Alkoxycarbonyl, Morpholino, Carboxyl, Nitro, Phenyl, Phenyloxy, Phenyl-C₁₋₄-alkyl, Arylsulfonyl, C₁₋₄-Alkylsulfonyl oder -S(O)₂-N(R712)R713 steht,
R75 für C₁₋₄-Alkyl oder Halogen steht,
R76 für Halogen, Hydroxyl, C₁₋₄-Alkyl, C₁₋₄-Alkoxy, Carboxyl oder C₁₋₄-Alkoxycarbonyl steht,
R77 für C₁₋₄-Alkyl oder C₁₋₄-Alkoxy steht,
R8 gegebenenfalls durch R81 substituiert ist und für Het 4 steht, wobei
Het4 über ein Ringkohlenstoffatom an das Pyrroloisochinolingerüst gebunden ist und für einen Oxadiazolyl- oder Oxazolylrest steht,
R81 für C₁₋₄-Alkyl, C₃₋₇-Cycloalkyl, C₃₋₇-Cycloalkylmethyl, Phenyl oder R811- und/oder R812-substituiertes Phenyl steht, wobei
R811 für C₁₋₄-Alkyl, C₁₋₄-Alkoxy oder Halogen steht,
R812 für C₁₋₄-Alkyl, C₁₋₄-Alkoxy oder Halogen steht,
und die Stereoisomere sowie die Salze dieser Verbindungen und Stereoisomere.

2. Verbindungen der Formel I nach Anspruch 1,
worin
R1 für C₁₋₄-Alkoxy, C₃₋₇-Cycloalkoxy, C₃₋₇-Cycloalkylmethoxy oder vollständig oder überwiegend fluorsubstituiertes C₁₋₄-Alkoxy steht,
R2 für Wasserstoff, Halogen oder C₁₋₄-Alkoxy steht und
R3 für C₁₋₄-Alkoxy steht oder
R2 und R3 in ortho-Stellung zueinander an die Benzoringgruppierung gebunden sind und gemeinsam eine C₁₋₂-Alkylendioxybrücke bilden oder
R2 und R3 in ortho-Stellung zueinander an die Benzoringgruppierung gebunden sind und gemeinsam eine vollständig oder überwiegend fluorsubstituierte C₁₋₂-Alkylendioxybrücke bilden oder
R1 und R2 in ortho-Stellung zueinander an die Benzoringgruppierung gebunden sind und gemeinsam eine C₁₋₂-Alkylendioxybrücke bilden und R3 für Wasserstoff steht oder
R1 und R2 in ortho-Stellung zueinander an die Benzoringgruppierung gebunden sind und gemeinsam eine vollständig oder überwiegend fluorsubstituierte C₁₋₂-Alkylendioxybrücke bilden und R3 für Wasserstoff steht
und keine der Gruppen R1, R2 und R3 an die 10-Position des Pyrrolo[2.1-a]isochinolinrings gebunden ist,
R4 für Wasserstoff oder C₁₋₄-Alkyl steht,
R41 für Wasserstoff oder C₁₋₄-Alkyl steht,
R5 für Wasserstoff, C₁₋₄-Alkyl, Cyano oder C₁₋₄-Alkoxycarbonyl steht,
R51 für Wasserstoff oder C₁₋₄-Alkyl steht
oder
R4 und R5 gemeinsam eine C₃₋₄-Alkylenbrücke bilden und R41 und R51 beide für Wasserstoff stehen
R6 für C₁₋₆-Alkyl oder durch R61 substituiertes C₁₋₄-Alkyl steht, wobei
R61 für C₁₋₄-Alkoxycarbonyl oder Carboxyl steht,
R7 für Het2, R71- und/oder R72- und/oder R73-substituiertes Phenyl, R74-substituiertes Het2 oder Naphthyl steht, wobei
Het2 entweder
für einen monocyclischen 5-gliedrigen Heteroarylrest mit einem bis vier Heteroatomen, die jeweils aus der Gruppe bestehend aus Stickstoff, Sauerstoff und Schwefel ausgewählt sind,
oder
für einen monocyclischen 6-gliedrigen Heteroarylrest mit einem oder zwei Stickstoffatomen oder
für ein anelliertes bicyclisches 9- oder 10-gliedriges Heteroaryl mit einem bis drei Heteroatomen, die jeweils aus der Gruppe bestehend aus Stickstoff, Sauerstoff und Schwefel ausgewählt sind,
oder
für N-Oxypyridyl
steht,
R71 für Hydroxyl, Halogen, Nitro, C₁₋₄-Alkyl, C₁₋₄-Alkoxy, Amino, Mono- oder Di-C₁₋₄-alkylamino, C₁₋₄-Alkylsulfonylamino, Carboxyl, Aryloxy, Mono- oder Di-C₁₋₄-alkylaminocarbonyl, Carbamoyl, Tetrazolyl oder -N(H)S(O)₂-N(R712)R713 steht, wobei
Aryl für Phenyl oder R711-substituiertes Phenyl steht, wobei
R711 für Halogen oder C₁₋₄-Alkyl steht,
R712 für C₁₋₄-Alkyl steht und
R713 für C₁₋₄-Alkyl steht oder
R712 und R713 gemeinsam und unter Einbeziehung des Stickstoffatoms, an das sie gebunden sind, einen Rest Het3 bilden, wobei
Het3 für Morpholin-4-yl steht,
R72 für Halogen, C₁₋₄-Alkyl oder C₁₋₄-Alkoxy steht,
R73 für C₁₋₄-Alkyl oder C₁₋₄-Alkoxy steht,
R74 für C₁₋₄-Alkyl, Phenyl-C₁₋₄-alkyl, Arylsulfonyl, C₁₋₄-Alkylsulfonyl oder -S(O)₂-N(R712)R713 steht,
R8 gegebenenfalls an einem Ringkohlenstoffatom durch R81 substituiert ist und für Het 4 steht, wobei
Het4 über ein Ringkohlenstoffatom an das Pyrroloisochinolingerüst gebunden ist und für einen [1,2,4]Oxadiazolyl- oder Oxazolylrest steht,
R81 für C₁₋₄-Alkyl, C₃₋₇-Cycloalkyl, C₃₋₇-Cycloalkylmethyl, Phenyl oder R811- und/oder R812-substituiertes Phenyl steht, wobei
R811 für C₁₋₄-Alkyl, C₁₋₄-Alkoxy oder Halogen steht,
R812 für C₁₋₄-Alkyl, C₁₋₄-Alkoxy oder Halogen steht,
und die Stereoisomere sowie die Salze dieser Verbindungen und Stereoisomere.

3. Verbindungen der Formel I nach Anspruch 1,
worin
R1 an die 8-Position des Pyrrolo[2.1-a]-isochinolinrings gebunden ist und für C₁₋₄-Alkoxy steht,
R2 an die 7-Position des Pyrrolo[2.1-a]-isochinolinrings gebunden ist und für Wasserstoff, Halogen oder C₁₋₄-Alkoxy steht,
R3 an die 9-Position des Pyrrolo[2.1-a]-isochinolinrings gebunden ist und für C₁₋₄-Alkoxy steht,
R4 für Wasserstoff steht,
R41 für Wasserstoff steht,
R5 für Wasserstoff oder C₁₋₄-Alkyl steht,
R51 für Wasserstoff oder C₁₋₄-Alkyl steht
oder
R4 und R5 gemeinsam eine Tetramethylenbrücke bilden und R41 und R51 beide für Wasserstoff stehen,
R6 für C₁₋₄-Alkyl steht,
R7 für Het2, R71- und/oder R72- und/oder R73-substituiertes Phenyl, R74-substituiertes Het2 oder Naphthyl steht, wobei
Het2 entweder
für einen monocyclischen 5-gliedrigen Heteroarylrest mit einem bis vier Heteroatomen, die jeweils aus der Gruppe bestehend aus Stickstoff, Sauerstoff und Schwefel ausgewählt sind,
oder
für einen monocyclischen 6-gliedrigen Heteroarylrest mit einem oder zwei Stickstoffatomen
oder
für ein anelliertes bicyclisches 9- oder 10-gliedriges Heteroaryl mit einem bis drei Heteroatomen, die jeweils aus der Gruppe bestehend aus Stickstoff, Sauerstoff und Schwefel ausgewählt sind,
oder
für N-Oxypyridyl
steht,
R71 für Hydroxyl, Halogen, C₁₋₄-Alkyl, C₁₋₄-Alkoxy oder Aryloxy steht, wobei
Aryl für Phenyl oder R711-substituiertes Phenyl steht, wobei
R711 für Halogen oder C₁₋₄-Alkyl steht,
R72 für Halogen, C₁₋₄-Alkyl oder C₁₋₄-Alkoxy steht,
R73 für C₁₋₄-Alkyl oder C₁₋₄-Alkoxy steht,
R74 für C₁₋₄-Alkyl oder Phenyl-C₁₋₄-alkyl steht,
R8 an einem Ringkohlenstoffatom durch R81 substituiert ist und für Het4 steht, wobei
Het4 über ein Ringkohlenstoffatom an das Pyrroloisochinolingerüst gebunden ist und für einen [1,2,4]Oxadiazolyl- oder Oxazolylrest steht,
R81 für C₁₋₄-Alkyl, C₃₋₅-Cycloalkyl, C₃₋₅-Cycloalkylmethyl, Phenyl oder R811- und/oder R812-substituiertes Phenyl steht, wobei
R811 für C₁₋₄-Alkyl, C₁₋₄-Alkoxy oder Halogen steht,
R812 für C₁₋₄-Alkyl, C₁₋₄-Alkoxy oder Halogen steht,
und die Stereoisomere sowie die Salze dieser Verbindungen und Stereoisomere.

4. Verbindungen der Formel I nach Anspruch 1,
worin
R1 an die 8-Position des Pyrrolo[2.1-a]-isochinolinrings gebunden ist und für C₁₋₂-Alkoxy steht,
R2 an die 7-Position des Pyrrolo[2.1-a]-isochinolinrings gebunden ist und für Wasserstoff, Chlor oder Fluor steht,
R3 an die 9-Position des Pyrrolo[2.1-a]-isochinolinrings gebunden ist und für C₁₋₂-Alkoxy steht,
R4 für Wasserstoff steht,
R41 für Wasserstoff steht,
R5 für Wasserstoff oder C₁₋₂-Alkyl steht,
R51 für Wasserstoff steht,
R6 für C₁₋₂-Alkyl oder durch R61 substituiertes C₁₋₂-Alkyl steht, wobei
R61 für C₁₋₂-Alkoxycarbonyl oder Carboxyl steht,
R7 für Het2, R71- und/oder R72- und/oder R73-substituiertes Phenyl, R74-substituiertes Het2 oder Naphthyl steht, wobei
Het2 entweder
für einen monocyclischen 5-gliedrigen Heteroarylrest mit einem bis vier Heteroatomen, die jeweils aus der Gruppe bestehend aus Stickstoff, Sauerstoff und Schwefel ausgewählt sind,
oder
für einen monocyclischen 6-gliedrigen Heteroarylrest mit einem oder zwei Stickstoffatomen oder
für ein anelliertes bicyclisches 9- oder 10-gliedriges Heteroaryl mit einem bis drei Heteroatomen, die jeweils aus der Gruppe bestehend aus Stickstoff, Sauerstoff und Schwefel ausgewählt sind,
oder
für N-Oxypyridyl
steht,
R71 für Hydroxyl, Chlor, Fluor, C₁₋₂-Alkyl, C₁₋₂-Alkoxy, Carboxyl oder Aryloxy steht, wobei
Aryl für Phenyl oder R711-substituiertes Phenyl steht, wobei
R711 für Chlor, Fluor oder C₁₋₂-Alkyl steht,
R72 für Chlor, Fluor, C₁₋₂-Alkyl oder C₁₋₂-Alkoxy steht,
R73 für C₁₋₂-Alkyl oder C₁₋₂-Alkoxy steht,
R74 für C₁₋₂-Alkyl oder Phenyl-C₁₋₂-alkyl steht,
R8 für Het4 steht, wobei
Het4 für 3-(R81)-[1,2,4]Oxadiazol-5-yl- oder 5-(R81)-Oxazol-2-yl steht,
R81 für C₁₋₄-Alkyl, C₃₋₅-Cycloalkyl, C₃₋₅-Cycloalkylmethyl, Phenyl oder R811-substituiertes Phenyl steht, wobei
R811 für C₁₋₂-Alkyl, C₁₋₂-Alkoxy, Chlor oder Fluor steht,
und die Stereoisomere sowie die Salze dieser Verbindungen und Stereoisomere.

5. Verbindungen der Formel I nach Anspruch 1,
worin
R1 an die 8-Position des Pyrrolo[2.1-a]-isochinolinrings gebunden ist und für C₁₋₂-Alkoxy steht,
R2 an die 7-Position des Pyrrolo[2.1-a]-isochinolinrings gebunden ist und für Wasserstoff, Chlor oder Fluor steht,
R3 an die 9-Position des Pyrrolo[2.1-a]-isochinolinrings gebunden ist und für C₁₋₂-Alkoxy steht,
R4 für Wasserstoff steht,
R41 für Wasserstoff steht,
R5 für Wasserstoff oder C₁₋₂-Alkyl steht,
R51 für Wasserstoff steht,
R6 für C₁₋₄-Alkyl steht,
R7 für Naphthyl oder
4-Hydroxy-3,5-dimethylphenyl, 4-Methoxy-3,5-dimethylphenyl, 2-Methyl-4-hydroxyphenyl oder 2-Fluor-3,4-dimethoxyphenyl,
Pyridyl oder Chinolinyl oder
2-Methylpyridin-4-yl oder 3-Methylpyridin-4-yl
steht,
R8 für Het4 steht, wobei
Het4 für 3-(R81)-[1,2,4]Oxadiazol-5-yl- oder 5-(R81)-Oxazol-2-yl steht,
R81 für C₁₋₄-Alkyl, C₃₋₅-Cycloalkyl, C₃₋₅-Cycloalkylmethyl, Phenyl oder R811-substituiertes Phenyl steht, wobei
R811 für C₁₋₄-Alkyl, C₁₋₄-Alkoxy oder Halogen steht,
und die Stereoisomere sowie die Salze dieser Verbindungen und Stereoisomere.

6. Verbindungen der Formel I nach einem der Ansprüche 1 bis 5, umfassend eines oder mehrere der folgenden Merkmale:
R1 ist an die 8-Position des Pyrrolo[2.1-a]-isochinolinrings gebunden und steht für C₁₋₂-Alkoxy, wie z.B. Methoxy,
R2 ist an die 7-Position des Pyrrolo[2.1-a]-isochinolinrings gebunden und steht für Chlor oder Fluor,
R3 ist an die 9-Position des Pyrrolo[2.1-a]-isochinolinrings gebunden und steht für C₁₋₂-Alkoxy, wie z.B. Methoxy;
R4 steht für Wasserstoff,
R41 steht für Wasserstoff,
R5 steht für Methyl und
R51 steht für Wasserstoff,
und die Stereoisomere sowie die Salze dieser Verbindungen und Stereoisomere.

7. Verbindungen nach einem der Ansprüche 1 bis 5 mit der Formel Ia 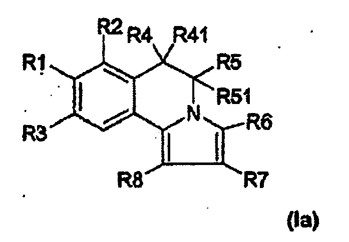 worin
R1 für Methoxy steht,
R3 für Methoxy steht,
R4 für Wasserstoff steht,
R41 für Wasserstoff steht,
R51 für Wasserstoff steht,
und worin beliebige der folgenden Substituentenbedeutungen für R2, R5, R6 und R8 zutreffen:
| | R2 | R5 | R6 | R8 |
|---|---|---|---|---|
| 1.) | Wasserstoff | Methyl | Methyl | 3-Methyl-[1,2,4]-diazol-5-yl |
| 2.) | Wasserstoff | Methyl | Methyl | 3-Cyclopropyl-[1,2,4]oxadiazol-5-yl |
| 3.) | Wasserstoff | Methyl | Methyl | 3-Phenyl-[1,2,4]oxadiazol-5-yl |
| 4.) | Wasserstoff | Wasserstoff | Methyl | 3-Methyl-[1,2,4]oxadiazol-5-yl |
| 5.) | Wasserstoff | Wasserstoff | Methyl | 3-Cyclopropyl-[1,2,4]oxadiazol-5-yl |
| 6.) | Wasserstoff | Wasserstoff | Methyl | 3-Phenyl-[1,2,4]oxadiazol-5-yl |
| 7.) | Fluor | Methyl | Methyl | 3-Methyl-[1,2,4]oxadiazol-5-yl |
| 8.) | Fluor | Methyl | Methyl | 3-Cyclopropyl-[1,2,4]oxadiazol-5-yl |
| 9.) | Fluor | Methyl | Methyl | 3-Phenyl-[1,2,4]oxadiazol-5-yl |
| 10.) | Chlor | Methyl | Methyl | 3-Methyl-[1,2,4]oxadiazol-5-yl |
| 11.) | Chlor | Methyl | Methyl | 3-Cyclopropyl-[1,2,4]oxadiazol-5-yl |
| 12.) | Chlor | Methyl | Methyl | 3-Phenyl-[1,2,4]oxadiazol-5-yl |
| 13.) | Fluor | Wasserstoff | Methyl | 3-Methyl-[1,2,4]oxadiazol-5-yl |
| 14.) | Fluor | Wasserstoff | Methyl | 3-Cyclopropyl-[1,2,4]oxadiazol-5-yl |
| 15.) | Fluor | Wasserstoff | Methyl | 3-Phenyl-[1,2,4]oxadiazol-5-yl |
| 16.) | Chlor | Wasserstoff | Methyl | 3-Methyl-[1,2,4]oxadiazol-5-yl |
| 17.) | Chlor | Wasserstoff | Methyl | 3-Cyclopropyl-[1,2,4]oxadiazol-5-yl |
| 18.) | Chlor | Wasserstoff | Methyl | 3-Phenyl-[1,2,4]oxadiazol-5-yl |
und die Stereoisomere sowie die Salze dieser Verbindungen und Stereoisomere.

8. Verbindung nach einem der Ansprüche 1 bis 7 zur Verwendung bei der Therapie.

9. Verwendung einer Verbindung nach einem der Ansprüche 1 bis 7 zur Herstellung von pharmazeutischen Zusammensetzungen zur Behandlung von neurologischen und/oder psychiatrischen Störungen, wie z.B. psychotischen Störungen, Angststörungen, Gemütsstörungen oder -episoden, Drogen- bzw. Arzneimittelsüchten, Bewegungsstörungen, kognitiven Funktionsstörungen, Zwangsneurosen oder neurodegenerativen Störungen.

10. Pharmazeutische Zusammensetzung, die als Wirkstoff eine wirksame Menge mindestens einer der Verbindungen nach einem der Ansprüche 1 bis 7 zusammen mit geeigneten pharmazeutischen Hilfsstoffen und/oder Trägerstoffen enthält.

11. Therapeutisch wirksame und tolerierbare Menge einer oder mehrerer Verbindungen nach einem der Ansprüche 1 bis 7 zur Verwendung bei der Behandlung von Säugetieren einschließlich Menschen, die an einer neurologischen oder psychiatrischen Störung leiden.

12. Therapeutisch wirksame und tolerierbare Menge einer oder mehrerer Verbindungen nach einem der Ansprüche 1 bis 7 zur Verwendung bei der Regulierung der Fruchtbarkeit bei Säugetieren einschließlich Menschen.

13. Therapeutisch wirksame und tolerierbare Menge einer oder mehrerer Verbindungen nach einem der Ansprüche 1 bis 7 zur Verwendung bei der Diabetesbehandlung von Säugetieren einschließlich Menschen.

14. Therapeutisch wirksame und tolerierbare Menge einer oder mehrerer Verbindungen nach einem der Ansprüche 1 bis 7 zur Verwendung bei der Verringerung des Körperfetts oder Körpergewichts oder der Behandlung von nicht-insulinabhängigem Diabetes, metabolischem Syndrom oder Glucoseintoleranz bei Säugetieren einschließlich Menschen.

15. Verwendung nach Anspruch 14, ferner umfassend die Verwendung eines unter Rimonabant, Orlistat, Sibutramin, Bromocriptin, Ephedrin, Leptin, Pseudoephedrin, Peptid YY₃₋₃₆ und Analogen davon ausgewählten Antiadipositums.

## Revendications

1. Composés de formule I 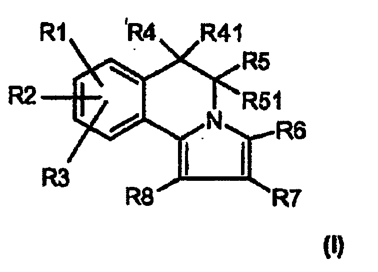 dans laquelle
R1 est un atome d'halogène, un groupe nitro, un groupe amino, un groupe mono- ou di-C₁₋₄-alkylamino, un groupe alkyle en C₁₋₄, un groupe hydroxyle, un groupe alcoxy en C₁₋₄, un groupe C₁₋₄-alcoxy-C₂₋₄-alcoxy, un groupe cycloalcoxy en C₃₋₇, un groupe C₃₋₇-cycloalkylméthoxy, ou un groupe alcoxy en C₁₋₄ totalement ou principalement substitué par du fluor,
R2 est un atome d'hydrogène, un atome d'halogène ou un groupe alcoxy en C₁₋₄, et
R3 est un atome d'hydrogène ou un groupe alcoxy en C₁₋₄, ou
R2 et R3, liés au fragment de noyau benzo en position ortho l'un par rapport à l'autre, forment conjointement un pont C₁₋₂-alkylènedioxy, ou
R2 et R3, liés au fragment de noyau benzo en position ortho l'un par rapport à l'autre, forment conjointement un pont C₁₋₂-alkylènedioxy totalement ou principalement substitué par du fluor, ou
R1 et R2, liés au fragment de noyau benzo en position ortho l'un par rapport à l'autre, forment conjointement un pont C₁₋₂-alkylènedioxy et R3 et un atome d'hydrogène, ou
R1 et R2, liés au fragment de noyau benzo en position ortho l'un par rapport à l'autre, forment conjointement un pont C₁₋₂-alkylènedioxy totalement ou principalement substitué par du fluor et R3 est un atome d'hydrogène,
R4 est un atome d'hydrogène, un atome de fluor, un atome de chlore, un groupe alkyle en C₁₋₄, un groupe trifluorométhyle, un groupe cyclopropyle, un groupe cyano, un groupe C₁₋₄-alcoxycarbonyle ou un groupe -CH₂-O-R411, dans lequel
R411 est un atome d'hydrogène, un groupe alkyle en C₁₋₄, un groupe C₁₋₄-alcoxy-C₂₋₄-alkyle ou un groupe C₁₋₄-alkylcarbonyle,
R41 est un atome d'hydrogène ou un groupe alkyle en C₁₋₄,
R5 est un atome d'hydrogène, un atome de fluor ou un groupe alkyle en C₁₋₄, et
R51 est un atome d'hydrogène ou un groupe alkyle en C₁₋₄,
ou
R4 est un atome d'hydrogène, un atome de fluor, un atome de chlore ou un groupe alkyle en C₁₋₄,
R41 est un atome d'hydrogène ou un groupe alkyle en C₁₋₄,
R5 est un atome d'hydrogène, un atome de fluor, un groupe alkyle en C₁₋₄, un groupe trifluorométhyle, un groupe cyclopropyle, un groupe cyano, un groupe C₁₋₄-alcoxycarbonyle ou un groupe -CH₂-O-R511, dans lequel
R511 est un atome d'hydrogène, un groupe alkyle en C₁₋₄, un groupe C₁₋₄-alcoxy-C₂₋₄-alkyle ou un groupe C₁₋₄-alkylcarbonyle, et
R51 est un atome d'hydrogène ou un groupe alkyle en C₁₋₄,
ou
R4 et R5 forment conjointement un pont alkylène en C₁₋₄, et R41 et R51 sont tous les deux un atome d'hydrogène,
R6 est un groupe alkyle en C₁₋₆, un groupe amino, un groupe formyle ou un groupe alkyle en C₁₋₄ substitué par R61, dans lequel
R61 est un groupe C₁₋₄-alcoxycarbonyle, un groupe carboxyle, un groupe alcoxy en C₁₋₄, un groupe hydroxyle, un atome d'halogène ou un groupe -N(R611)R612, dans lequel
R611 est un atome d'hydrogène, un groupe alkyle en C₁₋₄, un groupe cycloalkyle en C₃₋₇ ou un groupe C₃₋₇-cycloalkyl-C₁₋₄-alkyle, et
R612 est un atome d'hydrogène ou un groupe alkyle en C₁₋₄, ou
R611 et R612, conjointement et en incluant l'atome d'azote auquel ils sont liés, forment un radical Het1, dans lequel
Het1 est un radical de noyau hétérocyclique saturé à 5-7 chaînons, renfermant un atome d'azote, auquel R611 et R612 sont liés, et éventuellement un hétéroatome supplémentaire choisi parmi un groupe constitué d'un atome d'azote, d'un atome d'oxygène et d'un atome de soufre, et éventuellement substitué par R613 sur un atome d'azote du noyau, dans lequel
R613 est un groupe alkyle en C₁₋₄, un groupe cycloalkyle en C₃₋₇, un groupe C₃₋₇-cycloalkyl-C₁₋₄-alkyle, un groupe hydroxy-C₂₋₄-alkyle, un groupe C₁₋₄-alcoxy-C₂₋₄-alkyle, un groupe amino-C₂₋₄-alkyle, un groupe mono- ou di-C₁₋₄-alkylamino-C₂₋₄-alkyle, un groupe formyle, un groupe pyridyle ou un groupe pyrimidinyle,
R7 est un groupe phényle, un groupe Het2, un groupe phényle substitué par R71 et/ou R72 et/ou R73, un groupe Het2 substitué par R74 et/ou R75, un groupe naphtyle ou un groupe naphtyle substitué par R76 et/ou R77, dans lesquels
Het2 est lié au squelette pyrroloisoquinoléine par l'intermédiaire d'un atome de carbone du noyau, et est un radical de noyau hétérocyclique partiellement ou entièrement aromatique, monocyclique ou bicyclique condensé, à 5-10 chaînons, renfermant un à quatre hétéroatomes, dont chacun est choisi parmi un groupe constitué d'un atome d'azote, d'un atome d'oxygène et d'un atome de soufre,
R71 est un groupe hydroxyle, un atome d'halogène, un groupe nitro, un groupe cyano, un groupe trifluorométhyle, un groupe alkyle en C₁₋₄, un groupe alcoxy en C₁₋₄, un groupe cycloalcoxy en C₃₋₇, un groupe C₃₋₇-cycloalkylméthoxy, un groupe amino, un groupe mono- ou di-C₁₋₄-alkylamino, un groupe C₁₋₄-alkylsulfonylamino, un groupe arylsulfonylamino, un groupe C₁₋₄-alcoxycarbonyle, un groupe carboxyle, un groupe alkylthio en C₁₋₄, un groupe aryloxy-C₂₋₄-alcoxy, un groupe aryloxy-C₁₋₄-alkyle, un groupe aryloxy, un groupe aryl-C₁₋₄-alcoxy, un groupe aryle, un groupe C₁₋₄-alcoxy-C₂₋₄-alcoxy, un groupe C₁₋₄-alcoxy-C₁₋₄-alkyle, un groupe hydroxy-C₂₋₄-alcoxy, un groupe amino-C₂₋₄-alcoxy, un groupe mono- ou di-C₁₋₄-alkylamino-C₂₋₄-alcoxy, un groupe alcoxy en C₁₋₄ totalement ou principalement substitué par du fluor, un groupe mono- ou di-C₁₋₄-alkylaminocarbonyle, un groupe carbamoyle, un groupe tétrazolyle ou un groupe -N(H)S(O)₂-N(R712)R713, dans lesquels
aryle est un groupe phényle ou un groupe phényle substitué par R711, dans lequel
R711 est un atome d'halogène, un groupe alkyle en C₁₋₄, un groupe alcoxy en C₁₋₄, un groupe nitro ou un groupe cyano,
R712 est un groupe alkyle en C₁₋₄,
R713 est un groupe alkyle en C₁₋₄, ou
R712 et R713, conjointement et en incluant l'atome d'azote auquel ils sont liés, forment un radical Het3, dans lequel
Het3 est un groupe pyrrolidin-1-yle, un groupe pipéridin-1-yle ou un groupe morpholin-4-yle,
R72 est un atome d'halogène, un groupe alkyle en C₁₋₄, un groupe alcoxy en C₁₋₄ ou un groupe C₁₋₄-alcoxycarbonyle,
R73 est un groupe alkyle en C₁₋₄ ou un groupe alcoxy en C₁₋₄,
R74 est un atome d'halogène, un groupe alkyle en C₁₋₄, un groupe trifluorométhyle, un groupe alcoxy en C₁₋₄, un groupe cyano, un groupe amino, un groupe mono- ou di-C₁₋₄-alkylamino, un groupe C₁₋₄-alcoxycarbonyle, un groupe morpholino, un groupe carboxyle, un groupe nitro, un groupe phényle, un groupe phényloxy, un groupe phényl-C₁₋₄-alkyle, un groupe arylsulfonyle, un groupe C₁₋₄-alkylsulfonyle ou un groupe -S(O)₂-N(R712)R713,
R75 est un groupe alkyle en C₁₋₄ ou un atome d'halogène,
R76 est un atome d'halogène, un groupe hydroxyle, un groupe alkyle en C₁₋₄, un groupe alcoxy en C₁₋₄, un groupe carboxyle ou un groupe C₁₋₄-alcoxycarbonyle,
R77 est un groupe alkyle en C₁₋₄ ou un groupe alcoxy en C₁₋₄,
R8 est éventuellement substitué par R81, et est Het4, dans lequel
Het4 est lié au squelette pyrroloisoquinoléine par l'intermédiaire d'un atome de carbone du noyau, et est un radical oxadiazolyle ou un radical oxazolyle,
R81 est un groupe alkyle en C₁₋₄, un groupe cycloalkyle en C₃₋₇, un groupe C₃₋₇-cycloalkylméthyle, un groupe phényle, ou un groupe phényle substitué par R811 et/ou R812, dans lequel
R811 est un groupe alkyle en C₁₋₄, un groupe alcoxy en C₁₋₄ ou un atome d'halogène,
R812 est un groupe alkyle en C₁₋₄, un groupe alcoxy en C₁₋₄ ou un atome d'halogène,
et les stéréoisomères ainsi que les sels de ces composés et stéréoisomères.

2. Composés de formule I selon la revendication 1,
dans laquelle
R1 est un groupe alcoxy en C₁₋₄, un groupe cycloalcoxy en C₃₋₇, un groupe C₃₋₇-cycloalkylméthoxy, ou un groupe alcoxy en C₁₋₄ totalement ou principalement substitué par du fluor,
R2 est un atome d'hydrogène, un atome d'halogène ou un groupe alcoxy en C₁₋₄, et
R3 est un groupe alcoxy en C₁₋₄, ou
R2 et R3, liés au fragment de noyau benzo en position ortho l'un par rapport à l'autre, forment conjointement un pont C₁₋₂-alkylènedioxy, ou
R2 et R3, liés au fragment de noyau benzo en position ortho l'un par rapport à l'autre, forment conjointement un pont C₁₋₂-alkylènedioxy totalement ou principalement substitué par du fluor, ou
R1 et R2, liés au fragment de noyau benzo en position ortho l'un par rapport à l'autre, forment conjointement un pont C₁₋₂-alkylènedioxy et R3 est un atome d'hydrogène, ou
R1 et R2, liés au fragment de noyau benzo en position ortho l'un par rapport à l'autre, forment conjointement un pont C₁₋₂-alkylènedioxy totalement ou principalement substitué par du fluor et R3 est un atome d'hydrogène,
et aucun parmi R1, R2 et R3 n'est lié à la position 10 du noyau pyrrolo[2.1-a]isoquinoléine,
R4 est un atome d'hydrogène ou un groupe alkyle en C₁₋₄,
R41 est un atome d'hydrogène ou un groupe alkyle en C₁₋₄,
R5 est un atome d'hydrogène, un groupe alkyle en C₁₋₄, un groupe cyano ou un groupe C₁₋₄-alcoxycarbonyle,
R51 est un atome d'hydrogène ou un groupe alkyle en C₁₋₄,
ou
R4 et R5 forment conjointement un pont alkylène en C₃₋₄, et R41 et R51 sont tous les deux un atome d'hydrogène,
R6 est un groupe alkyle en C₁₋₆ ou un groupe alkyle en C₁₋₄ substitué par R61, dans lequel
R61 est un groupe C₁₋₄-alcoxycarbonyle ou un groupe carboxyle,
R7 est un groupe Het2, un groupe phényle substitué par R71 et/ou R72 et/ou R73, un groupe Het2 substitué par R74 ou un groupe naphtyle, dans lesquels
Het2 est soit
un radical hétéroaryle monocyclique à 5 chaînons, renfermant un à quatre hétéroatomes, dont chacun est choisi parmi un groupe constitué d'un atome d'azote, d'un atome d'oxygène et d'un atome de soufre,
soit
un radical hétéroaryle monocyclique à 6 chaînons, renfermant un ou deux atomes d'azote,
soit
un groupe hétéroaryle bicyclique condensé à 9-10 chaînons, renfermant un à trois hétéroatomes, dont chacun est choisi parmi un groupe constitué d'un atome d'azote, d'un atome d'oxygène et d'un atome de soufre,
soit
un groupe N-oxy-pyridyle,
R71 est un groupe hydroxyle, un atome d'halogène, un groupe nitro, un groupe alkyle en C₁₋₄, un groupe alcoxy en C₁₋₄, un groupe amino, un groupe mono- ou di-C₁₋₄-alkylamino, un groupe C₁₋₄-alkylsulfonylamino, un groupe carboxyle, un groupe aryloxy, un groupe mono- ou di-C₁₋₄-alkylaminocarbonyle, un groupe carbamoyle, un groupe tétrazolyle ou un groupe -N(H)S(O)₂-N(R712)R713, dans lesquels
aryle est un groupe phényle ou un groupe phényle substitué par R711, dans lequel
R711 est un atome d'halogène ou un groupe alkyle en C₁₋₄,
R712 est un groupe alkyle en C₁₋₄, et
R713 est un groupe alkyle en C₁₋₄, ou
R712 et R713, conjointement et en incluant l'atome d'azote auquel ils sont liés, forment un radical Het3, dans lequel
Het3 est un groupe morpholin-4-yle,
R72 est un atome d'halogène, un groupe alkyle en C₁₋₄ ou un groupe alcoxy en C₁₋₄,
R73 est un groupe alkyle en C₁₋₄ ou un groupe alcoxy en C₁₋₄,
R74 est un groupe alkyle en C₁₋₄, un groupe phényl-C₁₋₄-alkyle, un groupe arylsulfonyle, un groupe alkylsulfonyle en C₁₋₄ ou un groupe -S(O)₂-N(R712)R713,
R8 est éventuellement substitué par R81 sur un atome de carbone du noyau, et est un groupe Het4, dans lequel
Het4 est lié au squelette pyrroloisoquinoléine par l'intermédiaire d'un atome de carbone du noyau, et est un radical [1,2,4]oxadiazolyle ou un radical oxazolyle,
R81 est un groupe alkyle en C₁₋₄, un groupe cycloalkyle en C₃₋₇, un groupe C₃₋₇-cycloalkylméthyle, un groupe phényle, ou un groupe phényle substitué par R811 et/ou R812, dans lequel
R811 est un groupe alkyle en C₁₋₄, un groupe alcoxy en C₁₋₄ ou un atome d'halogène,
R812 est un groupe alkyle en C₁₋₄, un groupe alcoxy en C₁₋₄ ou un atome d'halogène,
et les stéréoisomères ainsi que les sels de ces composés et stéréoisomères.

3. Composés de formule I selon la revendication 1,
dans laquelle
R1 est lié à la position 8 du noyau pyrrolo[2.1-a]isoquinoléine et est un groupe alcoxy en C₁₋₄,
R2 est lié à la position 7 du noyau pyrrolo[2.1-a]isoquinoléine et est un atome d'hydrogène, un atome d'halogène ou un groupe alcoxy en C₁₋₄,
R3 est lié à la position 9 du noyau pyrrolo[2.1-a]isoquinoléine et est un groupe alcoxy en C₁₋₄,
R4 est un atome d'hydrogène,
R41 est un atome d'hydrogène,
R5 est un atome d'hydrogène ou un groupe alkyle en C₁₋₄,
R51 est un atome d'hydrogène ou un groupe alkyle en C₁₋₄,
ou
R4 et R5 forment conjointement un pont tétraméthylène, et R41 et R51 sont tous les deux un atome d'hydrogène,
R6 est un groupe alkyle en C₁₋₄,
R7 est un groupe Het2, un groupe phényle substitué par R71 et/ou R72 et/ou R73, un groupe Het2 substitué par R74 ou un groupe naphtyle, dans lesquels
Het2 est soit
un radical hétéroaryle monocyclique à 5 chaînons, renfermant un à quatre hétéroatomes, dont chacun est choisi parmi un groupe constitué d'un atome d'azote, d'un atome d'oxygène et d'un atome de soufre,
soit
un radical hétéroaryle monocyclique à 6 chaînons, renfermant un ou deux atomes d'azote,
soit
un groupe hétéroaryle- bicyclique condensé à 9-10 chaînons, renfermant un à trois hétéroatomes, dont chacun est choisi parmi un groupe constitué d'un atome d'azote, d'un atome d'oxygène et d'un atome de soufre,
soit
un groupe N-oxy-pyridyle,
R71 est un groupe hydroxyle, un atome d'halogène, un groupe alkyle en C₁₋₄, un groupe alcoxy en C₁₋₄ ou un groupe aryloxy, dans lequel
aryle est un groupe phényle ou un groupe phényle substitué par R711, dans lequel
R711 est un atome d'halogène ou un groupe alkyle en C₁₋₄,
R72 est un atome d'halogène, un groupe alkyle en C₁₋₄ ou un groupe alcoxy en C₁₋₄,
R73 est un groupe alkyle en C₁₋₄ ou un groupe alcoxy en C₁₋₄,
R74 est un groupe alkyle en C₁₋₄ ou un groupe phényl-C₁₋₄-alkyle,
R8 est substitué par R81 sur un atome de carbone du noyau, et est un groupe Het4, dans lequel
Het4 est lié au squelette pyrroloisoquinoléine par l'intermédiaire d'un atome de carbone du noyau, et est un radical [1,2,4]oxadiazolyle ou un radical oxazolyle,
R81 est un groupe alkyle en C₁₋₄, un groupe cycloalkyle en C₃₋₅, un groupe C₃₋₅-cycloalkylméthyle, un groupe phényle, ou un groupe phényle substitué par R811 et/ou R812, dans lequel
R811 est un groupe alkyle en C₁₋₄, un groupe alcoxy en C₁₋₄ ou un atome d'halogène,
R812 est un groupe alkyle en C₁₋₄, un groupe alcoxy en C₁₋₄ ou un atome d'halogène,
et les stéréoisomères ainsi que les sels de ces composés et stéréoisomères.

4. Composés de formule I selon la revendication 1,
dans laquelle
R1 est lié à la position 8 du noyau pyrrolo[2.1-a]isoquinoléine, et est un groupe alcoxy en C₁₋₂,
R2 est lié à la position 7 du noyau pyrrolo[2.1-a]isoquinoléine, et est un atome d'hydrogène, un atome de chlore ou un atome de fluor,
R3 est lié à la position 9 du noyau pyrrolo[2.1-a]isoquinoléine, et est un groupe alcoxy en C₁₋₂,
R4 est un atome d'hydrogène,
R41 est un atome d'hydrogène,
R5 est un atome d'hydrogène ou un groupe alkyle en C₁₋₂,
R51 est un atome d'hydrogène,
R6 est un groupe alkyle en C₁₋₂ ou un groupe alkyle en C₁₋₂ substitué par R61, dans lequel
R61 est un groupe C₁₋₂-alcoxycarbonyle ou un groupe carboxyle,
R7 est un groupe Het2, un groupe phényle substitué par R71 et/ou R72 et/ou R73, un groupe Het2 substitué par R74 ou un groupe naphtyle, dans lesquels
Het2 est soit
un radical hétéroaryle monocyclique à 5 chaînons, renfermant un à quatre hétéroatomes, dont chacun est choisi parmi un groupe constitué d'un atome d'azote, d'un atome d'oxygène et d'un atome de soufre,
soit
un radical hétéroaryle monocyclique à 6 chaînons, renfermant un ou deux atomes d'azote,
soit
un groupe hétéroaryle bicyclique condensé à 9-10 chaînons, renfermant un à trois hétéroatomes, dont chacun est choisi parmi un groupe constitué d'un atome d'azote, d'un atome d'oxygène et d'un atome de soufre,
soit
un groupe N-oxy-pyridyle,
R71 est un groupe hydroxyle, un atome de chlore, un atome de fluor, un groupe alkyle en C₁₋₂, un groupe alcoxy en C₁₋₂, un groupe carboxyle ou un groupe aryloxy, dans lequel
aryle est un groupe phényle ou un groupe phényle substitué par R711, dans lequel
R711 est un atome de chlore, un atome de fluor ou un groupe alkyle en C₁₋₂,
R72 est un atome de chlore, un atome de fluor, un groupe alkyle en C₁₋₂ ou un groupe alcoxy en C₁₋₂,
R73 est un groupe alkyle en C₁₋₂ ou un groupe alcoxy en C₁₋₂,
R74 est un groupe alkyle en C₁₋₂ ou un groupe phényl-C₁₋₂-alkyle,
R8 est un groupe Het4, dans lequel
Het4 est un groupe 3-(R81)-[1,2,4]oxadiazol-5-yle ou un groupe 5-(R81)-oxazol-2-yle,
R81 est un groupe alkyle en C₁₋₄, un groupe cycloalkyle en C₃₋₅, un groupe C₃₋₅-cycloalkylméthyle, un groupe phényle ou un groupe phényle substitué par R811, dans lequel
R811 est un groupe alkyle en C₁₋₂, un groupe alcoxy en C₁₋₂, un atome de chlore ou un atome de fluor,
et les stéréoisomères ainsi que les sels de ces composés et stéréoisomères.

5. Composés de formule I selon la revendication 1,
dans laquelle
R1 est lié à la position 8 du noyau pyrrolo[2.1-a]isoquinoléine, et est un groupe alcoxy en C₁₋₂,
R2 est lié à la position 7 du noyau pyrrolo[2.1-a]isoquinoléine, et est un atome d'hydrogène, un atome de chlore ou un atome de fluor,
R3 est lié à la position 9 du noyau pyrrolo[2.1-a]isoquinoléine, et est un groupe alcoxy en C₁₋₂,
R4 est un atome d'hydrogène,
R41 est un atome d'hydrogène,
R5 est un atome d'hydrogène ou un groupe alkyle en C₁₋₂,
R51 est un atome d'hydrogène,
R6 est un groupe alkyle en C₁₋₄,
R7 est un groupe naphtyle, ou
un groupe 4-hydroxy-3,5-diméthylphényle, un groupe 4-méthoxy-3,5-diméthylphényle, un groupe 2-méthyl-4-hydroxyphényle ou un groupe 2-fluoro-3,4-diméthoxyphényle,
un groupe pyridyle ou un groupe quinoléinyle, ou
un groupe 2-méthylpyridin-4-yle ou un groupe 3-méthylpyridin-4-yle,
R8 est un groupe Het4, dans lequel
Het4 est un groupe 3-(R81)-[1,2,4]oxadiazol-5-yle ou un groupe 5-(R81)-oxazol-2-yle,
R81 est un groupe alkyle en C₁₋₄, un groupe cycloalkyle en C₃₋₅, un groupe C₃₋₅-cycloalkylméthyle, un groupe phényle, ou un groupe phényle substitué par R811, dans lequel
R811 est un groupe alkyle en C₁₋₄, un groupe alcoxy en C₁₋₄ ou un atome d'halogène,
et les stéréoisomères ainsi que les sels de ces composés et stéréoisomères.

6. Composés de formule I selon l'une quelconque des revendications 1 à 5, comprenant l'un ou plusieurs parmi les suivants :
R1 est lié à la position 8 du noyau pyrrolo[2.1-a]isoquinoléine, et est un groupe alcoxy en C₁₋₂, tel que, par exemple, un groupe méthoxy,
R2 est lié à la position 7 du noyau pyrrolo[2.1-a]isoquinoléine, et est un atome de chlore ou un atome de fluor, et
R3 est lié à la position 9 du noyau pyrrolo[2.1-a]isoquinoléine, et est un groupe alcoxy en C₁₋₂, tel que, par exemple, un groupe méthoxy ;
R4 est un atome d'hydrogène,
R41 est un atome d'hydrogène,
R5 est un groupe méthyle, et
R51 est un atome d'hydrogène ;
et les stéréoisomères ainsi que les sels de ces composés et stéréoisomères.

7. Composés selon l'une quelconque des revendications 1 à 5, qui sont de formule Ia 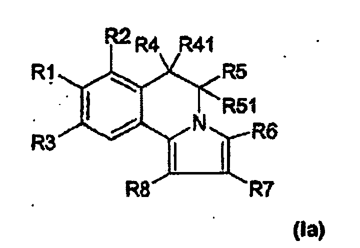 dans laquelle
R1 est un groupe méthoxy,
R3 est un groupe méthoxy,
R4 est un atome d'hydrogène,
R41 est un atome d'hydrogène,
R51 est un atome d'hydrogène,
et dans laquelle s'appliquent des significations quelconques parmi les significations suivantes des substituants pour R2, R5, R6 et R8 :
| | R2 | R5 | R6 | R8 |
|---|---|---|---|---|
| 1.) | hydrogène | méthyle | méthyle | 3-méthyl-[1,2,4]oxadiazol-5-yle |
| 2.) | hydrogène | méthyle | méthyle | 3-cyclopropyl-[1,2,4]oxadiazol-5-yle |
| 3.) | hydrogène | méthyle | méthyle | 3-phényl-[1,2,4]-oxadiazol-5-yle |
| 4.) | hydrogène | hydrogène | méthyle | 3-méthyl-[1,2,4]-oxadiazol-5-yle |
| 5.) | hydrogène | hydrogène | méthyle | 3-cyclopropyl-[1,2,4]oxadiazol-5-yle |
| 6.) | hydrogène | hydrogène | méthyle | 3-phényl-[1,2,4]oxadiazol-5-yle |
| 7.) | fluor | méthyle | méthyle | 3-méthyl-[1,2,4]oxadiazol-5-yle |
| 8.) | fluor | méthyle | méthyle | 3-cyclopropyl-[1,2,4]oxadiazol-5-yle |
| 9.) | fluor | méthyle | méthyle | 3-phényl-[1,2,4]oxadiazol-5-yle |
| 10.) | chlore | méthyle | méthyle | 3-méthyl-[1,2,4]oxadiazol-5-yle |
| 11.) | chlore | méthyle | méthyle | 3-cyclopropyl-[1,2,4]oxadiazol-5-yle |
| 12.) | chlore | méthyle | méthyle | 3-phényl-[1,2,4]oxadiazol-5-yle |
| 13.) | fluor | hydrogène | méthyle | 3-méthyl-[1,2,4]oxadiazol-5-yle |
| 14.) | fluor | hydrogène | méthyle | 3-cyclopropyl-[1,2,4]oxadiazol-5-yle |
| 15.) | fluor | hydrogène | méthyle | 3-phényl-[1,2,4]oxadiazol-5-yle |
| 16.) | chlore | hydrogène | méthyle | 3-méthyl-[1,2,4]oxadiazol-5-yle |
| 17.) | chlore | hydrogène | méthyle | 3-cyclopropyl-[1,2,4]oxadiazol-5-yle |
| 18.) | chlore | hydrogène | méthyle | 3-phényl-[1,2,4]oxadiazol-5-yle |
et les stéréoisomères ainsi que les sels de ces composés et stéréoisomères.

8. Composé selon l'une quelconque des revendications 1 à 7, destiné à être utilisé en thérapie.

9. Utilisation d'un composé selon l'une quelconque des revendications 1 à 7, pour la fabrication de compositions pharmaceutiques destinées au traitement de troubles neurologiques et/ou psychiatriques, tels que, par exemple, des troubles psychotiques, des troubles d'anxiété, des troubles ou des crises de l'humeur, des toxicomanies, des troubles du mouvement, des troubles de déficience cognitive, des troubles obsessifs/impulsifs, ou des troubles neurodégénératifs.

10. Composition pharmaceutique comprenant, en tant qu'ingrédient actif, une quantité efficace d'au moins l'un des composés selon l'une quelconque des revendications 1 à 7 conjointement avec des auxiliaires et/ou des excipients pharmaceutiques appropriés.

11. Quantité thérapeutiquement efficace et tolérable d'un ou de plusieurs composés selon l'une quelconque des revendications 1 à 7, destinée à être utilisée pour le traitement de mammifères, notamment d'êtres humains, soufrant d'un trouble neurologique ou psychiatrique.

12. Quantité thérapeutiquement efficace et tolérable d'un ou de plusieurs composés selon l'une quelconque des revendications 1 à 7, destinée à être utilisée pour la régulation de la fertilité chez des mammifères, notamment des êtres humains.

13. Quantité thérapeutiquement efficace et tolérable d'un ou de plusieurs composés selon l'une quelconque des revendications 1 à 7, destinée à être utilisée pour le traitement du diabète chez des mammifères, notamment des êtres humains.

14. Quantité thérapeutiquement efficace et tolérable d'un ou de plusieurs composés selon l'une quelconque des revendications 1 à 7, destinée à être utilisée pour la réduction de la graisse corporelle ou du poids corporel ou le traitement du diabète non insulino-dépendant, du syndrome métabolique ou de l'intolérance du glucose chez des mammifères, notamment des êtres humains.

15. Utilisation selon la revendication 14, comprenant en outre l'utilisation d'un agent anti-obésité choisi parmi le rimonabant, l'orlistat, la sibutramine, la bromocriptine, l'éphédrine, la leptine, la pseudoéphédrine, le peptide YY₃₋₃₆ et leurs analogues.
